# EUROPEAN PATENT APPLICATION

(11) **EP 4 563 578 A1**
(43) Date of publication of application: **04.06.2025**
(21) Application number: 23845724.6
(22) Date of filing: 28.07.2023
(51) Int. Cl.: C07D 491/22, A61P 35/00, A61K 31/4375

(54) **TOXIN MOLECULE SUITABLE FOR ANTIBODY-DRUG CONJUGATE**

(30) Priority: 28.07.2022 CN 202210903758
(71) Applicant: Minghui Pharmaceutical (Hangzhou) Limited, Hangzhou, Zhejiang 310018 (CN); Minghui Pharmaceutical (Shanghai) Limited, Pilot Free Trade Zone Pudong New Area Shanghai 201203 (CN)
(72) Inventor: CHEN, Yile, Hangzhou, Zhejiang 310018 (CN); LI, Ao, Hangzhou, Zhejiang 310018 (CN); YAO, Zhili, Hangzhou, Zhejiang 310018 (CN); CAO, Guoqing, Hangzhou, Zhejiang 310018 (CN)
(74) Representative: Mewburn Ellis LLP
(86) International application number: PCT/CN2023/109975
(87) International publication number: WO 2024/022520

(57) **Abstract**

The present application provides a toxin molecule suitable for an antibody-drug conjugate. In particular, the present application provides a compound represented by formula (I) below, or a pharmaceutically acceptable salt or hydrate thereof. The compound of the present application can be used in the preparation of a pharmaceutical composition for treating diseases associated with tumor cell proliferation.

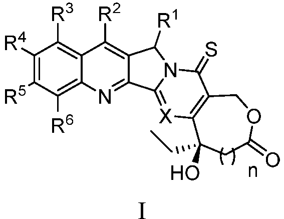

## Description

### Technical field

The present application relates to the field of pharmaceutical chemistry, in particular, the present application provides a toxin molecule with tumor cell proliferation inhibitory activity.

### Background

Antibody drug conjugate (ADC) which connects monoclonal antibodies or antibody fragments to biologically active cytotoxins *via* stable chemical linkers, fully utilizes the specific binding of antibodies to antigens on the surface of the tumor cells against the normal cells, and the efficiency of cytotoxins, while avoiding defects such as low efficacy of the antibodies, and excessive toxic side effects of the cytotoxins. This also means that, compared to previous traditional chemotherapy drugs, antibody-drug conjugates can more accurately bind to tumor cells and have less impact on normal cells.

At present, many ADC drugs have been used in clinical, such as Kadcyla, which is an ADC drug incorporating the HER2 targeted trastuzumab with the cytotoxic agent DM1.At the same time, patents for antibodies and ADC drugs targeting B7H3 have also been reported.

There are several types of cytotoxic small molecules used for antibody drug conjugates: one of them is camptothecin derivatives, which have anti-tumor effects by inhibiting topoisomerase I. Applications of camptothecin derivative exatecan in antibody drug conjugate (ADC) have been reported, but there is still need for developing more effective ADC drugs in the field.

DNA topoisomerase (Topo) is a widespread essential enzyme in organisms, which is involved in all key intranuclear processes such as DNA replication, transcription, recombination, and repairment. According to the difference between instant DNA strand breaks caused by topoisomerase, topoisomerase can be divided into two categories: topoisomerase I and topoisomerase II. Topoisomerase I and topoisomerase II together catalyze the unwinding of superhelical DNA during DNA replication, but topoisomerase II is involved in double-stranded breaks, while topoisomerase I only causes single-stranded breaks. Camptothecin and its analogs terminate progressive unwinding by reversibly binding to the DNA topoisomerase I-DNA complex, forming a ternary complex of camptothecin and its analogs-DNA topoisomerase I-DNA, which ultimately makes the replication forks crash into the ternary complex and induces unrepairable DNA breaks, thereby causing cell death.

### Summary of the invention

The purpose of the present application is to provide a toxin molecule suitable to be used in antibody drug conjugates.

In a first aspect of the present application, provided is a compound as shown in formula (I), or a pharmaceutically acceptable salt or hydrate thereof:

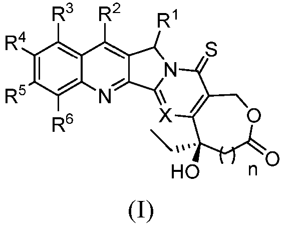

Wherein, n is 0 or 1,
X is selected from the group consisting of N and CR⁰;
R⁰ is selected from the group consisting of hydrogen, deuterium, halogen, C₁-C₈ alkyl, C₁-C₈ alkoxy, OH, NH₂, N₃ and NO₂;
R¹ is selected from the group consisting of hydrogen, deuterium, halogen, C₁-C₈ alkyl, C₁-C₈ alkoxy, C₁-C₈ haloalkyl, C₁-C₈ haloalkoxy, N₃, NO₂, NH₂, NH-OH, -NR'R", -COOR', -CONR'R", -NHR‴NR'R"; wherein R', R" and R‴ are each independently selected from hydrogen, alkyl, aryl, arylalkyl, acyl, alkoxycarbonyl, and aryloxycarbonyl;
R², R³, R⁴, R⁵ and R⁶ are each independently selected from the group consisting of hydrogen, deuterium, halogen, hydroxyl, cyano, NH₂, NO₂, substituted or unsubstituted C₁-C₈ alkyl, substituted or unsubstituted C₁-C₈ alkoxy, substituted or unsubstituted C₁-C₈ alkylthio, substituted or unsubstituted C₁-C₈ deuterated alkyl, -(CH₂)ₘtri(C₁-C₄ alkyl)silyl, -(CH₂)ₘ(C₃-C₈ cycloalkyl), -(CH₂)ₘ(3-12 membered heterocyclyl), -(CH₂)ₘN(R⁷)₂, -(CH₂)ₘS(CH₂)ₚR⁷, -(CH₂)ₘS(O)(CH₂)ₚR⁷, -(CH₂)ₘS(O)₂(CH₂)ₚR⁷, -(CH₂)ₘNH(CH₂)ₚR⁷, -(CH₂)ₘNHC(O)(CH₂)ₚR⁷, -(CH₂)ₘOC(O)(CH₂)ₚR⁷, -(CH₂)ₘC(O)(CH₂)ₚR⁷, and -CH=N(OtBu); wherein, m and p are each independently 0, 1, 2, 3, or 4;
or R² and R³ together with the carbon atoms to which they are attached form a substituted or unsubstituted C₅-C₈ carbocycle or a substituted or unsubstituted 5-12 membered heterocycle;
or R² and R³ together with the carbon atoms to which they are attached form a structure selected from the group consisting of a saturated or unsaturated 5-6 membered carbocycle that is unsubstituted or substituted by one or more R^{a}, and a saturated or unsaturated 5-6 membered heterocycle that is unsubstituted or substituted by one or more R^{a};
or R³ and R⁴, or R⁴ and R⁵, together with the carbon atoms to which they are attached form a structure selected from the group consisting of a saturated or unsaturated 5-12 membered carbocycle that is unsubstituted or substituted by one or more R^{a}, and a saturated or unsaturated 5-12 membered heterocycle that is unsubstituted or substituted by one or more R^{a}; wherein R^{a} is a substituted or unsubstituted substitutent and selected from the group consisting of hydrogen, deuterium, halogen, cyano, nitro, hydroxyl, amino, C₁-C₆ alkyl-NH-, (C₁-C₆ alkyl)₂N-, C₁-C₆ alkyl, C₂-C₆ alkenyl, C₂-C₆ alkynyl, C₁-C₆ alkoxy, allyl, benzyl, C₆-C₁₂ aryl, C₁-C₆ alkoxy-C₁-C₆ alkyl, C₁-C₆ alkoxy-carbonyl, phenoxycarbonyl, C₂-C₆ alkynyl-carbonyl, C₂-C₆ alkenyl-carbonyl, C₃-C₆ cycloalkyl-carbonyl, C₁-C₆ alkyl-sulfonyl, phenyl, 5-7 membered heteroaryl, C₃-C₈ cycloalkyl, 3-12 membered heterocyclyl, -(CH₂)ₘN(R⁷)₂, -(CH₂)ₘS(CH₂)ₚR⁷, -(CH₂)ₘS(O)(CH₂)ₚR⁷, -(CH₂)ₘS(O)₂(CH₂)ₚR⁷ -(CH₂)ₘNH(CH₂)ₚR⁷, -(CH₂)ₘNHC(O)(CH₂)ₚR⁷, -(CH₂)ₘOC(O)(CH₂)ₚR⁷, and -(CH₂)ₘC(O)(CH₂)ₚR⁷; wherein, m and p are each independently 0, 1, 2, 3 or 4, preferably 0, 1 or 2;
each R⁷ is independently selected from the group consisting of hydrogen, deuterium, halogen, substituted or unsubstituted C₁-C₈ alkyl, C₁-C₈ haloalkyl, C₁-C₈ deuterated alkyl, substituted or unsubstituted C₁-C₈ alkoxy, hydroxyl, amino, cyano, nitro, thiol, substituted or unsubstituted C₁-C₈ alkylene-OH, substituted or unsubstituted C₁-C₈ alkylene-NH₂, SO₂Me, -OC(O)(substituted or unsubstituted C₁-C₄ alkyl), -C(O)(substituted or unsubstituted C₁-C₄ alkyl), substituted or unsubstituted phenyl, substituted or unsubstituted 5-7 membered heteroaryl, substituted or unsubstituted C₃-C₈ cycloalkyl, and substituted or unsubstituted 3-12 membered heterocyclyl;
unless otherwise specified, each of the above groups can be substituted by substituents selected from the group consisting of deuterium, halogen, cyano, nitro, hydroxyl, amino, C₁-C₆ alkyl-NH-, (C₁-C₆ alkyl)₂N-, C₁-C₆ alkyl, C₂-C₆ alkenyl, C₂-C₆ alkynyl, C₁-C₆ alkoxy, halogenated C₁-C₆ alkyl, halogenated C₂-C₆ alkenyl, halogenated C₂-C₆ alkynyl, halogenated C₁-C₆ alkoxy, allyl, benzyl, C₆-C₁₂ aryl, C₁-C₆ alkoxy-C₁-C₆ alkyl, C₁-C₆ alkoxy-carbonyl, phenoxycarbonyl, C₂-C₆ alkynyl-carbonyl, C₂-C₆ alkenyl-carbonyl, C₃-C₆ cycloalkyl-carbonyl, C₁-C₆ alkyl-sulfonyl, phenyl, 5-7 membered heteroaryl, C₃-C₈ cycloalkyl, 3-12 membered heterocyclyl;
with the proviso that the compound is other than any structure selected from the group consisting of

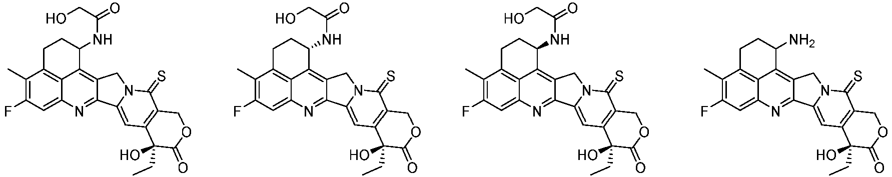

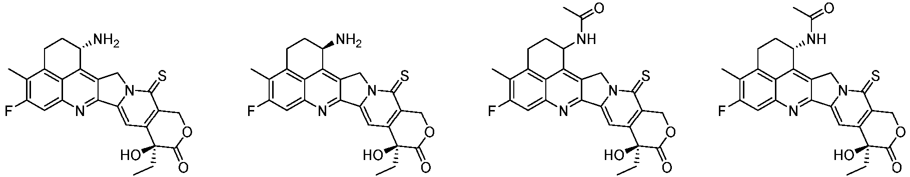

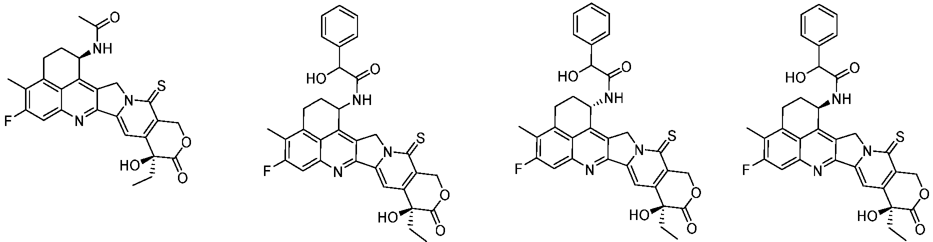

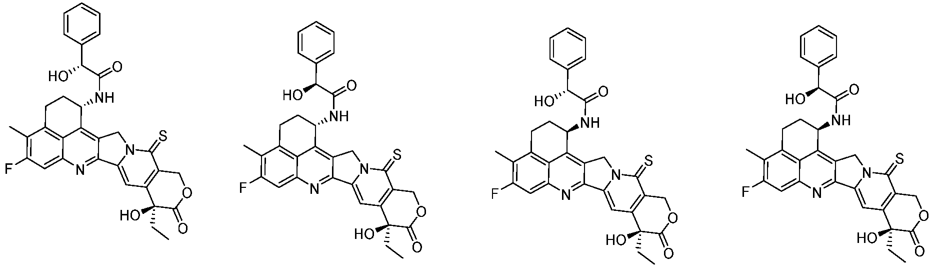

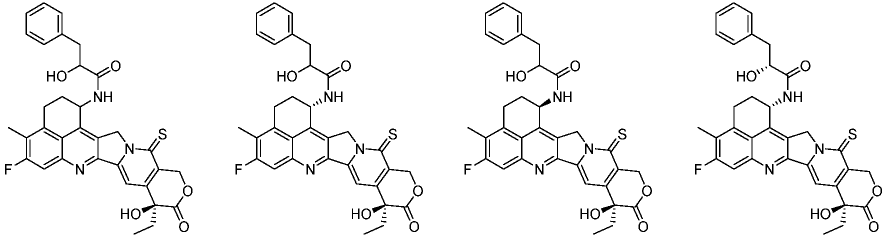

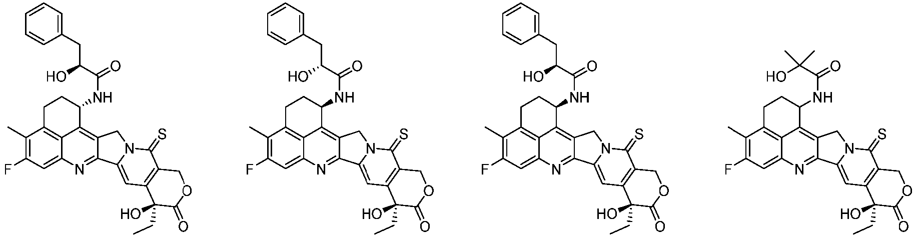

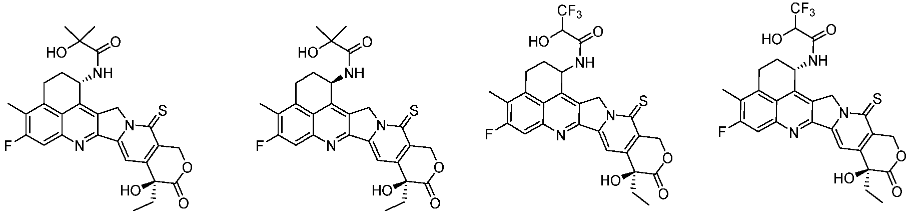

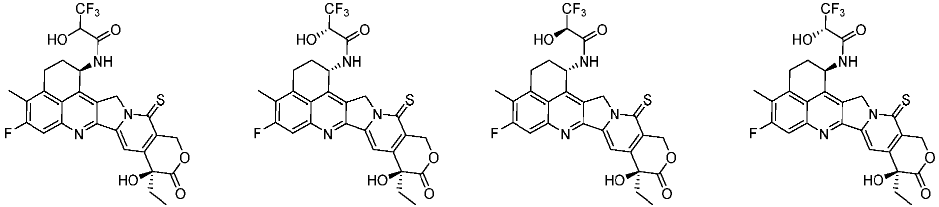

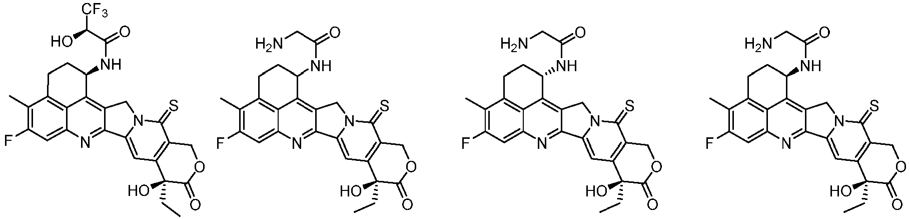

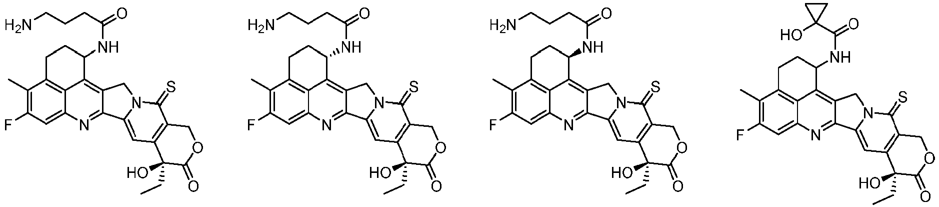

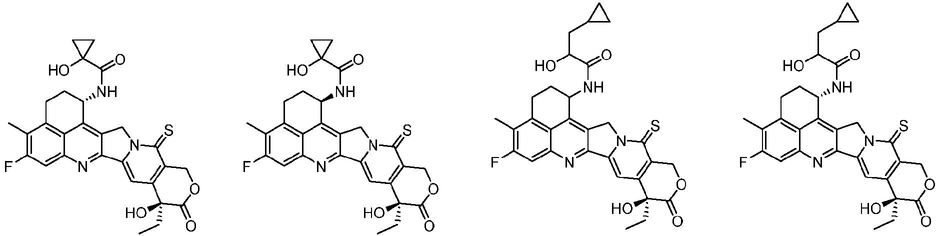

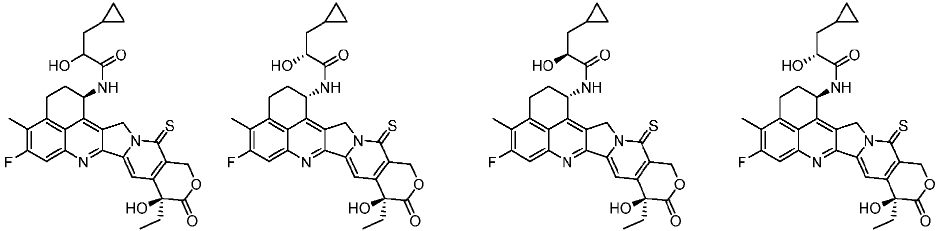

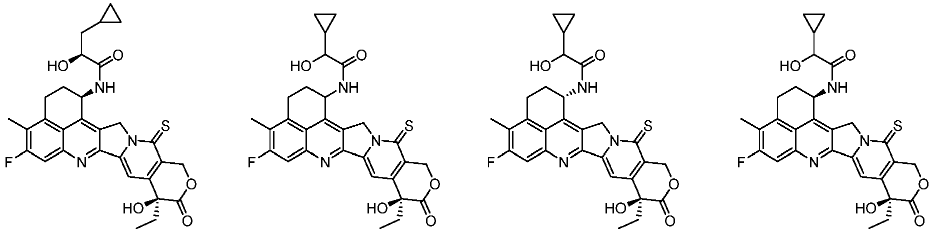

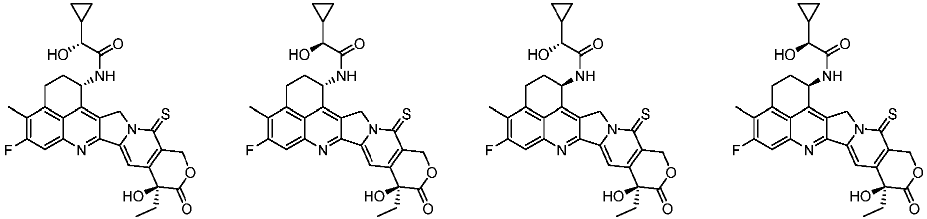

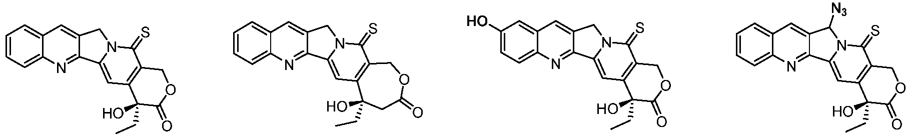

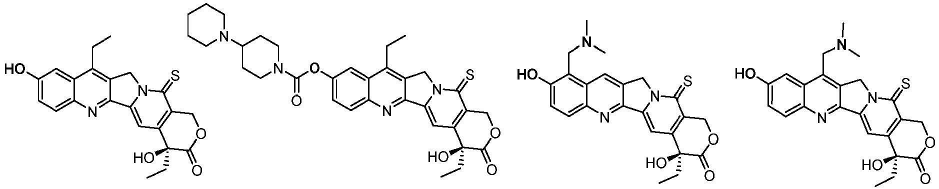

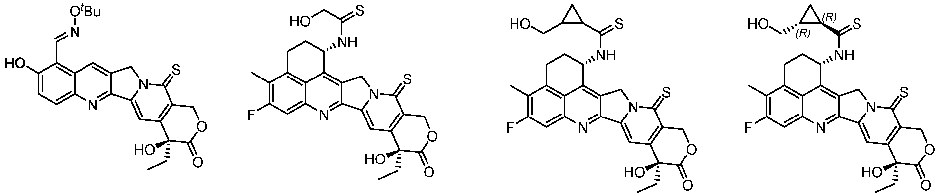

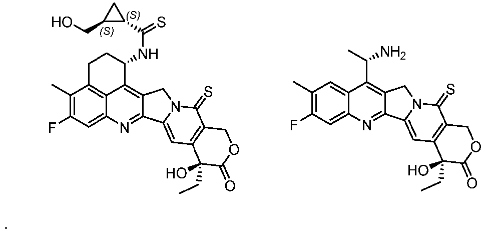

In another preferred embodiment, said compound of formula (I) has the structure as shown in formula (II) or formula (III) below:

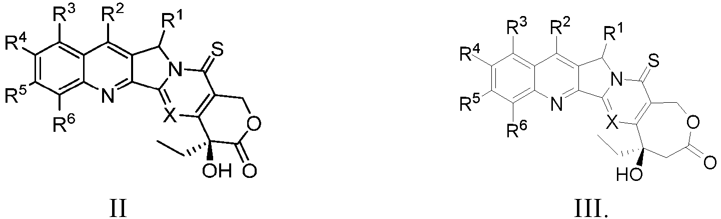

In another preferred embodiment, said compound of formula (I) has the structure as shown in formula (IV) or formula (V) below:

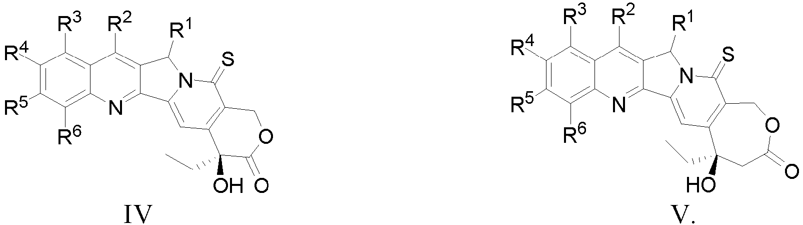

In another preferred embodiment, said compound of formula (I) has the structure as shown in formula (VI) or formula (VII) below:

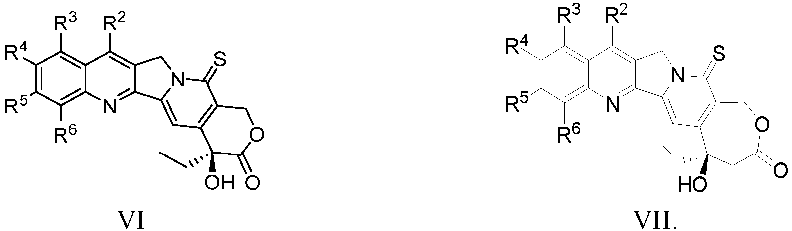

In another preferred embodiment, characterized in that the R⁴ is selected from the group consisting of hydrogen, deuterium, halogen, hydroxyl, cyano, NH₂, NO₂, substituted or unsubstituted C₁-C₈ alkyl, substituted or unsubstituted C₁-C₈ alkoxy, substituted or unsubstituted C₁-C₈ alkylthio, substituted or unsubstituted C₁-C₈ deuterated alkyl, -(CH₂)ₘ(C₃-C₈ cycloalkyl), -(CH₂)ₘ(3-12 membered heterocyclyl), -(CH₂)ₘN(R⁷)₂, -(CH₂)ₘS(O)(CH₂)ₚR⁷, -(CH₂)ₘS(O)₂(CH₂)ₚR⁷, and -(CH₂)ₘNH(CH₂)ₚR⁷; wherein, m and p are each independently 0, 1 or 2, and R⁷ is defined as described above;
R⁵ is selected from the group consisting of hydrogen, deuterium, halogen, NH₂, OH, substituted or unsubstituted C₁-C₈ alkyl, and substituted or unsubstituted C₁-C₈ alkoxy;
or R⁴ and R⁵ together with the carbon atoms to which they are attached form a structure selected from the group consisting of a saturated or unsaturated 5-6 membered carbocycle that is unsubstituted or substituted by one or more R^{a}, and a saturated or unsaturated 5-6 membered heterocycle that is unsubstituted or substituted by one or more R^{a}; wherein R^{a} is defined as described above.

In another preferred embodiment, R⁴ is selected from the group consisting of hydrogen, deuterium, halogen, hydroxyl, amino, C₁-C₃ alkyl, C₁-C₃ alkoxy, deuterated C₁-C₃ alkyl, deuterated C₁-C₃ alkoxy, halogenated C₁-C₃ alkyl, halogenated C₁-C₃ alkoxy, C₂-C₄ alkynyl, C₃-C₆ cycloalkyl;

In another preferred embodiment, R⁵ is selected from the group consisting of hydrogen, deuterium, halogen, hydroxyl, amino, C₁-C₃ alkyl, C₁-C₃ alkoxy, deuterated C₁-C₃ alkyl, deuterated C₁-C₃ alkoxy, halogenated C₁-C₃ alkyl, halogenated C₁-C₃ alkoxy;

In another preferred embodiment, R⁴ is selected from the group consisting of methyl, ethyl, methoxy, deuteromethoxy, monofluoromethyl, difluoromethyl, trifluoromethyl, monofluoromethoxy, difluoromethoxy, trifluoromethoxy, ethynyl, cyclopropyl

In another preferred embodiment, R⁵ is selected from the group consisting of methyl, ethyl, methoxy, deuteromethoxy, monofluoromethyl, difluoromethyl, trifluoromethyl, monofluoromethoxy, difluoromethoxy, trifluoromethoxy;

In another preferred embodiment, R⁴ and R⁵ are linked together to form substituted or unsubstituted -OCH₂O-, or substituted or unsubstituted -O(CH₂)₂O-.

In another preferred embodiment, R⁴ and R⁵ are linked together to form -OCH₂O-, -OCF₂O-, -O(CH₂)₂O-.

In another preferred embodiment, said compound of formula (I) has the structure as shown in formula (VIII) below: 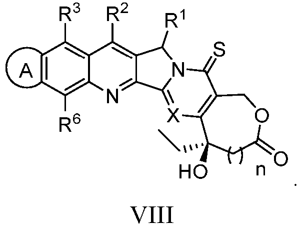 wherein ring A is a saturated or unsaturated 5-6 membered carbocyclic ring that is substituted or substituted by one or more R^{a}, or unsubstituted or unsaturated 5-6 membered heterocyclic ring that is unsubstituted or substituted by one or more R^{a}; wherein R^{a} is defined as described above, and R¹, R², R³, R⁶, X are defined as described above.

In another preferred embodiment, said compound of formula (I) has the structure as shown in formula (IX) below:

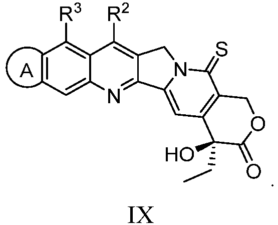

In another preferred embodiment, said compound of formula (I) has the structure shown in formulas (X)-(XV) below: 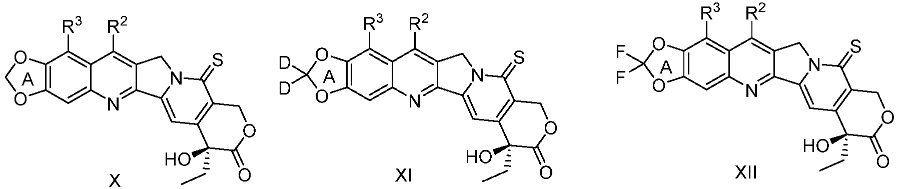 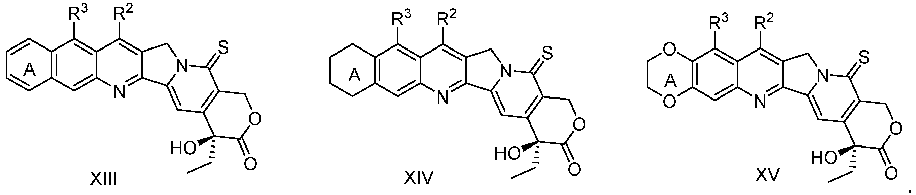 wherein ring A may be unsubstituted or optionally substituted; wherein the substituent is defined as described in the text.

In another preferred embodiment, R² and R³ are each independently selected from the group consisting of hydrogen, deuterium, halogen, NH₂, substituted or unsubstituted C₁-C₈ alkyl, substituted or unsubstituted C₁-C₈ deuterated alkyl, -(CH₂)ₘ(C₃-C₆ cycloalkyl), -(CH₂)ₘ(3-6 membered heterocyclyl), -(CH₂)ₘN(R⁷)₂, and -(CH₂)ₘOC(O)R⁷; wherein m is 0, 1, 2, 3, or 4, and R⁷ is defined as described above;
or, R² and R³ together with the carbon atoms to which they are attached form a structure selected from the group consisting of a saturated or unsaturated 5-6 membered carbocycle that is unsubstituted or substituted by one or more R^{a}, and a saturated or unsaturated 5-6 membered heterocycle that is unsubstituted or substituted by one or more R^{a}; wherein R^{a} is defined as described above.

In another preferred embodiment, said compound of formula (I) has the structure as shown in formula (XVI) below:

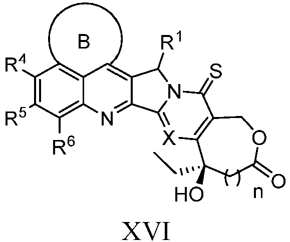

R² and R³ together with the carbon atoms to which they are attached form a structure selected from the group consisting of a saturated or unsaturated 5-6 membered carbocycle that is unsubstituted or substituted by one or more R^{a}, and a saturated or unsaturated 5-6 membered heterocycle that is unsubstituted or substituted by one or more R^{a}; wherein R^{a} is defined as described above.

In another preferred embodiment, said compound of formula (I) has the structure as shown in formula (XVII) below:

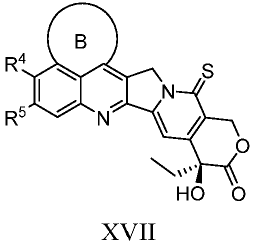

In another preferred embodiment, said compound of formula (I) has the structure shown in formulas (XVIII)-(X) below:

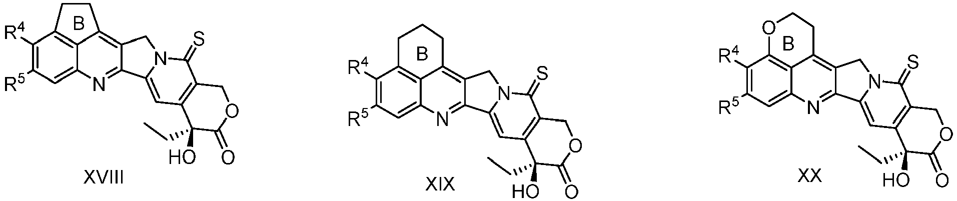

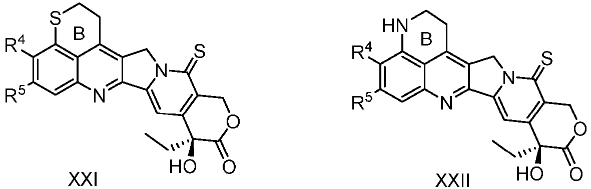

Ring B may be unsubstituted or optionally substituted; wherein the substituent is defined as described in the text.

In another preferred embodiment, said ring B is substituted with a substituent as shown in the formula below:

-Lₓ-R¹¹

Wherein, x is 1, 2, 3, 4, 5, or 6.

Each L is independently selected from the group consisting of CH₂, O, S, NH, NHC(O), C(O), C(NH), S(O), S(O)₂, C₆-C₁₀ aryl, 5-10 membered heteroaryl, 3-8 membered saturated or unsaturated carbocycle, and 4-10 membered saturated or unsaturated heterocycle,

R¹¹ is selected from the group consisting of OH, SH, NH₂.

In another preferred embodiment, said ring B may be unsubstituted or optionally substituted by -Lₓ-R¹¹,

Wherein, x is any integer from 0 to 10;

Each L is independently selected from the group consisting of CR^{w}R^{x}, O, S, NH, NR^{y}, NHC(O), C(O), C(NH), S(O), S(O)₂, C₆-C₁₀ aryl, 5-10 membered heteroaryl, 3-8 membered saturated or unsaturated carbocycle, 3-10 membered saturated or unsaturated heterocycle;

R¹¹ is selected from the group consisting of hydrogen, OH, SH, NH₂, NHR^{z}.

Said R^{w}, R^{x}, R^{y} and R^{z} are each independently selected from the group consisting of hydrogen, deuterium, halogen, hydroxyl, NH₂, substituted or unsubstituted C₁₋₄ alkyl, C₁₋₄ haloalkyl, C₄-C₁₀ cycloalkyl alkyl, substituted or unsubstituted C₃-C₆ cycloalkyl.

In another preferred embodiment, said R^{a} is selected from the group consisting of hydrogen, deuterium, halogen, cyano, nitro, hydroxyl, amino, C₁-C₆ alkyl-NH-, (C₁-C₆ alkyl)₂N-, C₁-C₆ alkyl, C₂-C₆ alkenyl, C₂-C₆ alkynyl, C₁-C₆ alkoxy, halogenated C₁-C₆ alkyl, halogenated C₂-C₆ alkenyl, halogenated C₂-C₆ alkynyl, halogenated C₁-C₆ alkoxy, allyl, benzyl, C₆-C₁₂ aryl, C₁-C₆ alkoxy-C₁-C₆ alkyl, C₁-C₆ alkoxy-carbonyl, -(CH₂)ₘN(R⁷)₂, -(CH₂)ₘNH(CH₂)ₚR⁷, -(C_{H2})ₘNHC(O)(CH₂)ₚR⁷; wherein m and p are each independently 0, 1, 2, 3, or 4, preferably 0, 1 or 2.

In another preferred embodiment, R¹ and R⁶ are each independently hydrogen atoms.

In another preferred embodiment, X is independently selected from N, CH.

In another preferred embodiment, X is independently selected from CH.

In another preferred embodiment, said R⁴ and R⁵ are each each independently selected from the group consisting of hydrogen, deuterium, halogen, hydroxyl, NH₂, substituted or unsubstituted C₁-C₄ alkyl, substituted or unsubstituted C₁-C₄ alkoxy, cyclopropyl;
or R⁴ and R⁵ together with the carbon atoms to which they are attached form an oxa-5-6 membered heterocycle that is unsubstituted or substituted by one or more R^{a}; wherein R^{a} is defined as described above.

In another preferred embodiment, R² is selected from the group consisting of hydrogen, deuterium, halogen, NH₂, substituted or unsubstituted C₁-C₈ alkyl, substituted or unsubstituted C₁-C₈ deuterated alkyl, -(CH₂)ₘ(C₃-C₆ cycloalkyl), -(CH₂)ₘ(3-6 membered heterocyclyl), -(CH₂)ₘN(R⁷)₂, and -(CH₂)ₘOC(O)R⁷; wherein m is 0, 1, 2, 3, or 4;
R³ is selected from the group consisting of hydrogen, deuterium, halogen, substituted or unsubstituted C₁-C₈ alkyl, substituted or unsubstituted C₁-C₈ deuterated alkyl, -(CH₂)ₘ(C₃-C₆ cycloalkyl), -(CH₂)ₘ(3-6 membered heterocyclyl), -(CH₂)ₘN(R⁷)₂, and -(CH₂)ₘOC(O) R⁷; wherein m is 0, 1, 2, 3 or 4;
or, R² and R³ together with the carbon atoms to which they are attached form a structure selected from the group consisting of a saturated or unsaturated 5-6 membered carbocycle that is unsubstituted or substituted by one or more R^{a}, and a saturated or unsaturated 5-6 membered heterocycle that is unsubstituted or substituted by one or more R^{a};
R⁴ is selected from the group consisting of hydrogen, deuterium, halogen, hydroxyl, cyano, NH₂, NO₂, substituted or unsubstituted C₁-C₈ alkyl, and substituted or unsubstituted C₁-C₈ alkoxy;
R⁵ is selected from the group consisting of hydrogen, deuterium, halogen, and substituted or unsubstituted C₁-C₈ alkyl;
or R⁴ and R⁵ are linked together to form a structure selected from the group consisting of -OCH₂O- or -O(CH₂)₂O-, and the structure is unsubstituted or substituted by one or more R^{a};
R⁷ is selected from the group consisting of hydrogen, deuterium, halogen, substituted or unsubstituted C₁-C₈ alkyl, hydroxyl, amino, cyano, nitro, and thiol;
wherein R^{a} is defined as described above.

In another preferred embodiment, said compound has the structure as shown in the following formulae:

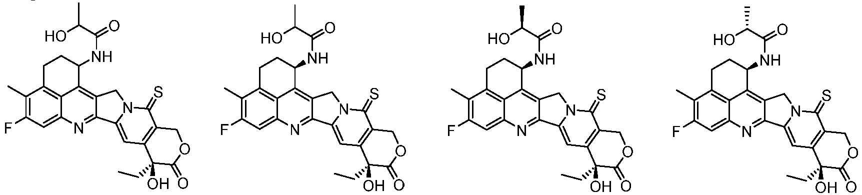

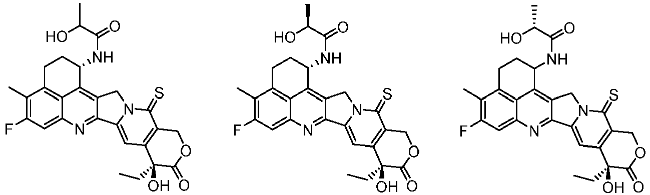

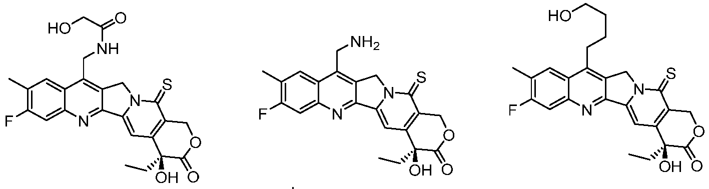

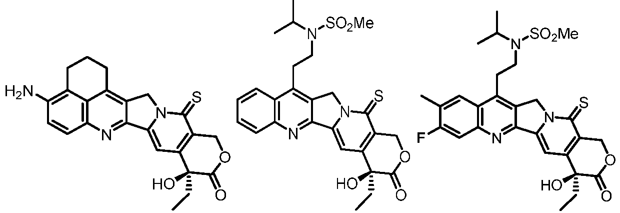

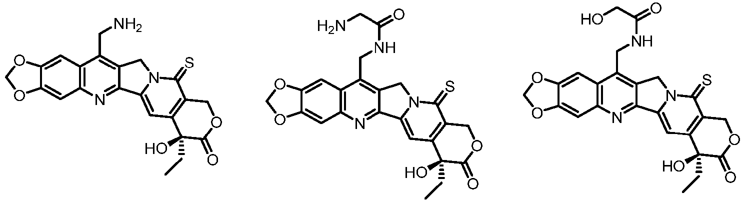

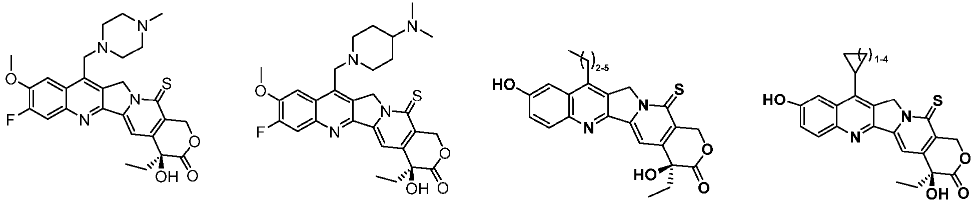

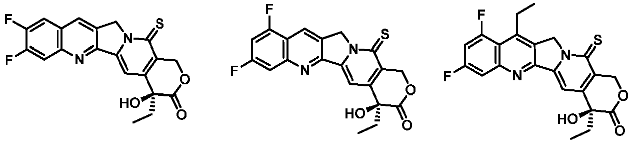

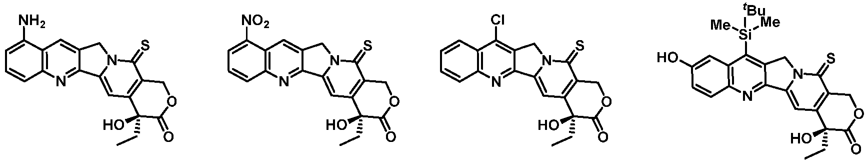

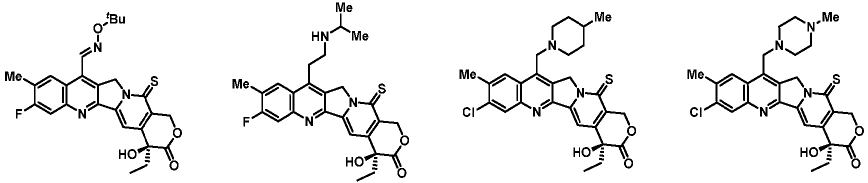

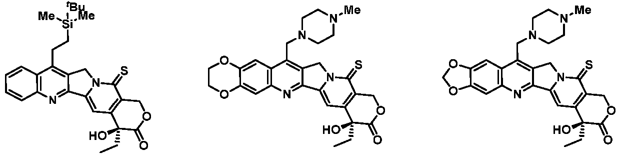

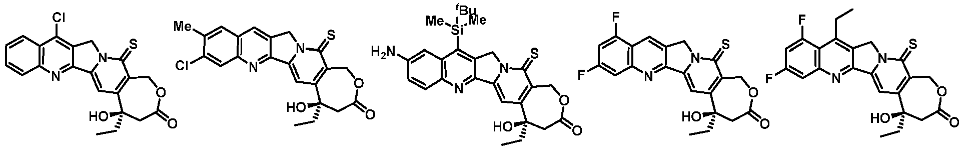

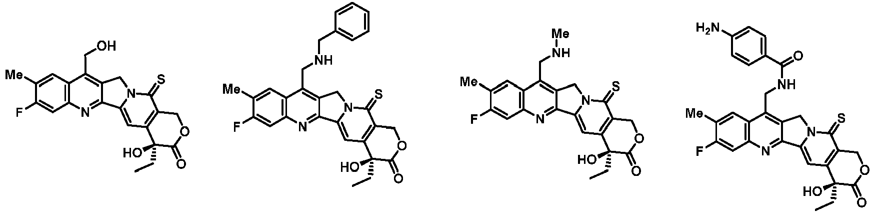

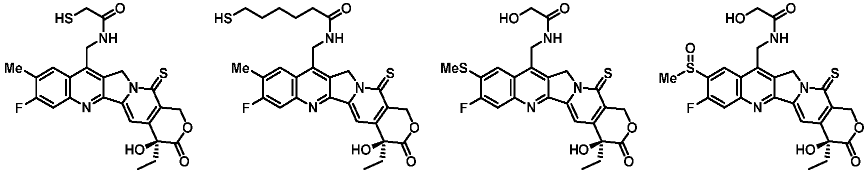

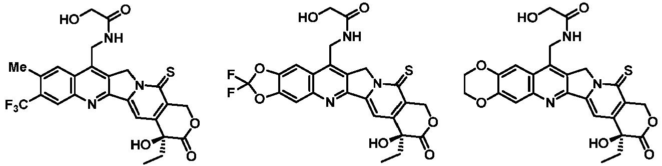

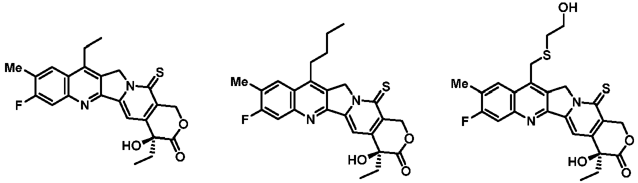

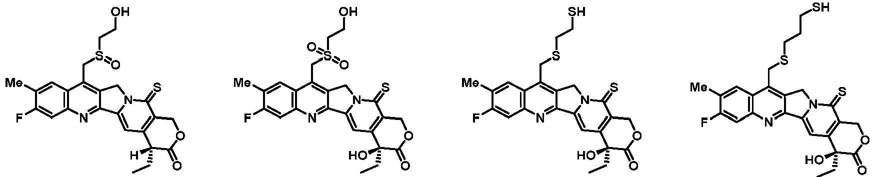

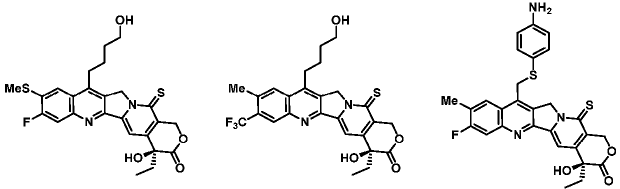

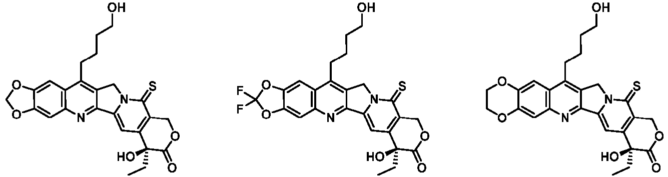

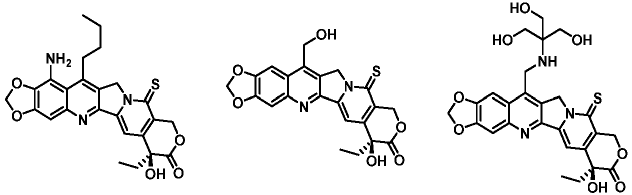

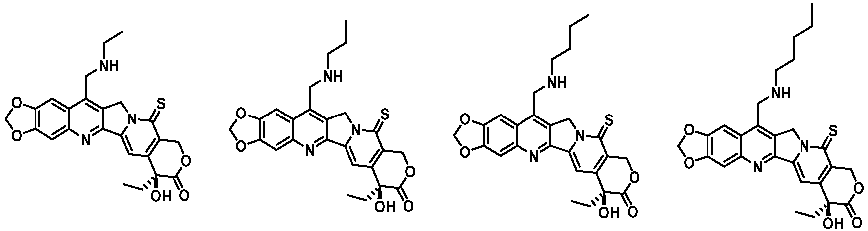

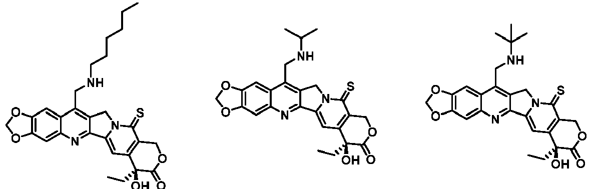

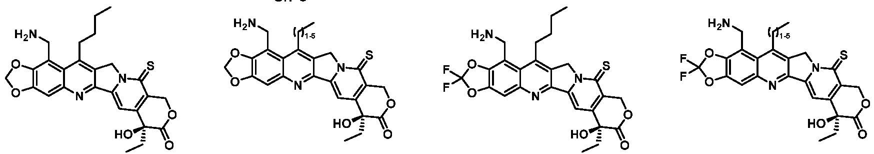

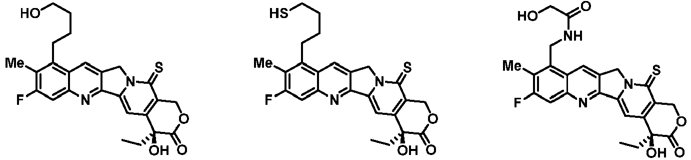

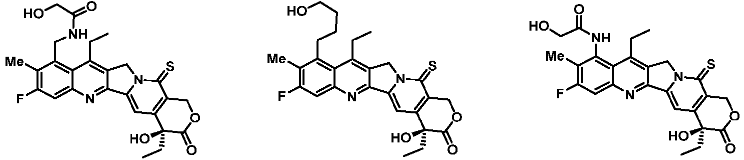

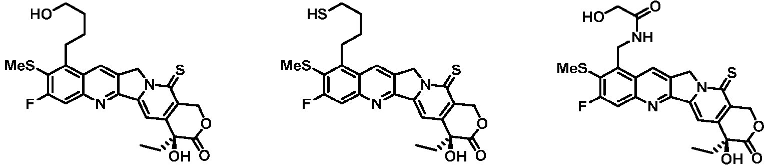

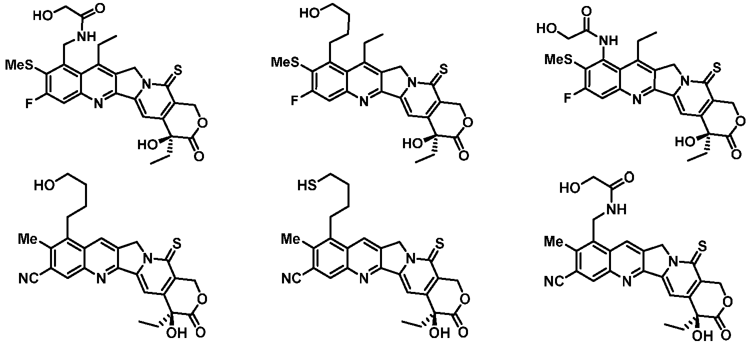

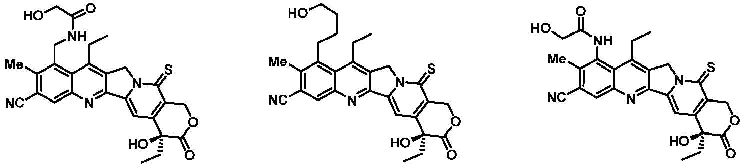

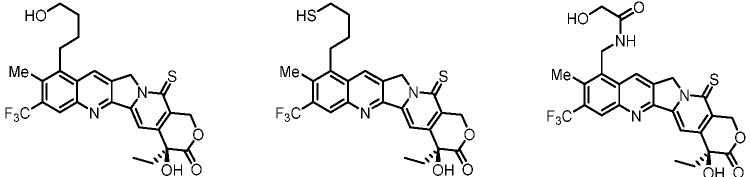

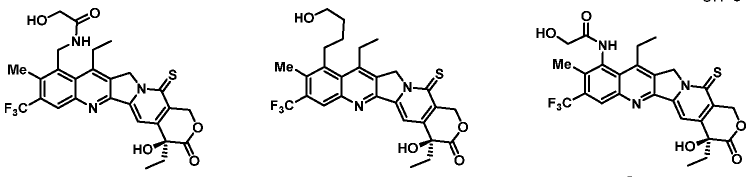

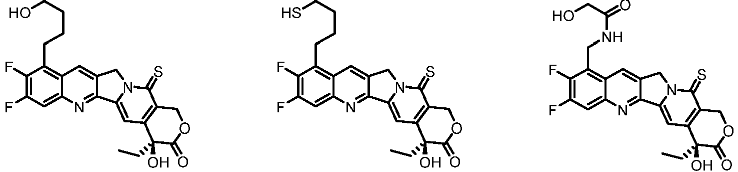

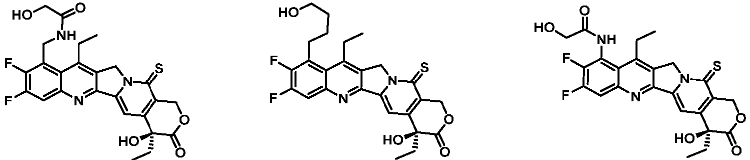

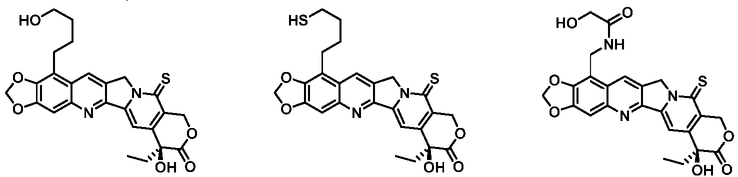

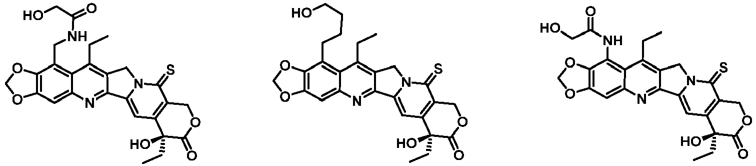

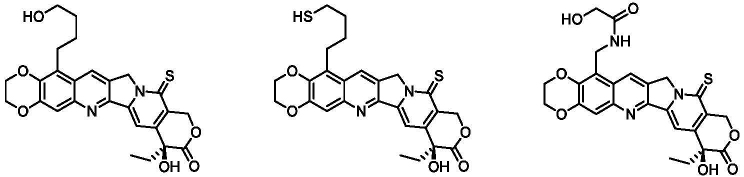

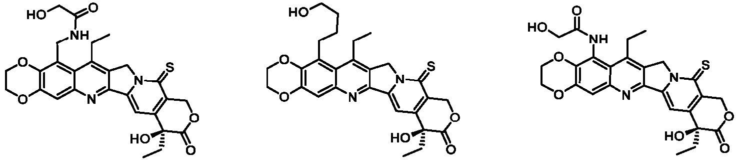

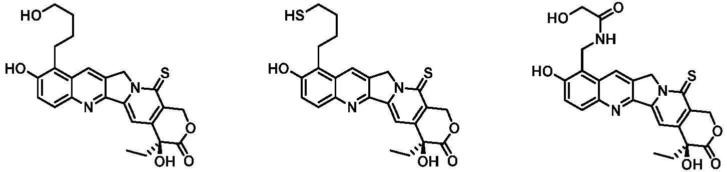

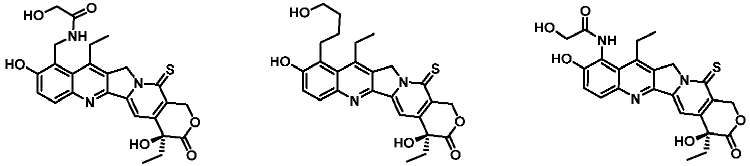

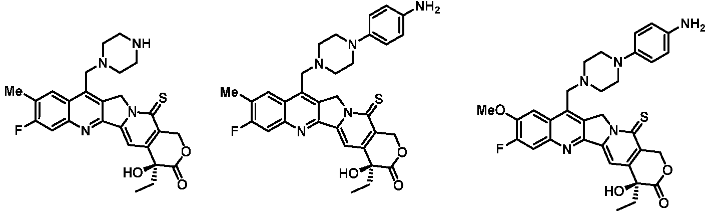

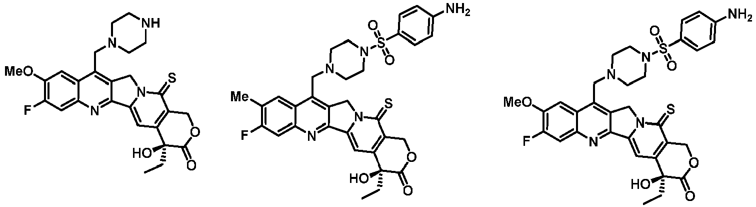

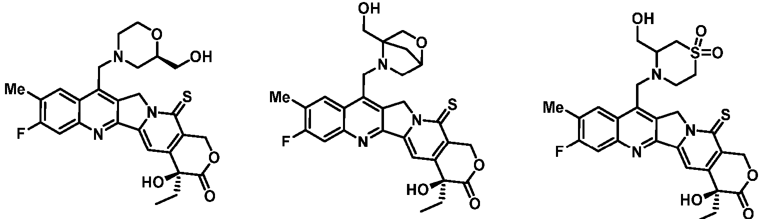

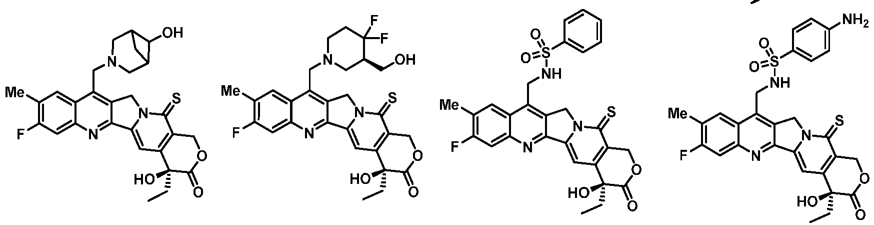

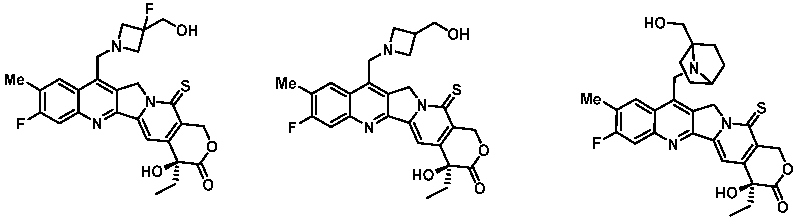

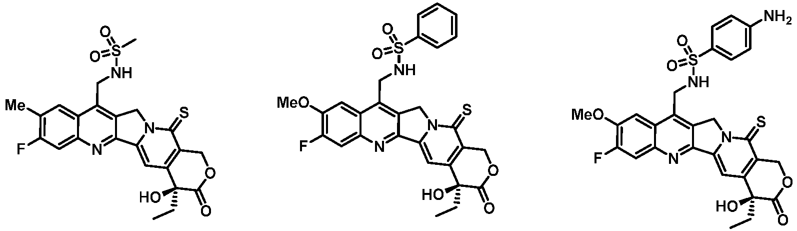

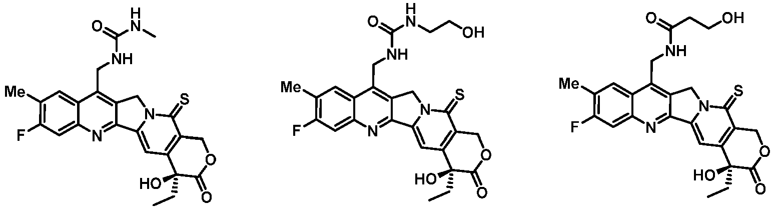

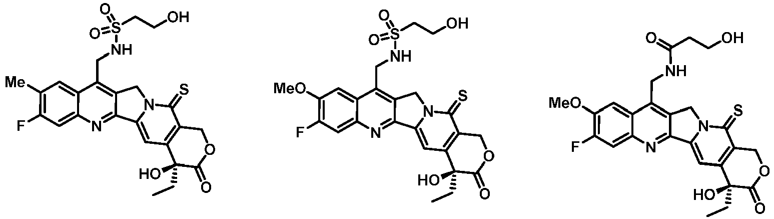

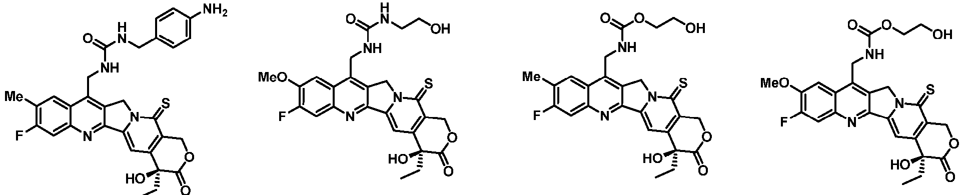

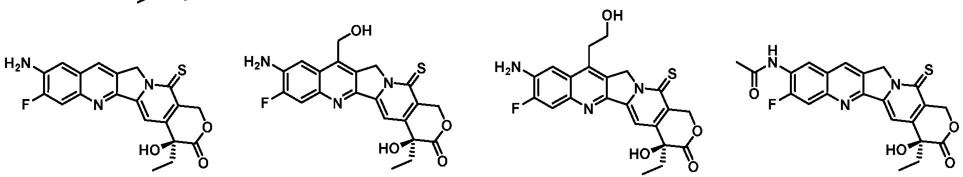

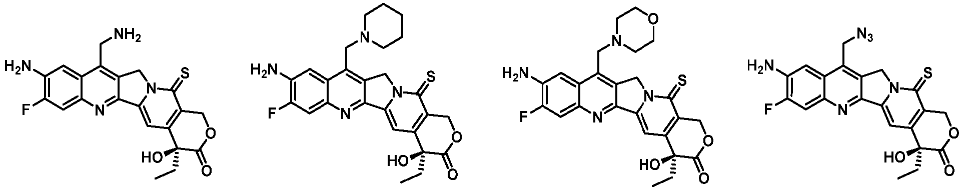

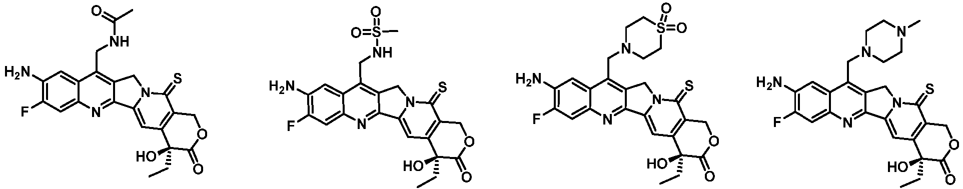

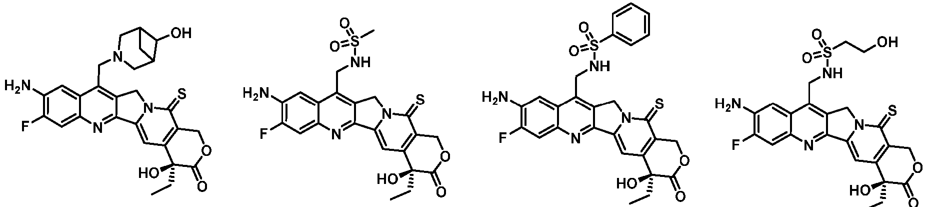

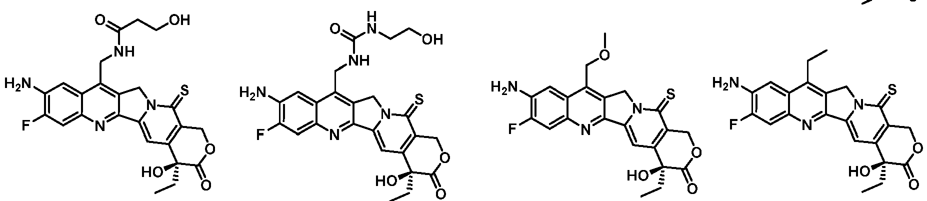

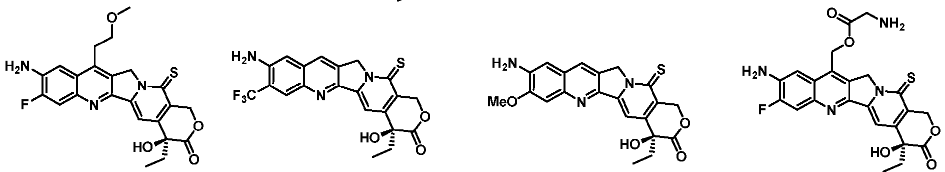

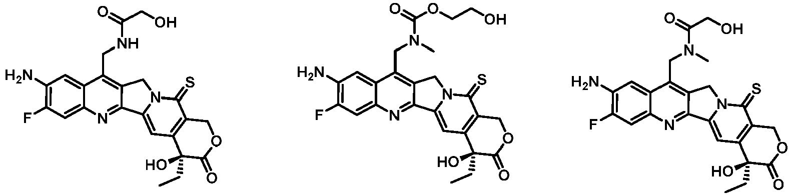

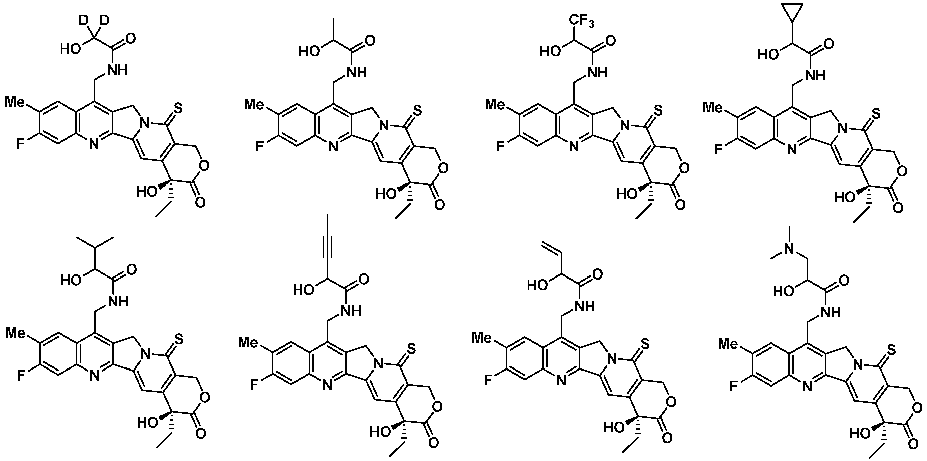

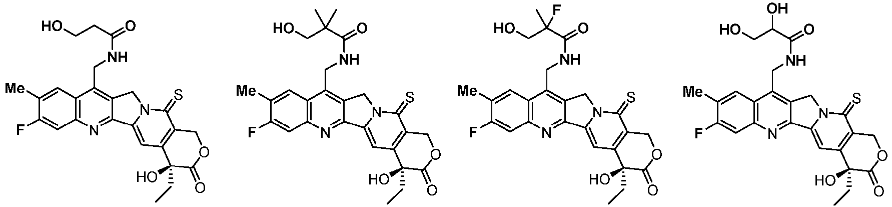

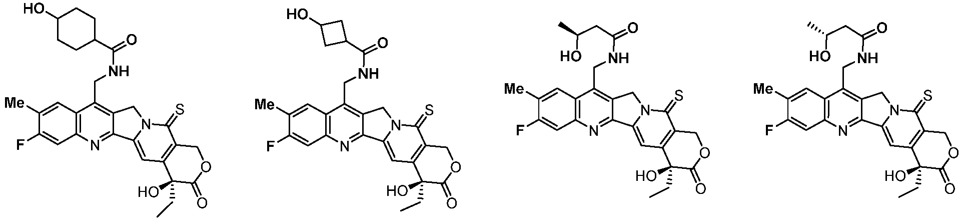

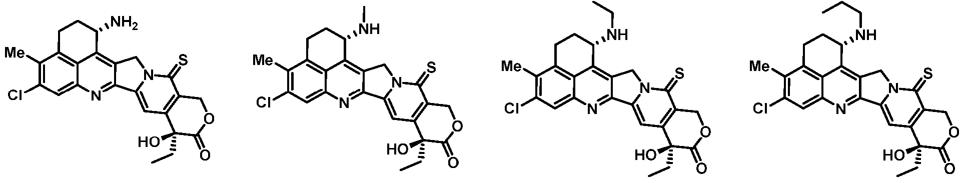

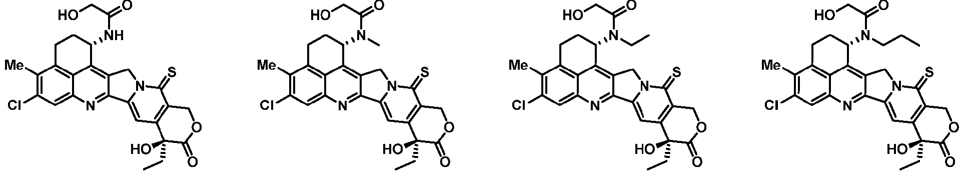

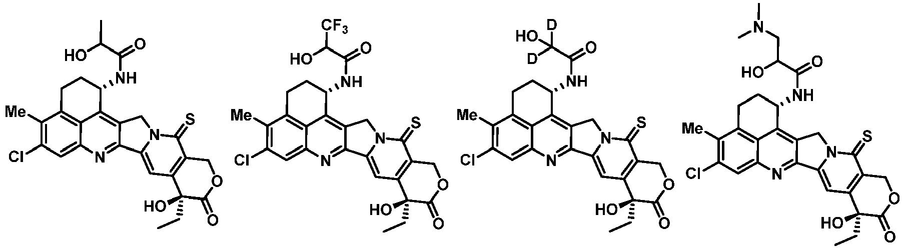

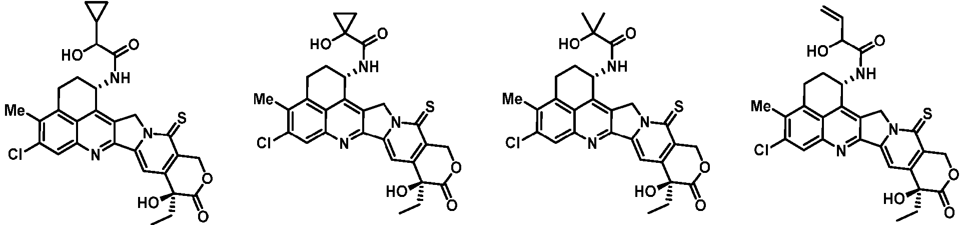

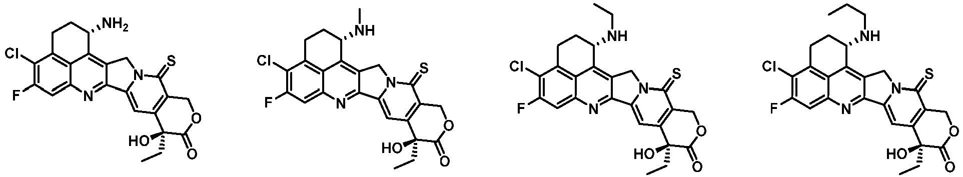

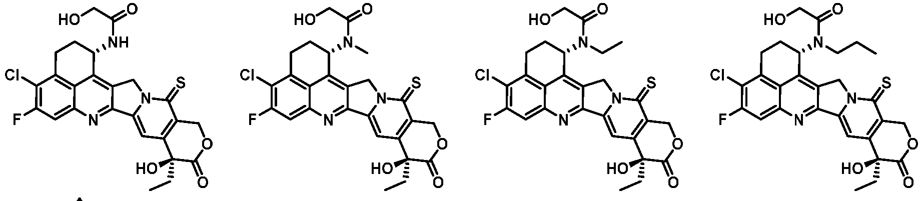

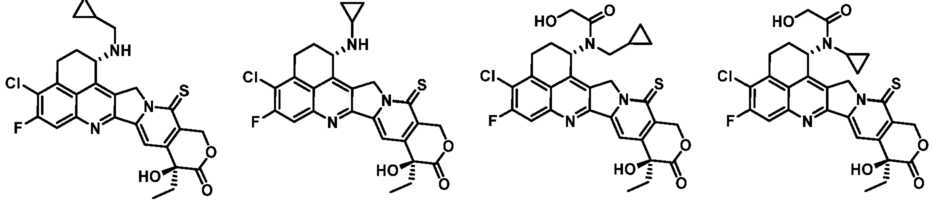

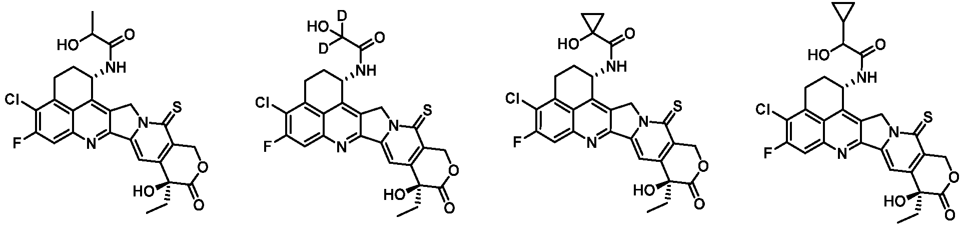

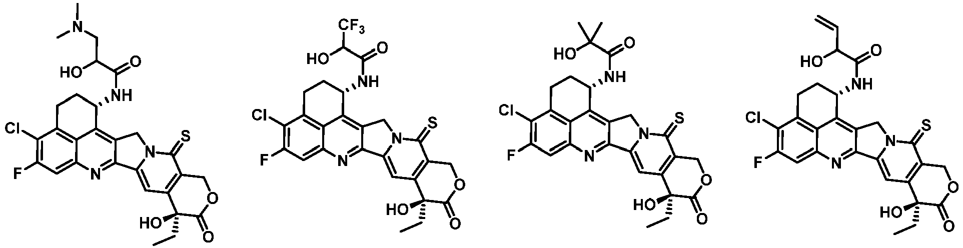

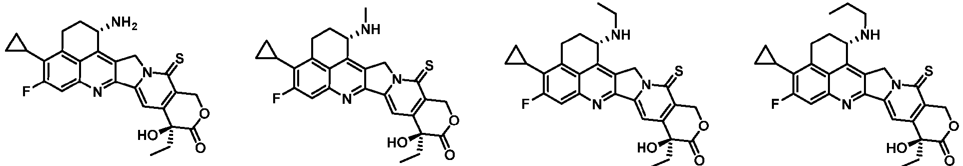

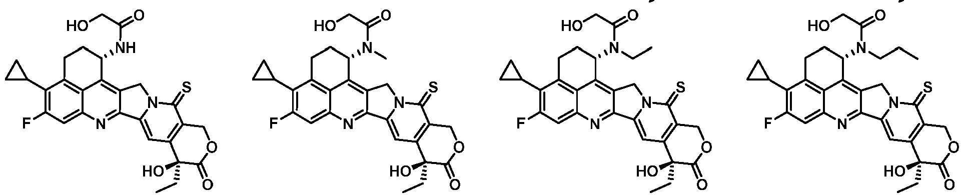

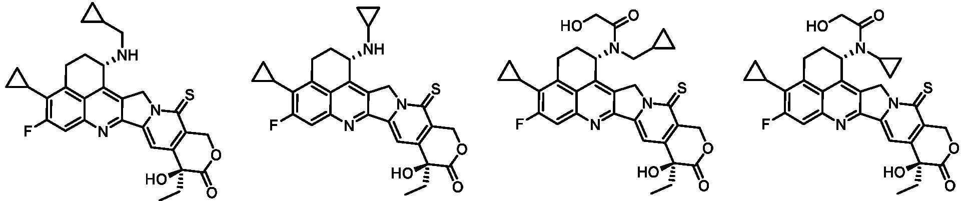

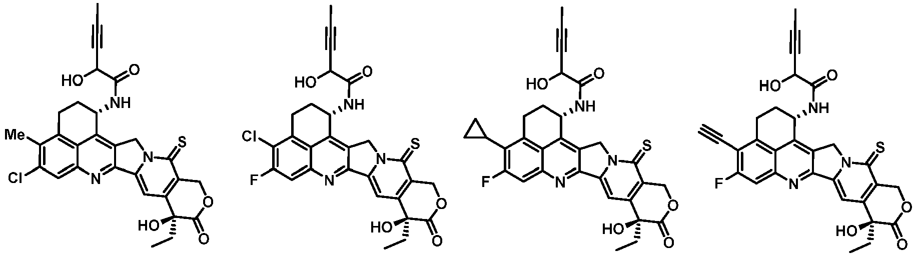

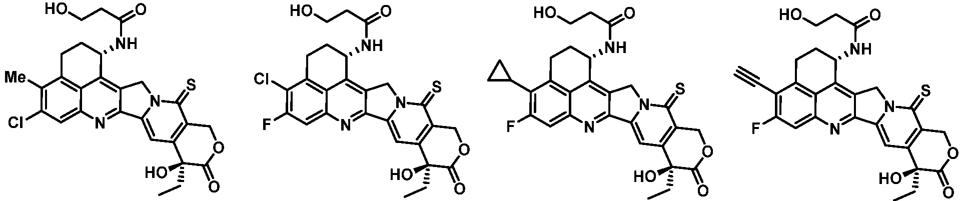

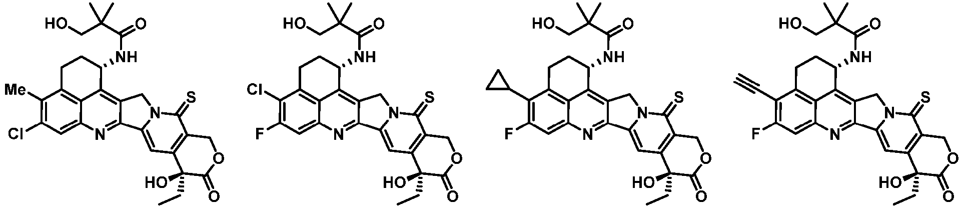

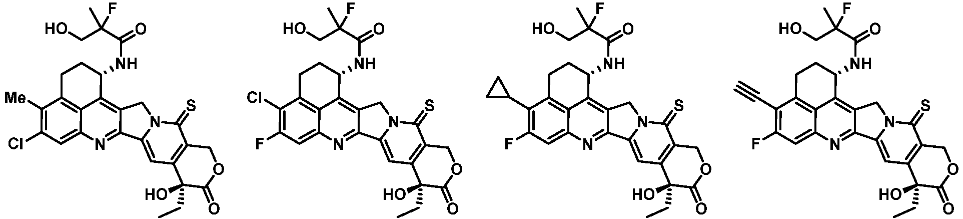

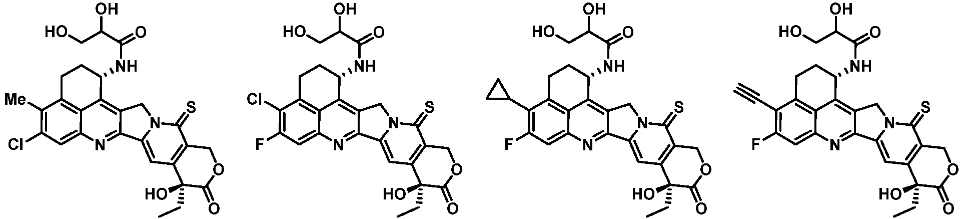

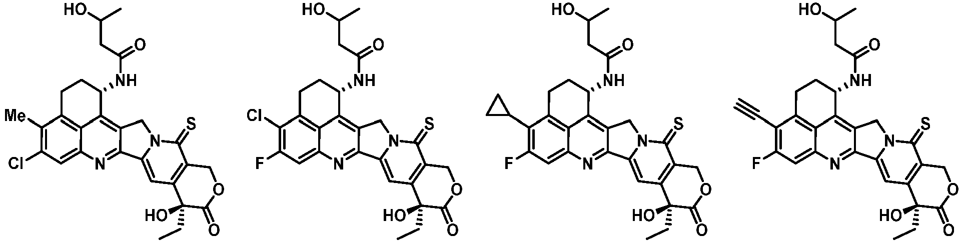

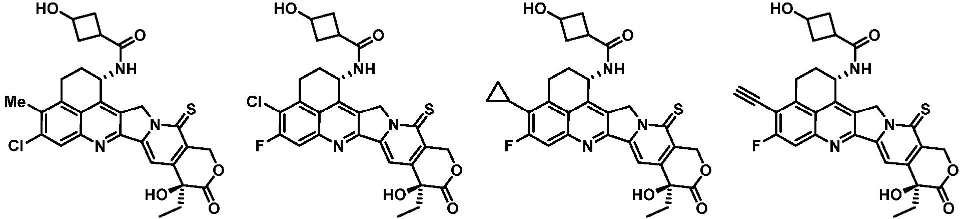

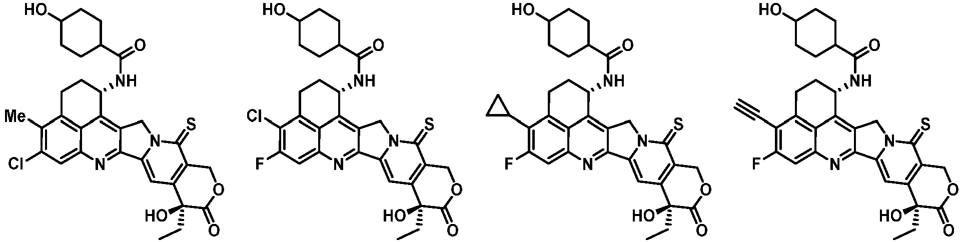

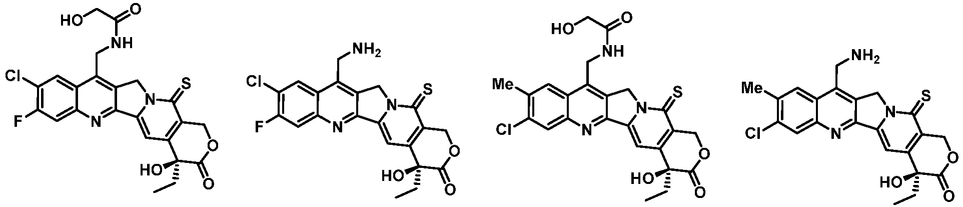

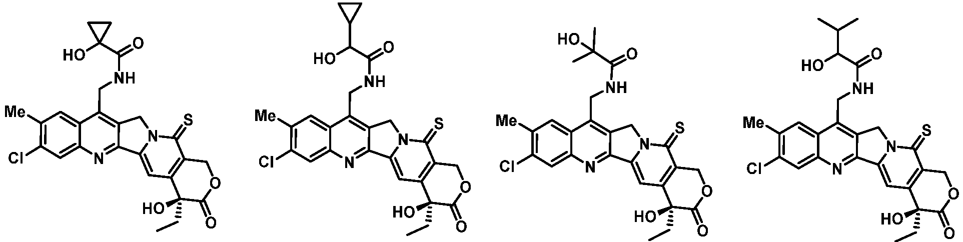

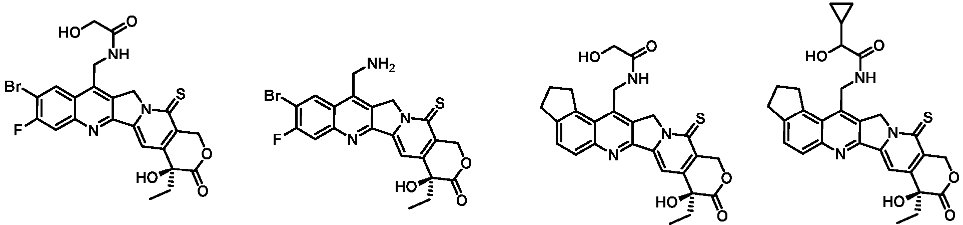

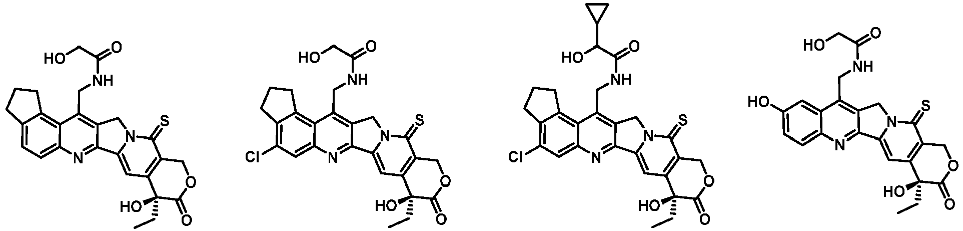

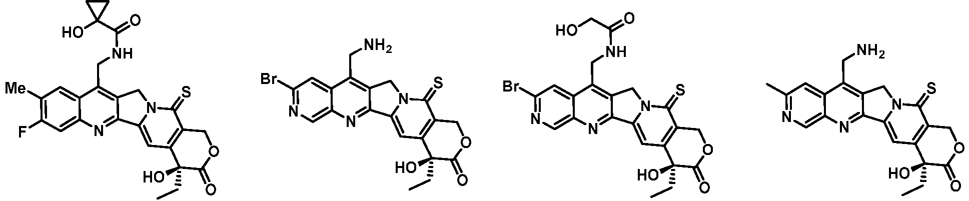

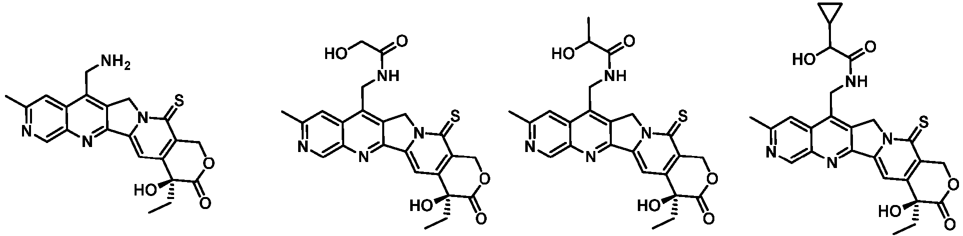

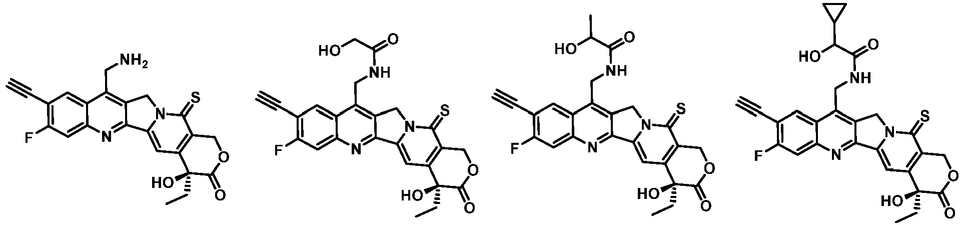

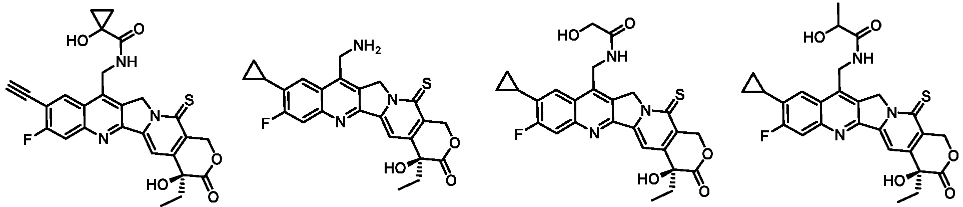

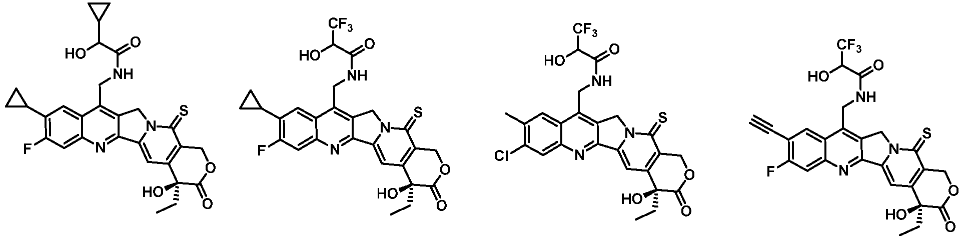

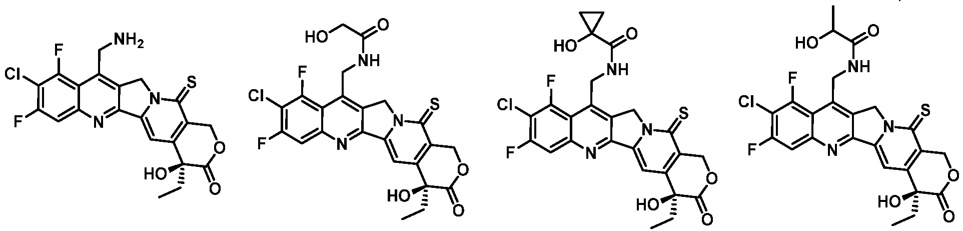

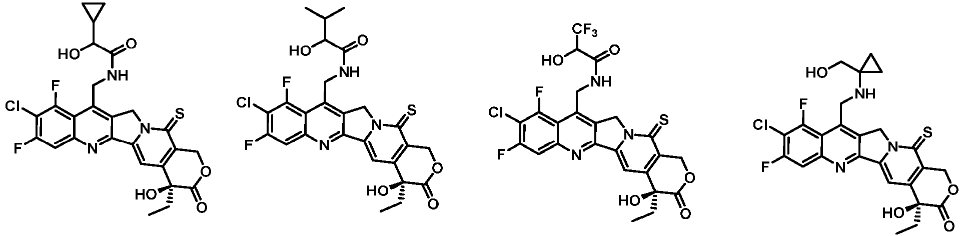

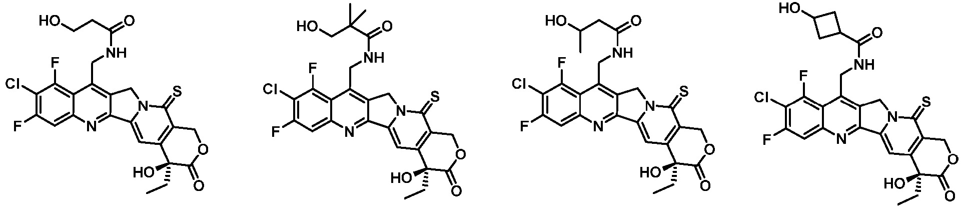

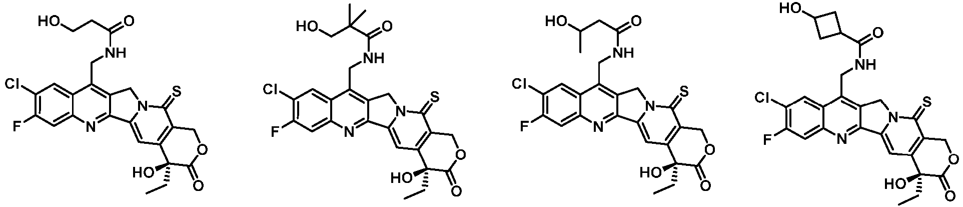

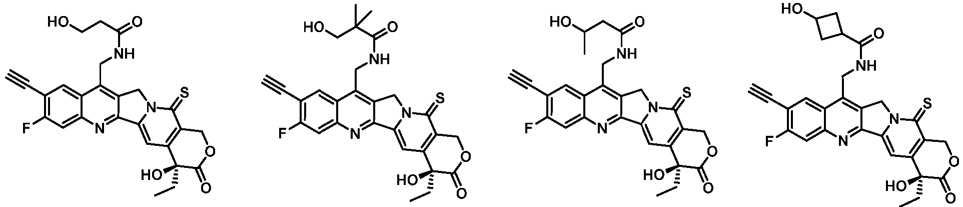

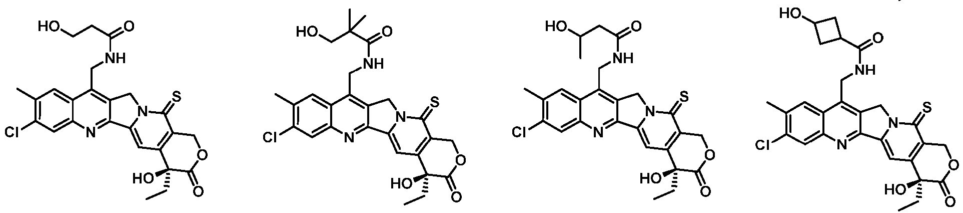

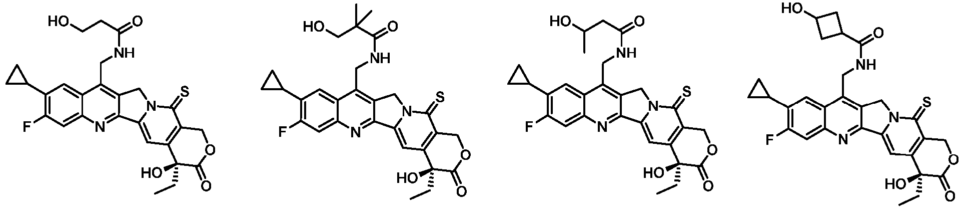

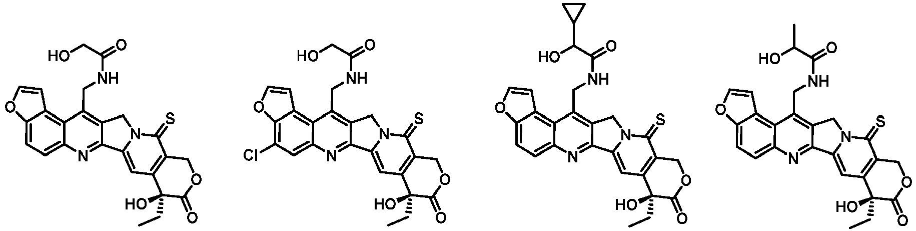

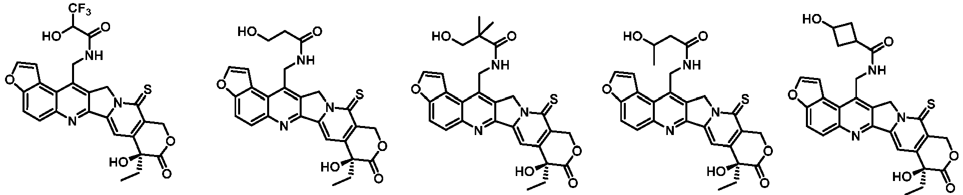

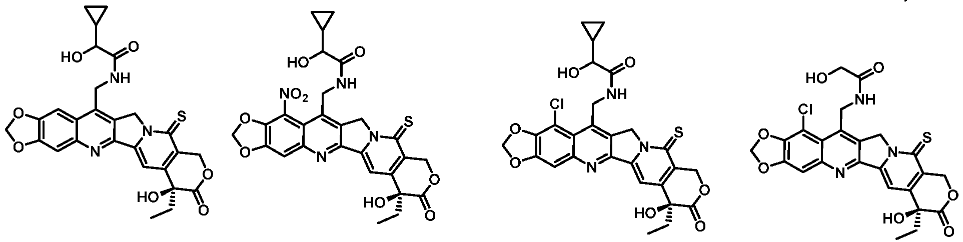

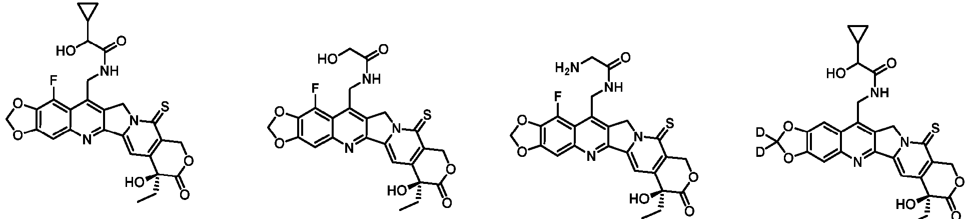

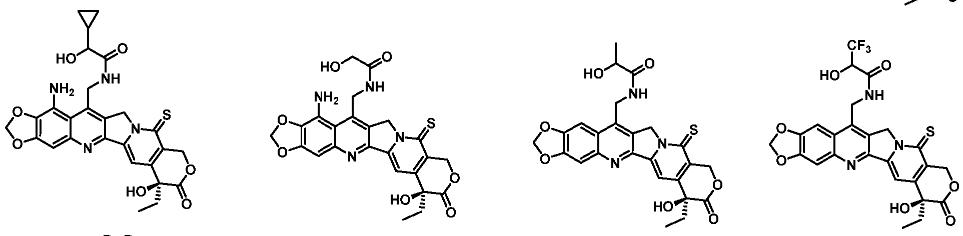

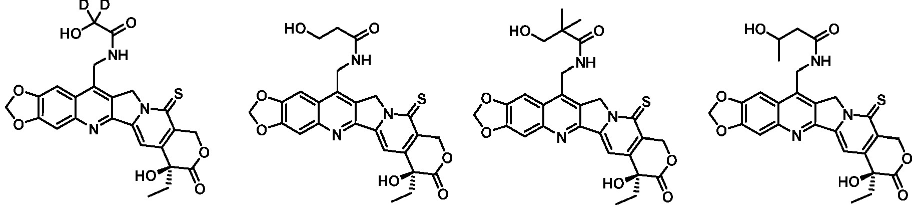

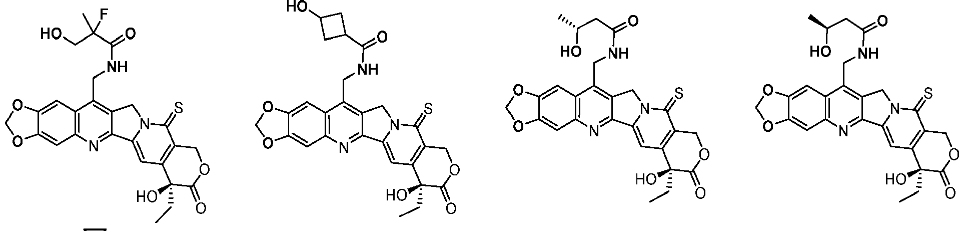

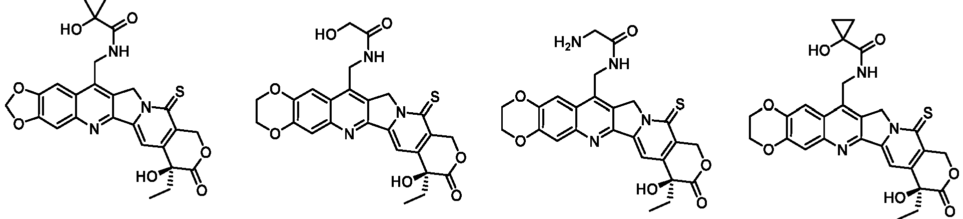

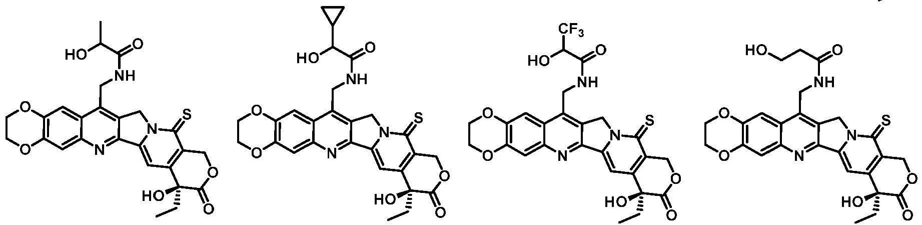

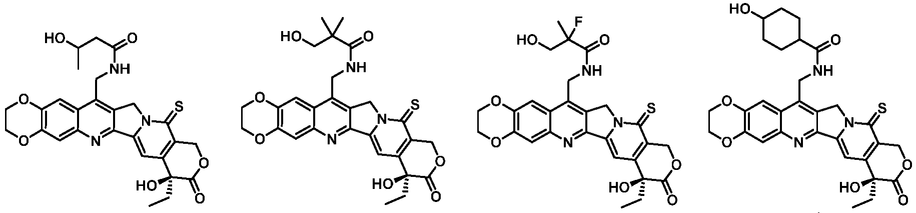

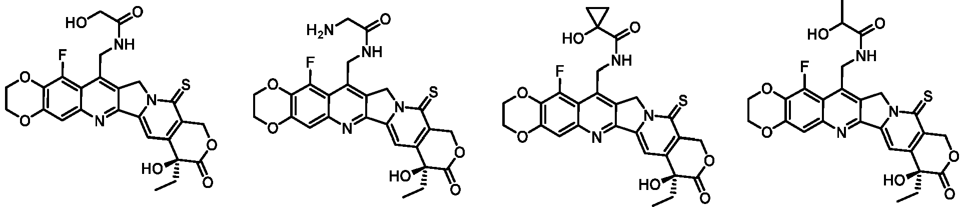

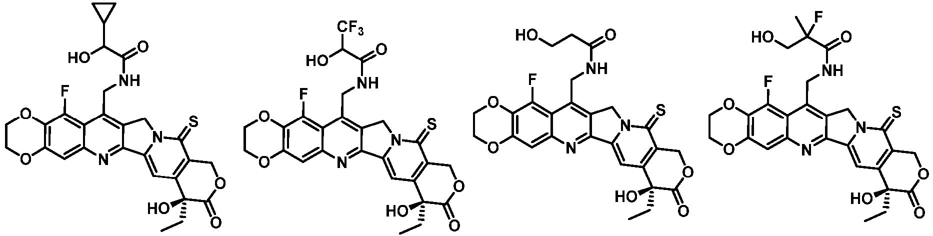

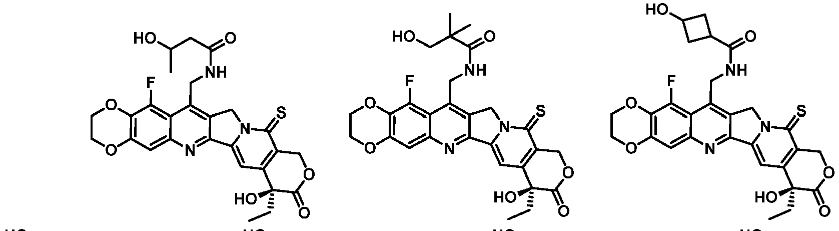

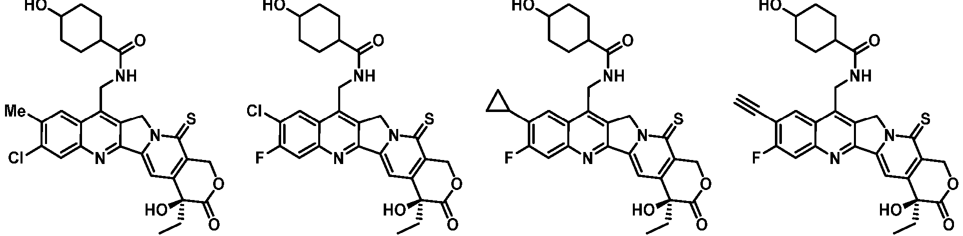

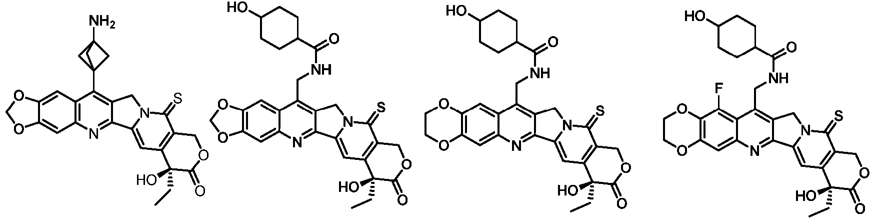

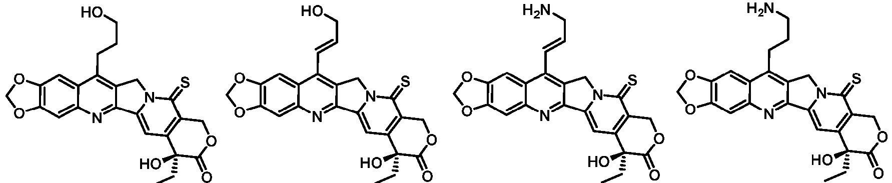

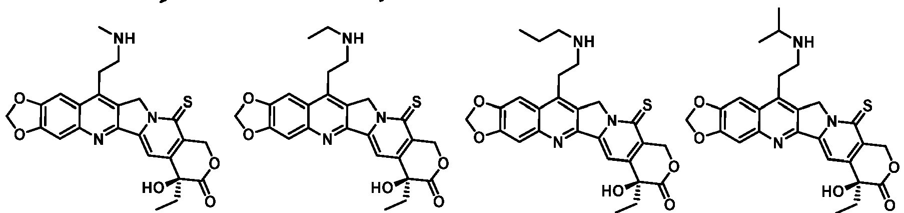

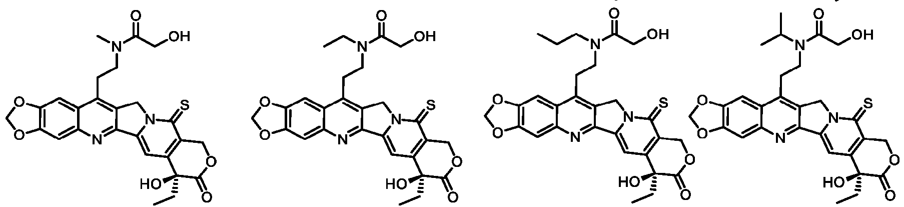

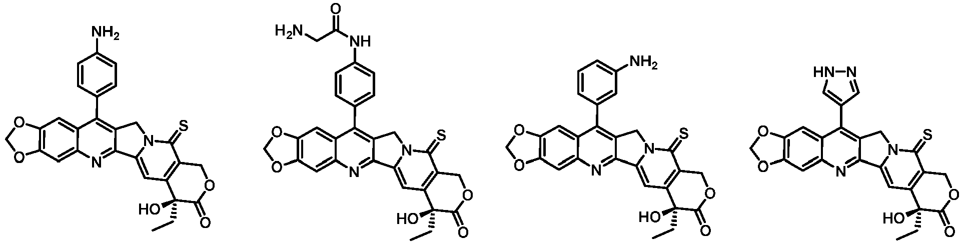

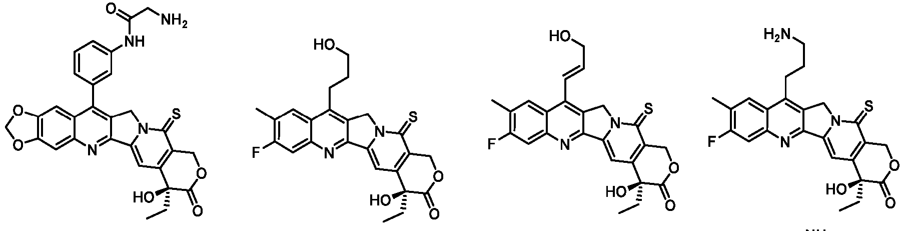

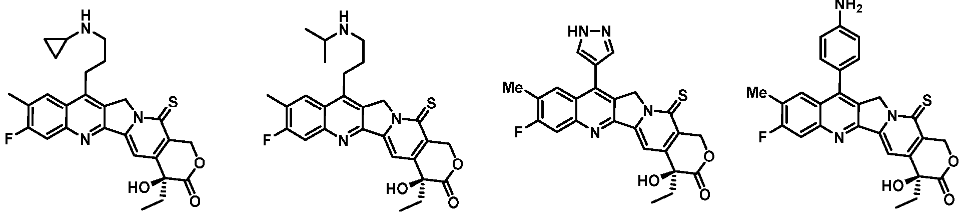

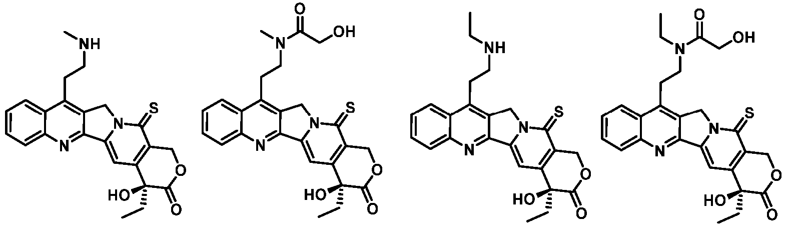

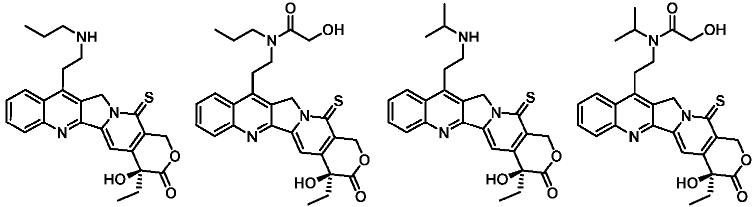

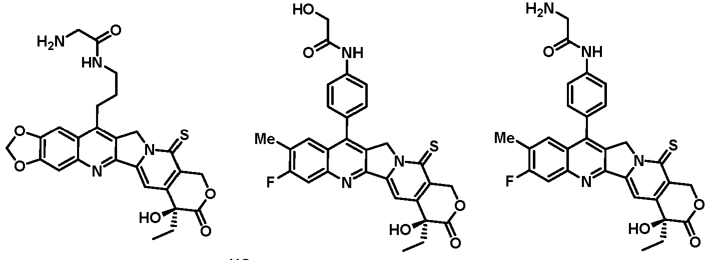

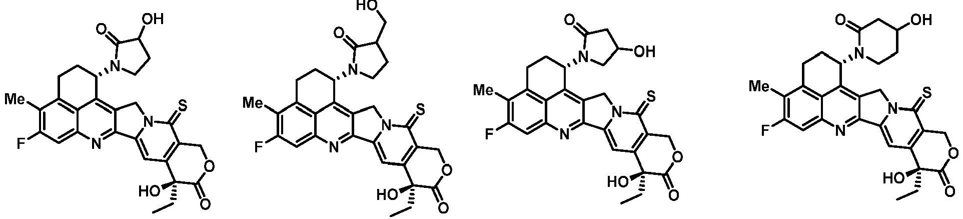

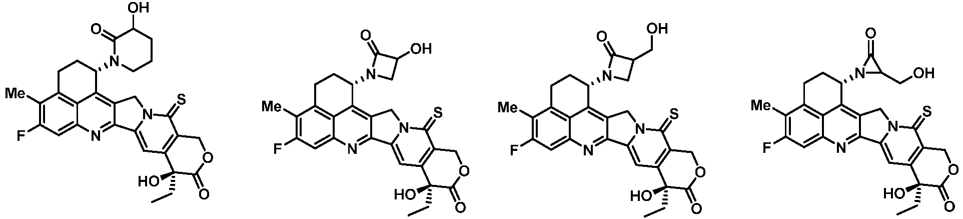

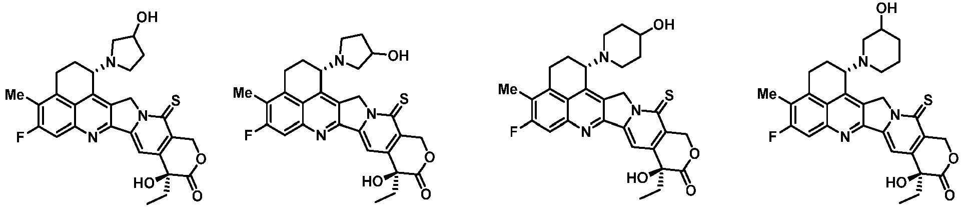

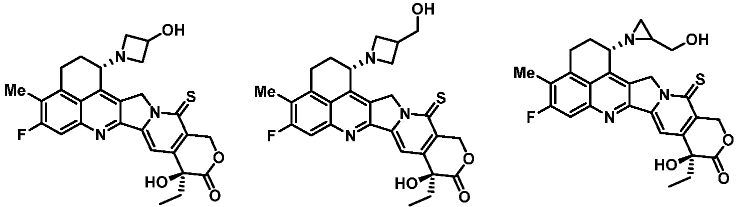

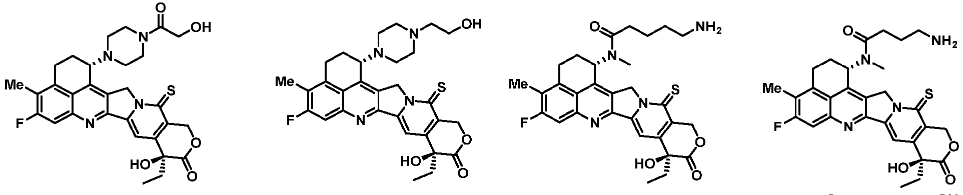

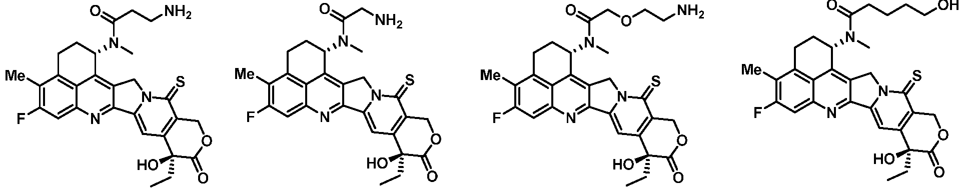

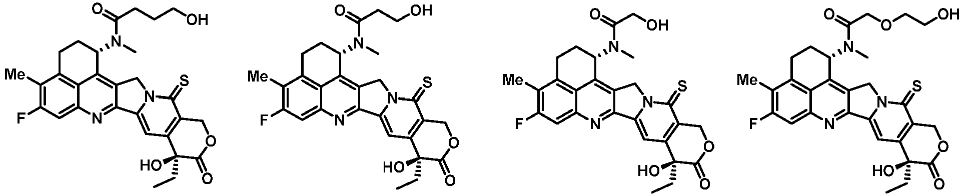

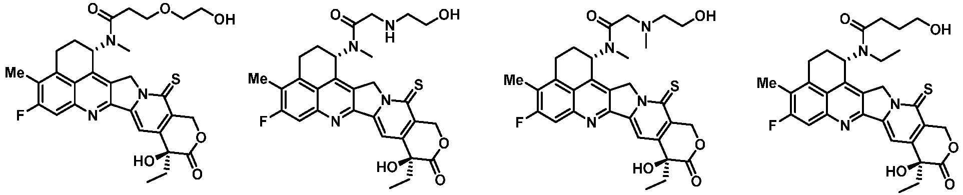

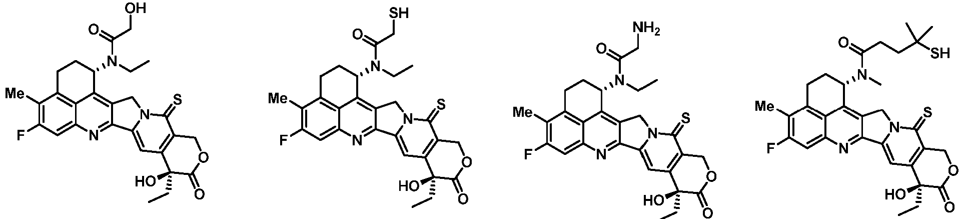

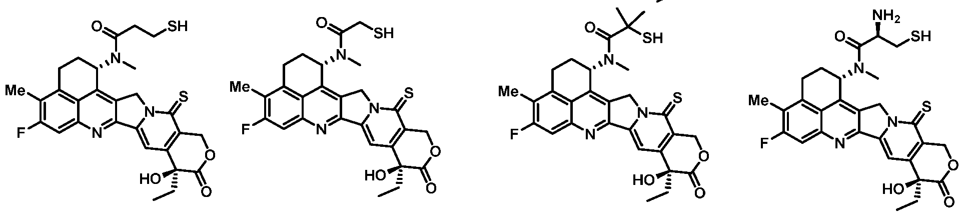

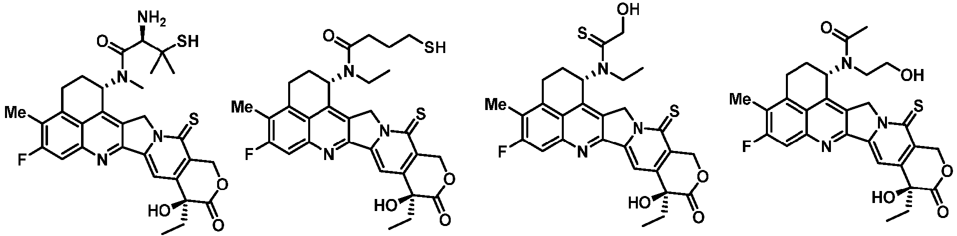

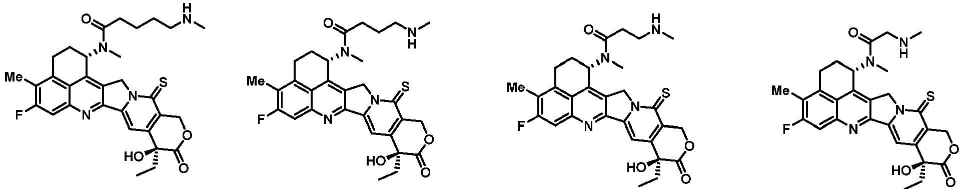

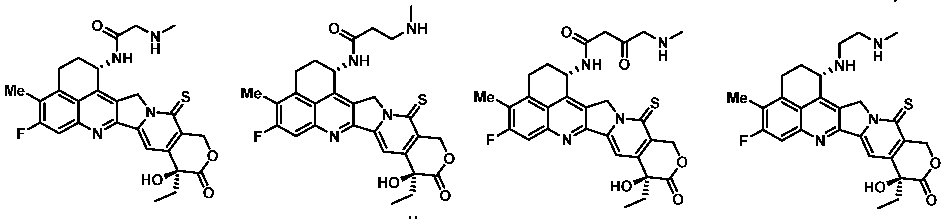

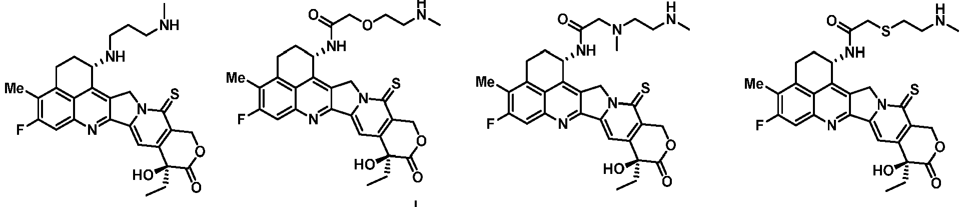

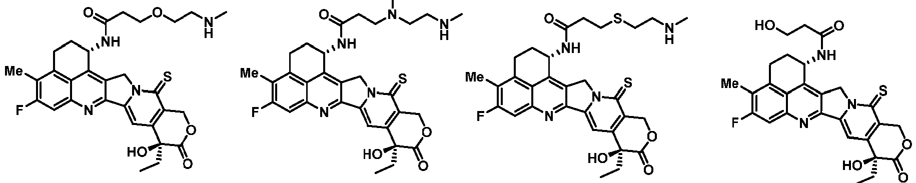

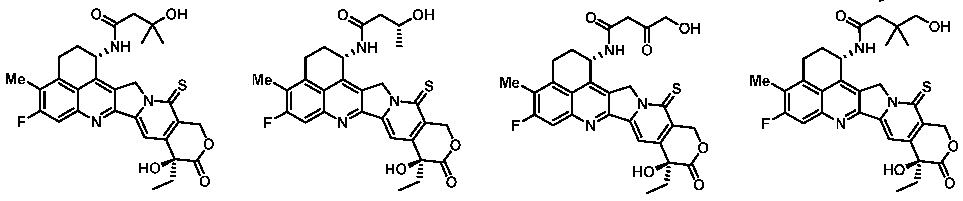

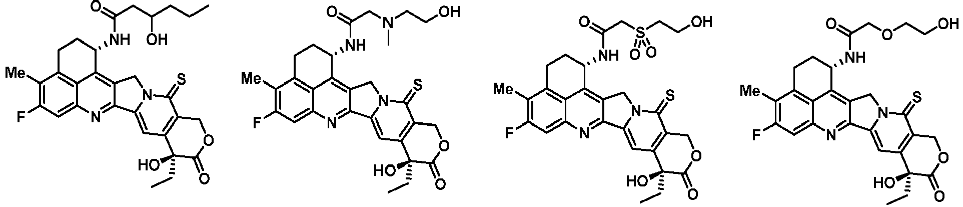

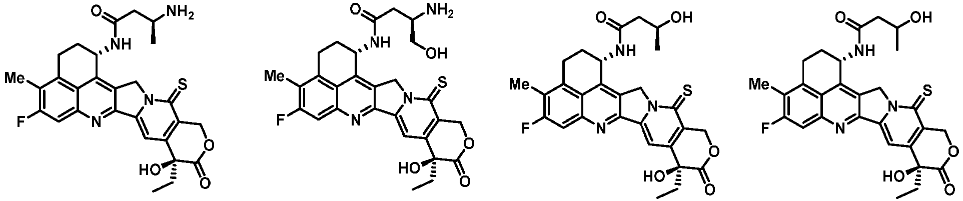

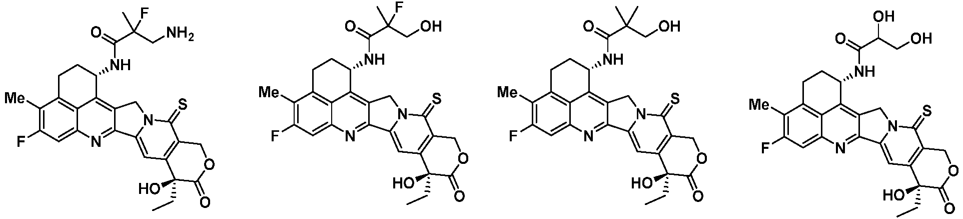

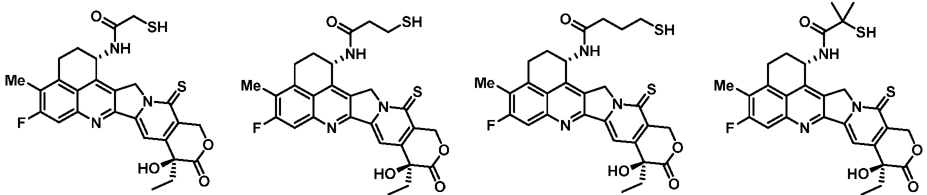

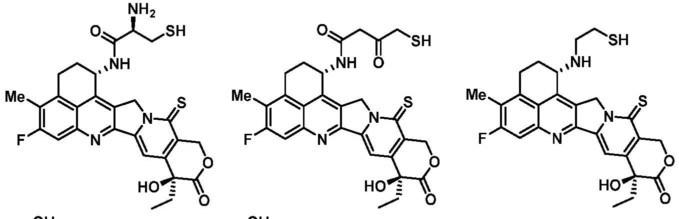

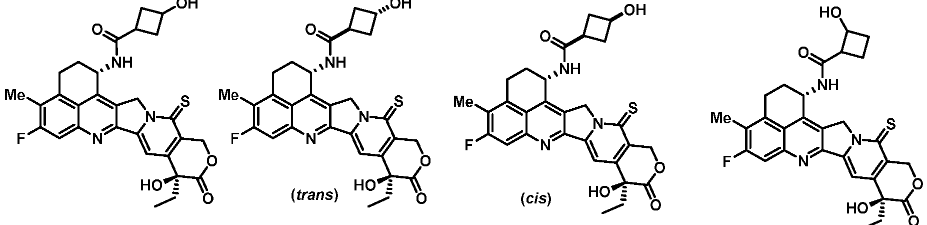

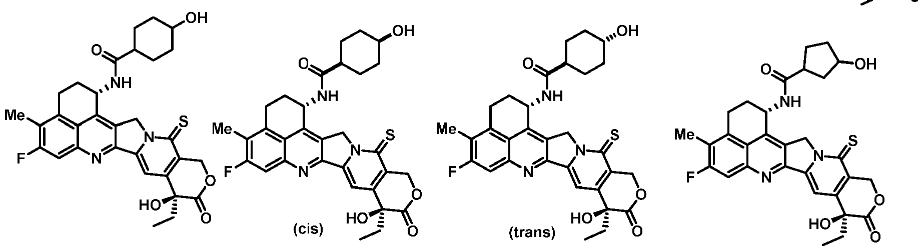

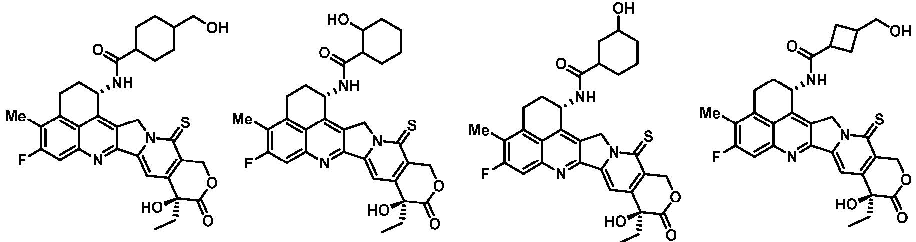

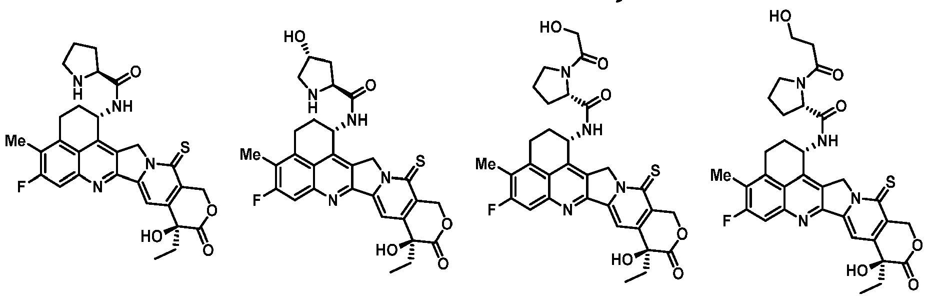

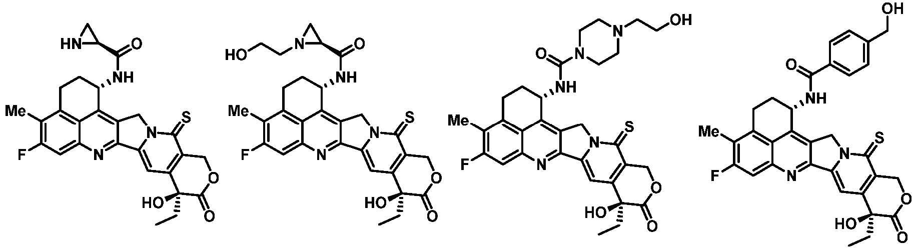

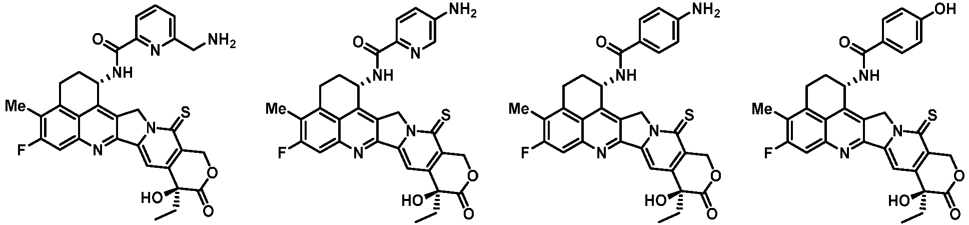

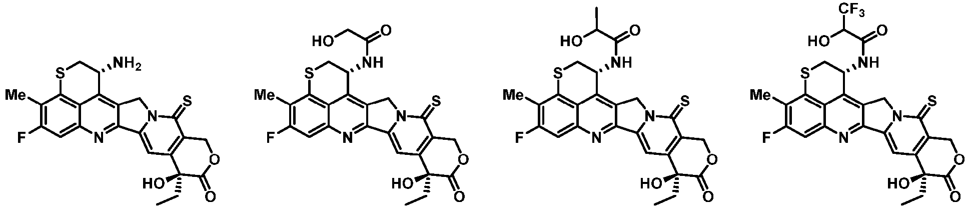

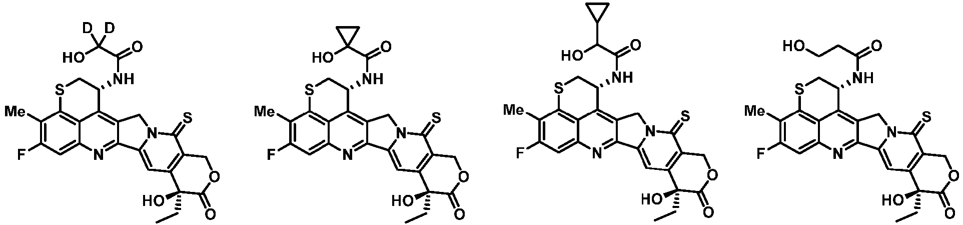

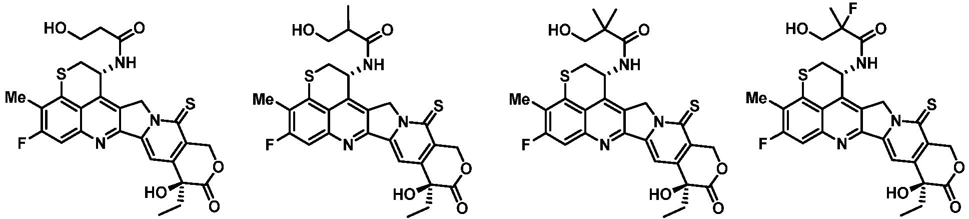

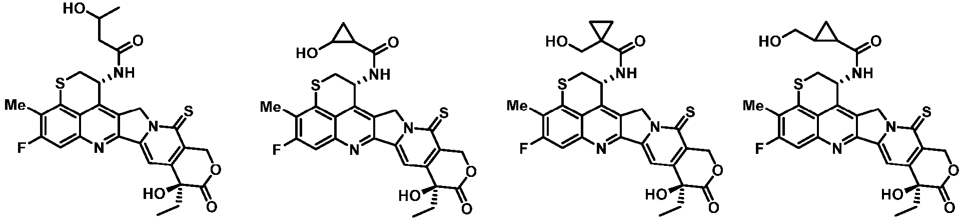

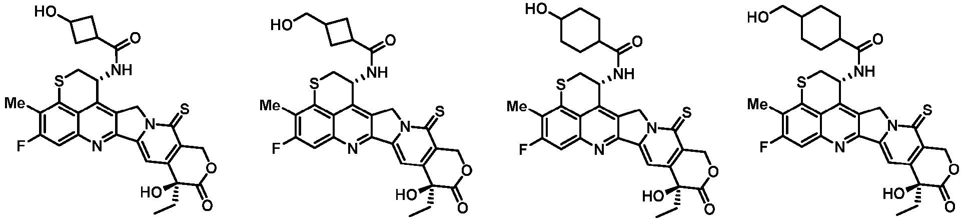

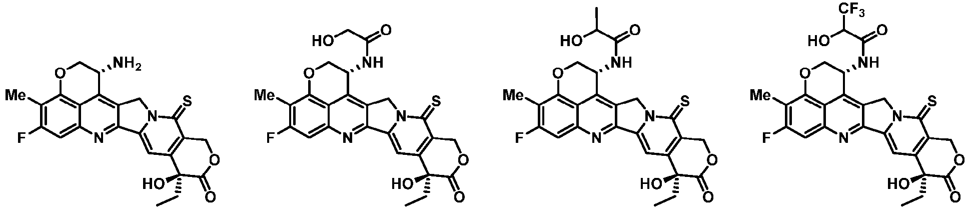

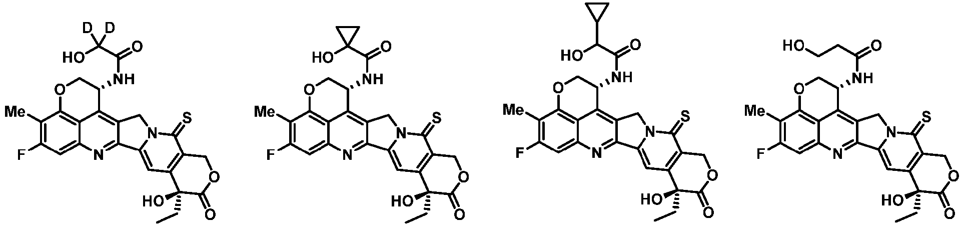

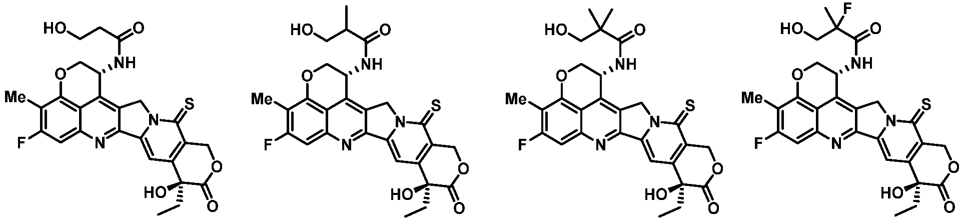

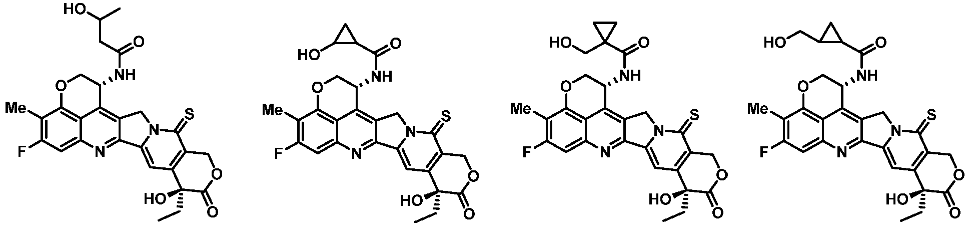

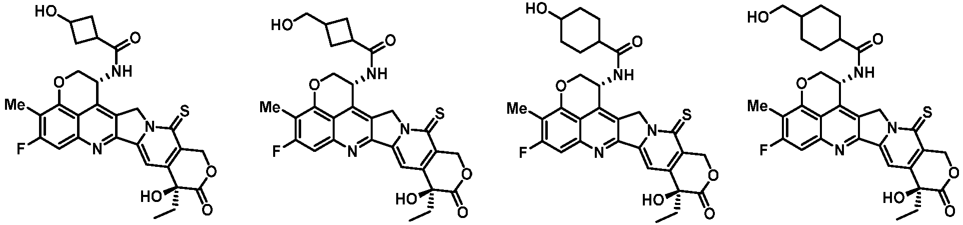

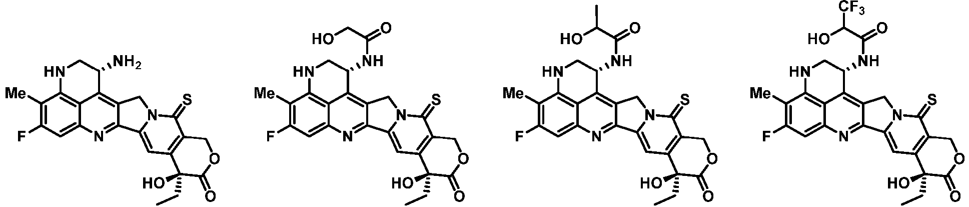

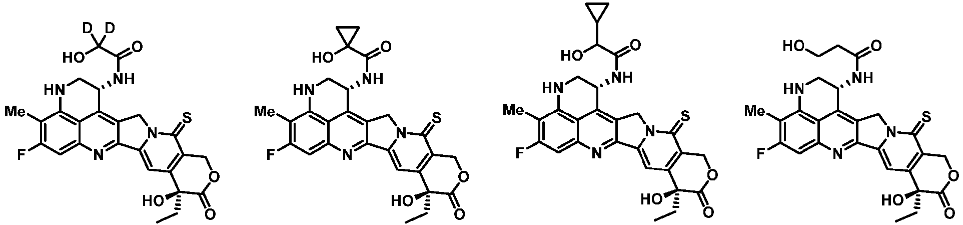

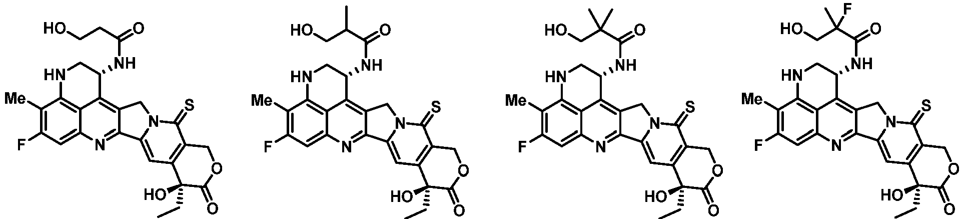

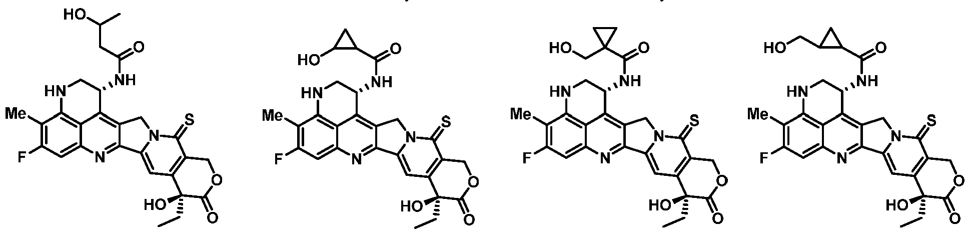

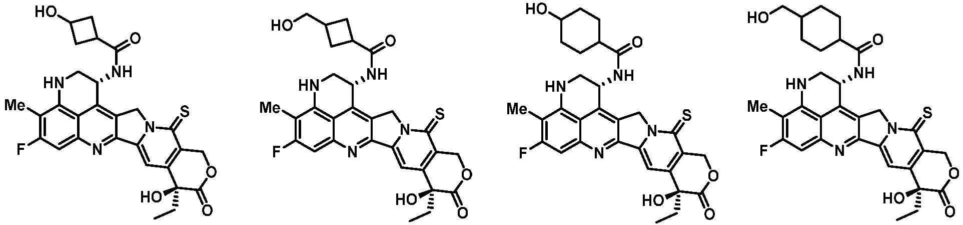

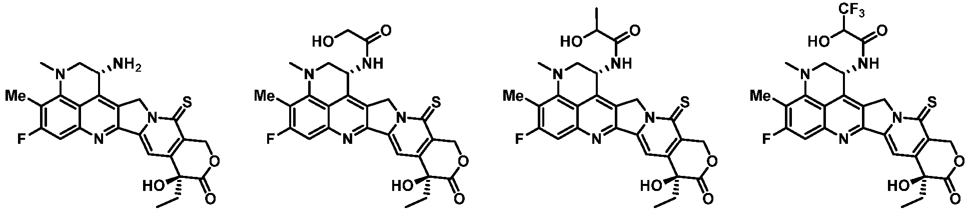

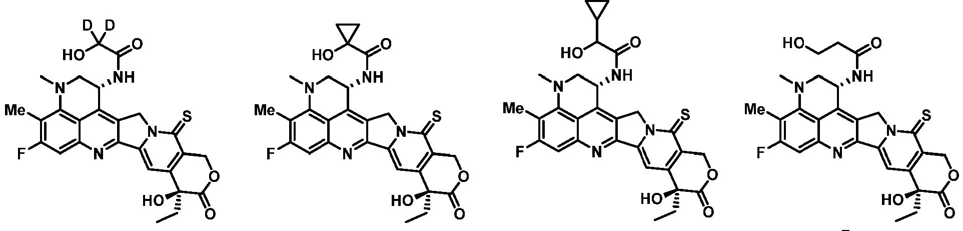

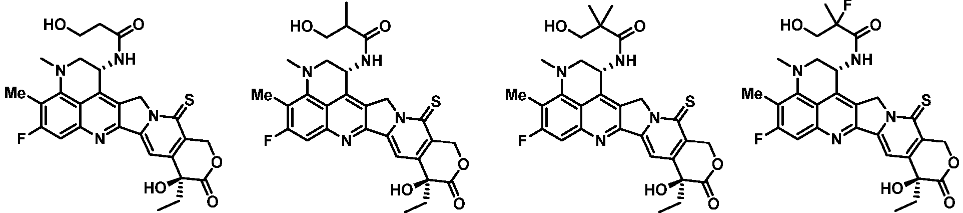

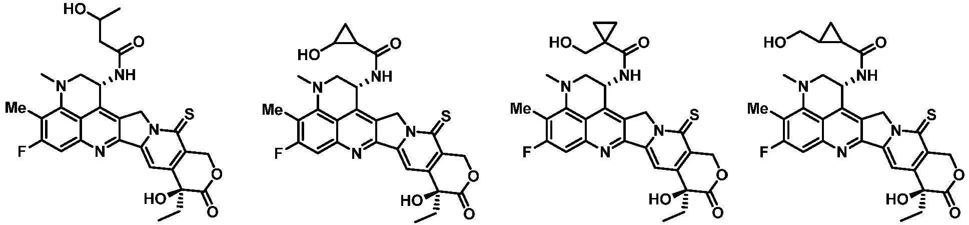

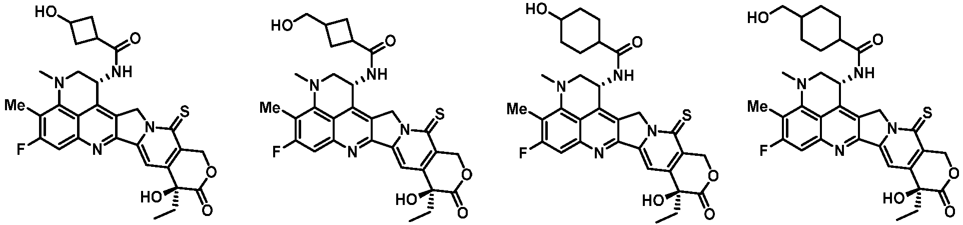

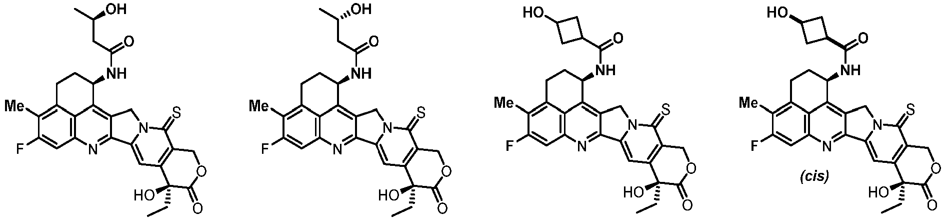

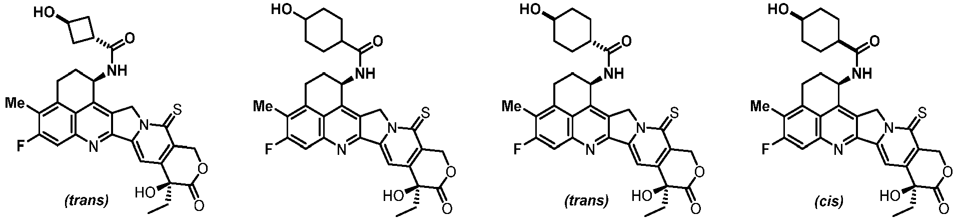

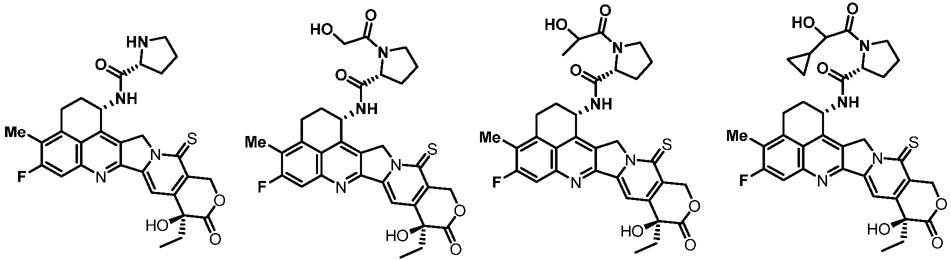

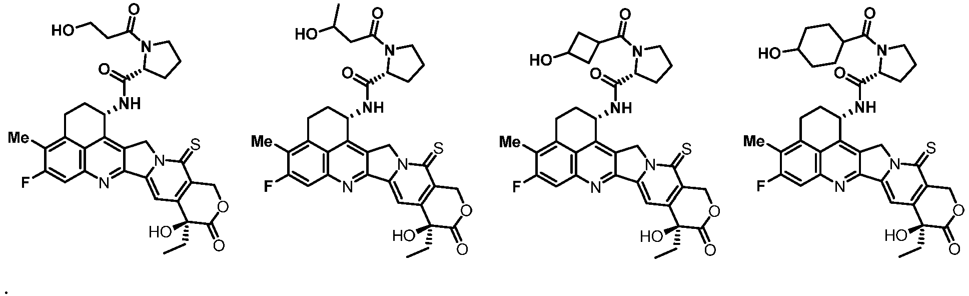

In a second aspect of the present application, a pharmaceutical composition is provided, which comprising any one of the compound of formula (I) as described in the first aspect of the present application, or a pharmaceutically acceptable salt thereof, or a hydrate thereof, and one or more pharmaceutically acceptable excipients, diluents or carriers.

In a third aspect of the present application, a use of the compound of formula (I) as described in the first aspect of the present application in the preparation of a pharmaceutical composition for treating diseases related tumor cell proliferation is provided.

In another preferred embodiment, the disease is selected from the group consisting of breast cancer, ovarian cancer, cervical cancer, lung cancer, uterine cancer, prostate cancer, kidney cancer, urethral cancer, bladder cancer, liver cancer, gastric cancer, endometrial cancer, salivary gland cancer, esophageal cancer, melanoma, glioma, neuroblastoma, sarcoma, pharyngeal cancer, colon cancer, rectal cancer, colorectal cancer, leukemia, bone cancer, skin cancer, thyroid cancer, pancreatic cancer, and lymphoma.

In a fourth aspect of the present application, a use of the compound of formula (I) as described in the first aspect of the present application is provided, wherein it is used as a toxin molecule in antibody-drug conjugate to prepare a conjugated drug, and the conjugated drug includes: an antibody-conjugated drug, a peptide-drug conjugate, a small molecule-conjugated drug, a polymer-conjugated drug, a lipid-drug conjugate and a protein-drug conjugate.

In a fifth aspect of the present application, provided is an intermediate compound as shown in formula (I-b),

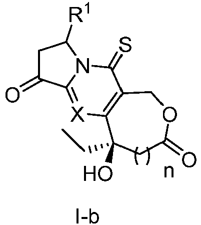

Wherein each group is defined as described above.

In another preferred embodiment, said compound has the structure as shown in the following formula (II-b) or (III-b):

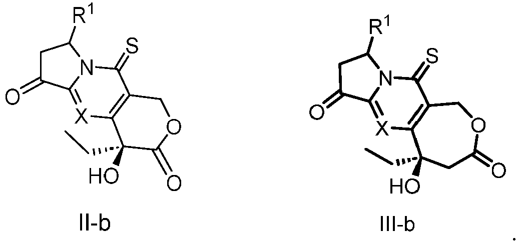

In another preferred embodiment, said compound has the structure as shown in the following formula (IV-b) or (V-b):

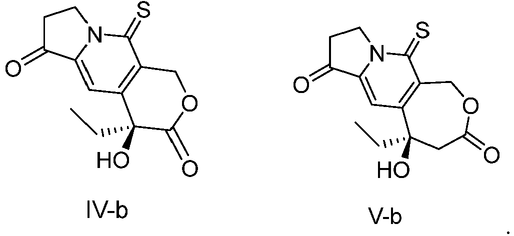

In a sixth aspect of the present application, provided is a method of preparing a compound of formula (I) as described in the first aspect of the present application, said method comprising the step: 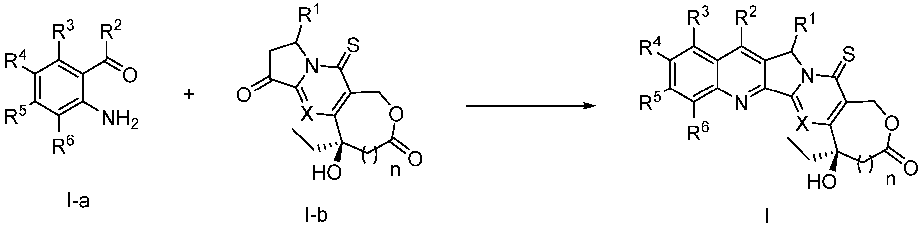 in an inert solvent, using a compound of formula (I-a) to react with a compound of formula (I-b) to obtain a compound of formula (I).

It should be understood that, within the scope of the present application, each of the above-described technical features of the present application and each of the technical features specifically described below (e.g., in the embodiments) can be combined with each other, thereby constituting a new or preferred technical solution. Limited by the space, we will not repeat them herein.

### DETAILED DESCRIPTION OF EMBODIMENTS

After long-term and in-depth research, the inventor unexpectedly discovered a compound as shown in formula (I). The compound has unexpected activity in inhibiting tumor cell proliferation and can be used to treat diseases associated with tumor cell proliferation. Based on the above findings, the inventors completed the present invention.

### Definitions of Terms

As used herein, the term "alkyl" includes a linear or branched alkyl. For example, C₁-C₈ alkyl represents a linear or branched chain alkyl with 1-8 carbon atoms, such as methyl, ethyl, propyl, isopropyl, butyl, isobutyl, tert-butyl, etc.

As used herein, the term "alkenyl" includes a linear or branched chain alkenyl. For example, C₂-C₆ alkenyl refers to a linear or branched chain alkenyl with 2-6 carbon atoms, such as vinyl, allyl, 1-propenyl, isopropenyl, 1-butenyl, 2-butenyl, and the like.

As used herein, the term "alkynyl" includes a linear or branched chain alkynyl. For example, C₂-C₆ alkynyl refers to a linear or branched chain alkynyl with 2-6 carbon atoms, such as ethynyl, propynyl, butynyl, and the like.

As used herein, the term "C₃-C₁₀ cycloalkyl" refers to a cycloalkyl with 3-10 carbon atoms. It can be a single ring, such as cyclopropyl, cyclobutyl, cyclopentyl, cyclohexyl, or similar groups. It can also be in the form of a double ring, such as a bridge ring or a spiro ring.

As used herein, the term "C₁-C₈ alkylamino" refers to anamino substituted by C₁-C₈ alkyl, which may be mono- or disubstituted; for example, methylamino, ethylamino, propylamino, isopropylamino, butylamino, isobutylamino, tert-butylamino, dimethylamino, diethylamino, dipropylamino, diisopropylamino, dibutylamino, diisobutylamino, di-*tert*-butylamino, etc.

As used herein, the term "C₁-C₈ alkoxy" refers to a linear or branched chain alkoxy with 1-8 carbon atoms; for example, methoxy, ethoxy, propoxy, isopropoxy, butoxy, iso-butyloxy, *tert*-butoxy, etc.

As used herein, the term "3-10 membered heterocycloalkyl with 1-3 heteroatoms selected from the group consisting of N, S, and O" refers to saturated or partially saturated cyclic group with 3-10 ring atoms, of which 1-3 atoms are heteroatoms selected from N, S and O. It can be in the form of a single ring or a double ring, such as a bridge ring or a spiro ring. Specific examples can be oxetane, azetidine, tetrahydro-2H-pyranyl, piperidinyl, tetrahydrofuranyl, morpholinyl, pyrrolidinyl, etc.

As used herein, the term "C₆-C₁₀ aryl" refers to an aryl with 6-10 carbon atoms, e.g., phenyl or naphthyl and the like.

As used herein, the term "5-10 membered heteroaryl with 1-3 heteroatoms selected from the group consisting of N, S, and O" refers to a cyclic aromatic group with 5-10 atoms, among which 1-3 atoms are heteroatoms selected from the group consisting of N, S, and O. It can be in the form of a single ring or a fused ring. Specific examples can be pyridinyl, pyridazinyl, pyrimidinyl, pyrazinyl, triazinyl, pyrrolyl, pyrazolyl, imidazolyl, (1,2,3)-triazolyl and (1,2,4)-triazolyl, tetrazolyl, furanyl, thienyl, isoxazolyl, thiazolyl, oxazolyl, etc.

Unless otherwise specified, the group in the present application is "substituted or unsubstituted", the groups of the present application can be substituted by substituents selected from the group consisting of halogen, cyano, nitro, hydroxyl, amino, C₁-C₆ alkyl-amino, C₁-C₆ alkyl, C₂-C₆ alkenyl, C₂-C₆ alkynyl, C₁-C₆ alkoxy, C₁-C₆ haloalkyl, halogenated C₂-C₆ alkenyl, halogenated C₂-C₆ alkynyl, halogenated C₁-C₆ alkoxy, allyl, benzyl, C₆-C₁₂ aryl, C₁-C₆ alkoxy-C₁-C₆ alkyl, C₁-C₆ alkoxy-carbonyl, phenoxy carbonyl, C₂-C₆ alkynyl-carbonyl, C₂-C₆ alkenyl-carbonyl, C₃-C₆ cycloalkyl-carbonyl, C₁-C₆ alkyl sulfonyl, etc.

As used herein, "halogen" or "halogen atom" refers to F, Cl, Br, and I. More preferably, the halogen or halogen atomis selected from F, Cl, and Br. "Halogenated" refers to being replaced by atoms selected from F, Cl, Br, and I.

Unless otherwise specified, the structural formula described in the application is intended to include all isomeric forms (such as enantiomers, diastereomers and geometric isomers (or conformational isomers)), for example, R and S configurations of asymmetric centers, (Z) and (E) isomers of double bonds, etc. Therefore, a single stereochemicalisomer of the compound of the application or a mixture of its enantiomers, diastereomers or geometric isomers (or conformational isomers) all fall within the scope of the application.

As used herein, the term "tautomer" means that structural isomers with different energies can cross the low energy barrier and transform into each other. For example, proton tautomer (i.e. proton shift) involes interconversion through proton migration, such as 1H-indazole and 2H-indazole. The valence tautomerism involves interconversion through some bonding electrons recombination.

As used herein, the term "hydrate" refers to a complex formed by the coordination of a compound of the present application with water.

The compound of this application can be prepared through various synthetic methods well-known to those skilled in the art, including the specific embodiments listed below, the embodiments formed by combining the specific embodiments with other chemical synthesis methods, and equivalent substitution methods well-known for those skilled in the art. Preferred embodiments include but are not limited to the embodiments of present application.

The solvent used in this application are commercially available, compounds are named manually or by ChemDraw^{®} software, commercially available compounds are named by supplier catalog.

### Pharmaceutical composition and method of administration

Due to the excellent inhibitory activity of the compounds of the present application on tumor cell proliferation, the compounds of the present application and their various crystal forms, pharmaceutically acceptable inorganic or organic salts, hydrates or solvates, as well as pharmaceutical composition containing the compounds of the present application as the main active ingredient, can be used for the prevention and/or treatment (stabilization, alleviation, or cure) of diseases associated with tumor cell proliferation.

The pharmaceutical composition of the present application includes compounds of the present application within a safe and effective range, as well as pharmaceutically acceptable excipients or carriers. The "safe and effective amount" refers to the amount of compound that is sufficient to significantly improve the condition without causing serious side effects. Typically, the pharmaceutical composition contains 1-2000 mg compound of the present application/formulation, and more preferably, 1-200 mg compound of the present application/formulation. Preferably, the "one dose" is a capsule or tablet.

"Pharmaceutically acceptable carrier" refers to one or more compatible solid or liquid fillers or gel substances, which are suitable for human use, and must be sufficient pure and low toxicity. "Compatible" herein refers to the ability of components of a composition to blend with each other and with the compounds of the application without significantly reducing the efficacy of the compounds. Examples of pharmaceutically acceptable carriers include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween^{®}), wetting agent (such as lauryl sodium sulfate), colouring agents, flavoringagents, stabilizers, antioxidants, preservatives, pyrogen-free water, etc.

The methods of administration of the compounds or pharmaceutical compositions of the present applicationare not particularly limited, and representative methods of administration include (but are not limited to) oral, parenteral (intravenous, intramuscular or subcutaneous).

Solid dosage forms for oral administration include capsules, tablets, pills, powders and granules. In these solid dosage forms, the active compounds are mixed with at least one conventional inert excipient (or carrier), such as sodium citrate or di-calcium phosphate, or mixed with any of the following components: (a) fillers or compatibilizer, such as starch, lactose, sucrose, glucose, mannitol and silicic acid; (b) binders, such as hydroxyl methyl cellulose, alginate, gelatin, polyvinylpyrrolidone, sucrose and arabic gum; (c) humectants, such as, glycerol; (d) disintegrating agents, such as agar, calcium carbonate, potato starch or tapioca starch, alginic acid, certain composite silicates, and sodium carbonate; (e) solution-retarding agents, such as paraffin; (f) absorption accelerators, such as quaternary ammonium compounds; (g) wetting agents, such as cetyl alcohol and glyceryl monostearate; (h) adsorbents, such as kaolin; and (i) lubricants, such as talc, stearin calcium, magnesium stearate, solid polyethylene glycol, lauryl sodium sulfate, or mixtures thereof. In capsules, tablets and pills, the dosage forms may also contain buffering agents.

The solid dosage forms such as tablets, dragees, capsules, pills and granules can be prepared by using coating and shell materials, such as enteric coatings and any other materials known in the art. They may include opacifying agents, and the active compound or compound in such a composition may be released in a certain part of the digestive tract in a delayed manner. Examples of the embedding components include polymers and waxes. If necessary, the active compound may also form a microcapsule form with one or more of the excipients described above.

Liquid dosage forms for oral administration include pharmaceutically acceptable emulsions, solutions, suspensions, syrups or tinctures. In addition to the active compounds, the liquid dosage form may contain inert diluents conventionally used in the art, such as water or other solvents, solubilizers and emulsifiers, for example, ethanol, isopropanol, ethyl carbonate, ethyl acetate, propylene glycol, 1,3-butanediol, dimethylformamide, as well as oil, in particular, cottonseed oil, peanut oil, corn germ oil, olive oil, castor oil and sesame oil, or mixtures thereof.

In addition to these inert diluents, the composition can also include adjuvants, such as wetting agents, emulsifying and suspending agents, sweetening agents, flavoring agents and perfuming agents.

In addition to the active compound, the suspension may comprise suspending agents, such as ethoxylated isooctadecanol, polyoxyethylene sorbitol and dehydrated sorbitol esters, microcrystalline cellulose, methanolic aluminum, agar, and mixtures thereof.

The composition for parenteral injection may comprise physiologically acceptable sterile aqueous or anhydrous solutions, dispersions, suspensions or emulsions, and sterile powders for redissolution into sterile injectable solutions or dispersions. Suitable aqueous and non-aqueous carriers, diluents, solvents, or excipients include water, ethanol, polyols, and suitable mixtures thereof.

The compounds of the present application can be administered alone or in combination with other pharmaceutically acceptable therapeutic agents.

When administered in combination, the pharmaceutical composition further includes one or more (2, 3, 4, or more) other pharmaceutically acceptable therapeutic agents. One or more (2, 3, 4, or more) of the other pharmaceutically acceptable therapeutic agents can be used simultaneously, separately or sequentially with the compounds of the present application for the prevention and/or treatment of diseases associated with tumour cell proliferation.

When the pharmaceutical composition is used, a safe and effective amount of the compound of the present application is administrated to a mammal (such as a human) in need of treatment, wherein the dose is considered as a pharmaceutically effective dose. For a person weighing 60 kg, the daily dose is usually 1 to 2000 mg, preferably 1 to 500 mg. Of course, the particular dose should also depend on various factors, such as the route of administration, patient healthy status, which are well within the skills of an experienced physician.

The present application will be further illustrated below in conjunction with specific embodiments. It should be understood that these examples only aim to illustrate the application but not to limit the scope of the application. The experimental methods in the following examples without specific conditions are usually following conventional conditions or conditions recommended by the manufacturer. Unless otherwise specified, percentages and portions are calculated by weight.

### Example 1

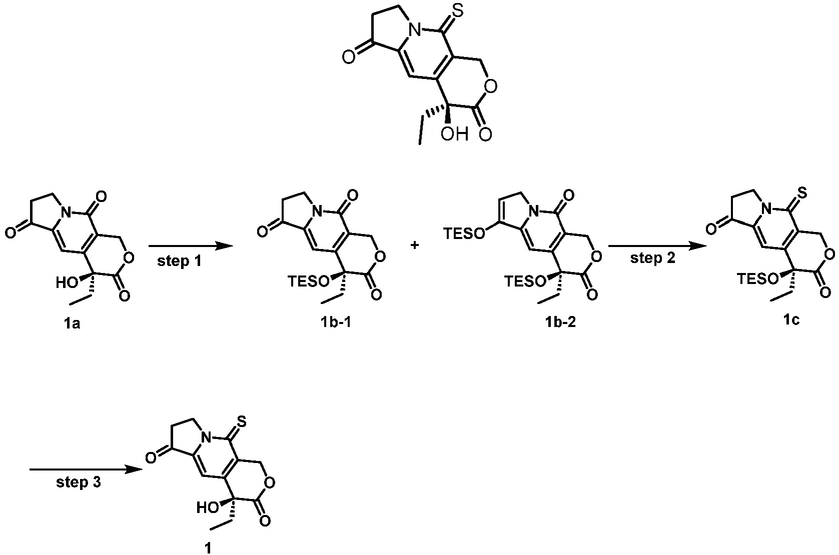

### Step 1

**1a** (1.95 g, 7.40 mmol) and imidazole (2.52 g, 37.00 mmol) were added to a three-neck flask (100 mL) successively, *N,N*-dimethylformamide (30 mL) was added after the flask being evacuated and backfilled with nitrogen. The mixture was cooled to 0°C followed by the addition of chlorotriethylsilane (4.45 g, 29.60 mmol) and 4-dimethylaminopyridine (0.90 g, 7.40 mmol) successively, the resulting mixture was stirred at 0°C for 2 h. The reaction mixture was diluted with ethyl acetate (200 mL), and washed with brine (25 mL x 4). The organic phase was dried over anhydrous sodium sulfate and filtered. The filtrate was concentrated under reduced pressure and the residue was purified by flash column chromatography on silica gel (ethyl acetate: hexanes= 0-100%) to give **1b-1** (0.90 g) and **1b-2** (1.50 g) with 73% overall yield.
**1b-1:**
   MS-ESI calc. for [M+H]⁺ 378, found 378.
**1b-2:**
   MS-ESI calc. for [M+H]⁺ 492, found 378 (lose one TES).

### Step 2

**1b-2**(1.10 g, 2.20 mmol), Lawesson's Reagent (1.80 g, 4.40 mmol) were added to a three-neck round-bottom flask (100 mL) successively, the mixture was degassed under vacuum and purged with N₂ followed by the addition of anhydrous toluene (30 mL), the resulting mixture was stirred at 90°C for 5-6 h. The reaction mixture was diluted with ethyl acetate (200 mL), and washed with brine (25 mL x 2). The organic phase was dried over anhydrous sodium sulfate and filtered. The filtrate was concentrated under reduced pressure and the residue was purified by flash column chromatography on silica gel (ethyl acetate: hexanes= 0-100%) to give **1c** (620 mg) with 51% yield.

MS-ESI calc. for [M+H]⁺ 394, found 394.

### Step 3

Triethylamine trihydrofluoride (552 mg, 3.43 mmol) was added to a solution of **1c** (540 mg, 1.37 mmol) in tetrahydrofuran (20 mL) under ice bath, and then the resulting mixture was gradually warmed to room temperature and stirred overnight. The reaction mixture was diluted with ethyl acetate (100 mL), and washed with brine (25 mL x 2). The organic phase was dried over anhydrous sodium sulfate and filtrated. The filtrate was concentrated under reduced pressure and the residue was purified by flash column chromatography on silica gel (ethyl acetate: hexanes= 0-100%) to give **1** (210 mg) with 55% yield.

MS-ESI calc. for [M+H]⁺ 280, found 280.

¹H NMR (500 MHz, CDCl₃) δ 7.56 (s, 1H), 6.12 (d, *J =* 17.5 Hz, 1H), 5.25 (d, *J =* 17.5 Hz, 1H), 4.71-4.52 (m, 2H), 3.09-3.03 (m, 2H), 1.80 (q, *J =* 7.0 Hz, 2H), 0.96 (t, *J =* 7.0 Hz, 3H).

### Example 2

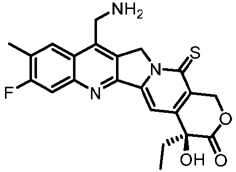

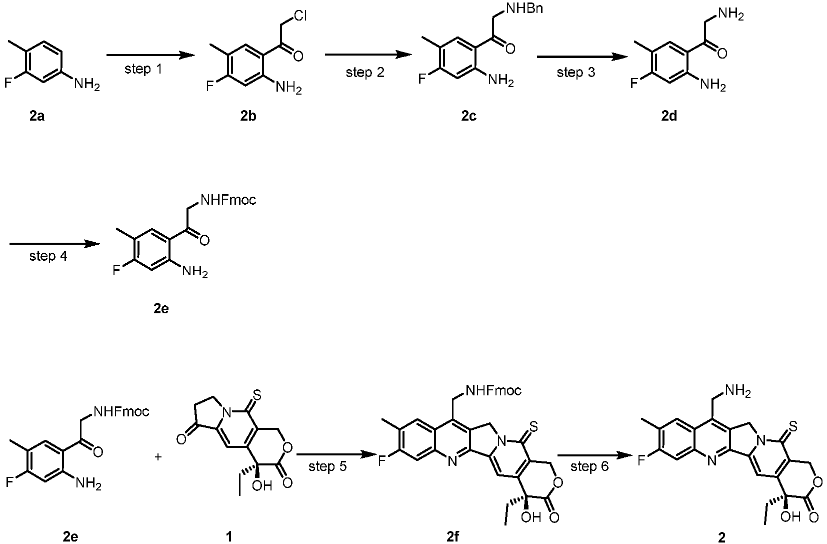

### Step 1

Boron trichloride (1.0 M in heptane, 94 mL, 94.00 mmol) was added to an over-dried there-neck flask (500 mL) under N₂ atomosphere followed with 1,2-dichloroethane (150 mL), the resulting solution was then cooled to 0°C, and **2a** (9.00 g,72.00 mmol) was added in batches. After stirring at 0°C for 10 min, chloroacetonitrile (7.05 g, 94.00 mmol) andaluminium trichloride (13.43 g, 101.00 mmol) were added, and the resulting mixture was gradually heated to 80°C and refluxed for 40 h. The reaction was cooled to 0°C, ice-water (50 mL) and HCl_{(aq)} (1N, 100 mL) was slowly added successively. The layers were separated after stirring at 0°C for 0.5 h. The aqueous phase was extracted with dichloromethane/methanol mixed solvent (V_{dichloromethane}:Vₘₑₜₕₐₙₒₗ= 4: 1, 200 mL).The combined organic phases were dried over anhydrous sodium sulfate and filtered. The filtrate was concentrated under reduced pressure and the residue was purified by flash column chromatography on silica gel (ethyl acetate: hexanes= 0-100%) to give crude **2b** (5.5 g) directly for the next step.

MS-ESI calc. for [M+H]⁺ 202, found 202.

### Step 2

Benzylamine (3 mL) was added to a solution of crude **2b** (700 mg) in anhydrous dichloromethane (5 mL) under N₂ atomosphere, the resulting mixture was stirred at room temperature for 5 h. The reaction mixture was concentrated under reduced pressure and the residue was purified by flash column chromatography on silica gel (ethyl acetate: hexanes= 0-100%) to give **2c** (390 mg) with 41% yield.

MS-ESI calc. for [M+H]⁺ 273, found 273.

### Step 3

To a single neck flask (100 mL) was added **2c** (390 mg, 1.43 mmol) and 10% wet Pd/C (90 mg) successively followed with anhydrous tetrahydrofuran (10 mL). After the flask was evacuated and backfilled with hydrogen with a hydrogen balloon, the mixture was stirred at room temperature for 20 h. The reaction mixture was filtered to remove insoluble material, and the filtrate was concentrated under reduced pressure to give crude **2d** (290 mg).

MS-ESI calc. for [M+H]⁺183, found 183.

### Step 4

9-Fluorenylmethyl chloroformate (410 mg, 1.59 mmol) and potassium carbonate (439 mg, 3.18 mmol) were added to a solution of crude **2d** (290 mg) intetrahedrofuran/water mixed solvent (V_{tetrahydrofuran}:V_{water}=1:1, 10 mL), the resulting mixture was stirred at 0°C for 0.5 h. The reaction mixture was diluted with ethyl acetate (100 mL), and washed with brine (25 mL). The organic phase was dried over anhydrous sodium sulfate and filtered. The filtrate was concentrated under reduced pressure and the residue was purified by flash column chromatography on silica gel (ethyl acetate: hexanes= 0-100%) to give **2e** (390 mg) with 67% yield over 2 steps.

MS-ESI calc. for [M+H]⁺ 405, found 405.

### Step 5

To a Schlenk tube (100 mL) was added **2e** (240 mg, 0.59 mmol), **1** (165 mg, 0.59 mmol) and *p*-toluenesulfonic acid (60 mg, 0.35 mmol) successively followed by addition of anhydrous toluene (6 mL), the resulting mixture was stirred at 110°C for 2 h. The reaction mixture was concentrated under reduced pressure and the residue was purified by flash column chromatography on silica gel (ethyl acetate: hexanes= 0-100%) to give **2f** (120 mg) with 31% yield.

MS-ESI calc. for [M+H]⁺ 648, found 648.

### Step 6

Diethylamine (135 mg, 1.85 mmol) was added to a solution of **2f** (120 mg, 0.19 mmol) in anhydrous tetrahydrofuran (2.5 mL), the resulting mixture was stirred at room temperature for 5 h. The reaction mixture was concentrated under reduced pressure and the residue was purified by flash column chromatography on silica gel (methanol: dichloromethane = 0-100%) to give **2** (30 mg) with 38% yield.

MS-ESI calc. for [M+H]⁺ 426, found 426.

¹H NMR (400 MHz, DMSO-*d₆*) δ 8.30 (d, *J =* 8.4 Hz, 1H), 7.91 (d, *J =* 10.8 Hz, 1H), 7.80 (s, 1H), 6.69 (s, 1H), 5.93 (d, *J =* 16.4 Hz, 1H), 5.71 (s, 2H), 5.51 (d, *J =* 16.8 Hz, 1H), 4.41 (s, 2H), 2.51 (s, 3H), 1.96-1.85 (m, 2H), 0.86 (t, *J =* 7.2 Hz, 3H).

### Example 3

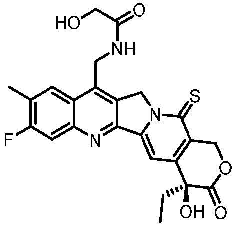

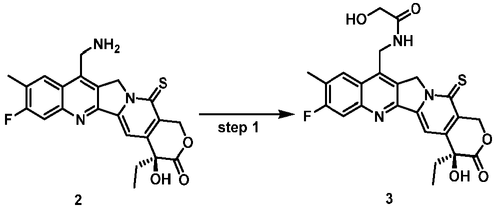

### Step 1

To a single neck flask (25 mL) was added **2** (18 mg, 0.04 mmol), glycolic acid (6 mg, 0.08 mmol) and *N,N*-dimethylformamide (1.5 mL) successively. The reaction mixture was cooled to 0°C followed by the addition of *N,N*-diisopropylethylamine (16 mg, 0.12 mmol) and O-(7-azabenzotriazol-1-yl)-N,N,N',N'-tetramethyluronium hexafluorophosphate (15 mg, 0.04 mmol), the resulting mixture was stirred at 0°C for 0.5 h. The reaction mixture was diluted with ethyl acetate (100 mL), washed with brine (25 mL x 3), dried over anhydrous sodium sulfate and filtered. The filtrate was concentrated under reduced pressure and the residue was purified by prep-TLC (methanol: dichloromethane) to give 3 (4.4 mg) with 21% yield.

MS-ESI calc. for [M+H]⁺ 484, found 484.

¹H NMR (400 MHz, DMSO-*d*₆) δ 8.80 (t, *J =* 6.0 Hz, 1H), 8.50 (d, *J =* 8.0 Hz, 1H), 7.94 (d, *J =* 10.8 Hz, 1H), 7.80 (s, 1H), 6.68 (s, 1H), 5.93 (d, *J =* 16.8 Hz, 1H), 5.78 (s, 2H), 5.52 (d, *J =* 16.4 Hz, 1H), 4.89 (d, *J =* 6.0 Hz, 2H), 3.86 (s, 2H), 2.51 (s, 3H), 1.95-1.83 (m, 2H), 0.86 (t, *J =* 7.2 Hz, 3H).

### Example 4

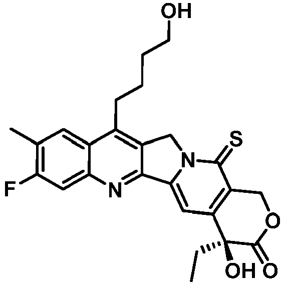

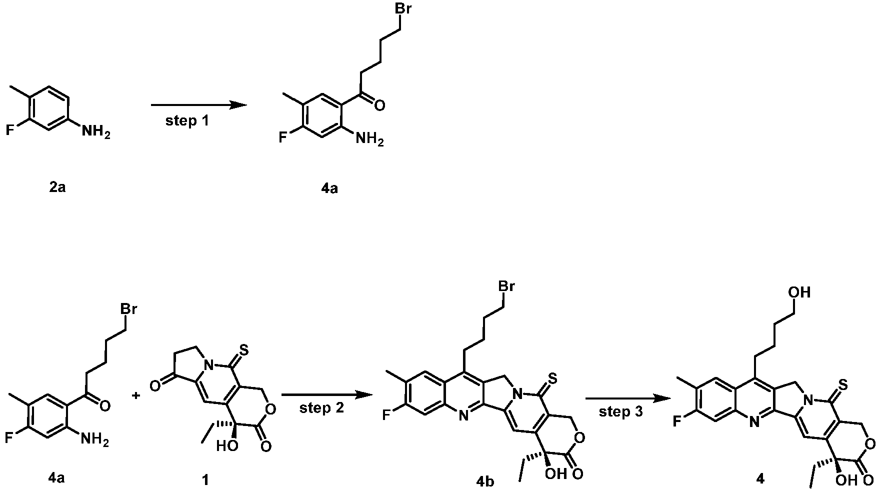

### Step 1

Boron trichloride (1.0 M in heptane, 12.80 mL, 12.80 mmol) was added to an over-dried there-neck flask (100 mL) under N₂ atomosphere followed with 1,2-dichloroethane (50 mL), the resulting solution was then cooled to 0°C, and **2a** (2.00 g, 16.00 mmol) was added in batches. After stirring at 0°C for 10 min, 5-bromovaleronitrile (3.63 g, 22.40 mmol) and aluminium trichloride (3.38 g, 22.40 mmol) were added, and the resulting mixture was gradually heated to 80°C and refluxed for 30 h. The reaction was cooled to 0°C, ice-water (50 mL) and HCl_{(aq)} (1N, 60 mL) were slowly added successively. The layers were separated after stirring at 0°C for 0.5 h. The aqueous phase was extracted with dichloromethane/methanol mixed solvent (V_{dichloromethane}:Vₘₑₜₕₐₙₒₗ= 4: 1, 200 mL).The combined organic phases were dried over anhydrous sodium sulfate and filtered. The filtrate was concentrated under reduced pressure and the residue was purified by flash column chromatography on silica gel (ethyl acetate: hexanes= 0-100%) to give crude **4a** (3.1 g) directly used in the next step.

MS-ESI calc. for [M+H]⁺288, 290, found 288, 290.

### Step 2

To a Schlenk tube (100 mL) was added **4a** (495 mg), **1** (320 mg, 1.15 mmol) and *p*-toluenesulfonic acid (198 mg, 1.15 mmol) successively followed by addition of anhydrous toluene (6 mL), the resulting mixture was stirred at 110°C for 2 h. The reaction mixture was concentrated under reduced pressure and the residue was purified by flash column chromatography on silica gel (ethyl acetate: hexanes= 0-100%) to give crude **4b** (200 mg).

MS-ESI calc. for [M+H]⁺ 531,533, found 531,533.

### Step 3

**4b** (90 mg) was dissolved in hexamethylphosphoramide (0.9 mL) and water (0.1 mL), the resulting mixture was stirred at 101°C for 4 h. The reaction mixture was diluted with ethyl acetate (100 mL), washed with brine (25 mL x 3), dried over anhydrous sodium sulfate and filtered. The filtrate was concentrated under reduced pressure and the residue was purified by prep-HPLC to give 4 (12 mg) with 15% yield.

MS-ESI calc. for [M+H]⁺ 469, found 469.

¹H NMR (400 MHz, DMSO-*d₆*) δ 8.26 (d, *J =* 8.0 Hz, 1H), 7.90 (d, *J =* 10.8 Hz, 1H), 7.79 (s, 1H), 5.92 (d, *J =* 16.4 Hz, 1H), 5.78 (s, 2H), 5.50 (s, 2H), 5.49 (d, *J =* 16.4 Hz, 1H), 3.29-3.20 (m, 2H), 2.51 (s, 3H), 1.94-1.82 (m, 2H), 1.82-1.70 (m, 2H),1.67-1.56 (m, 2H), 0.86 (t, *J =* 7.2 Hz, 3H).

### Example 5

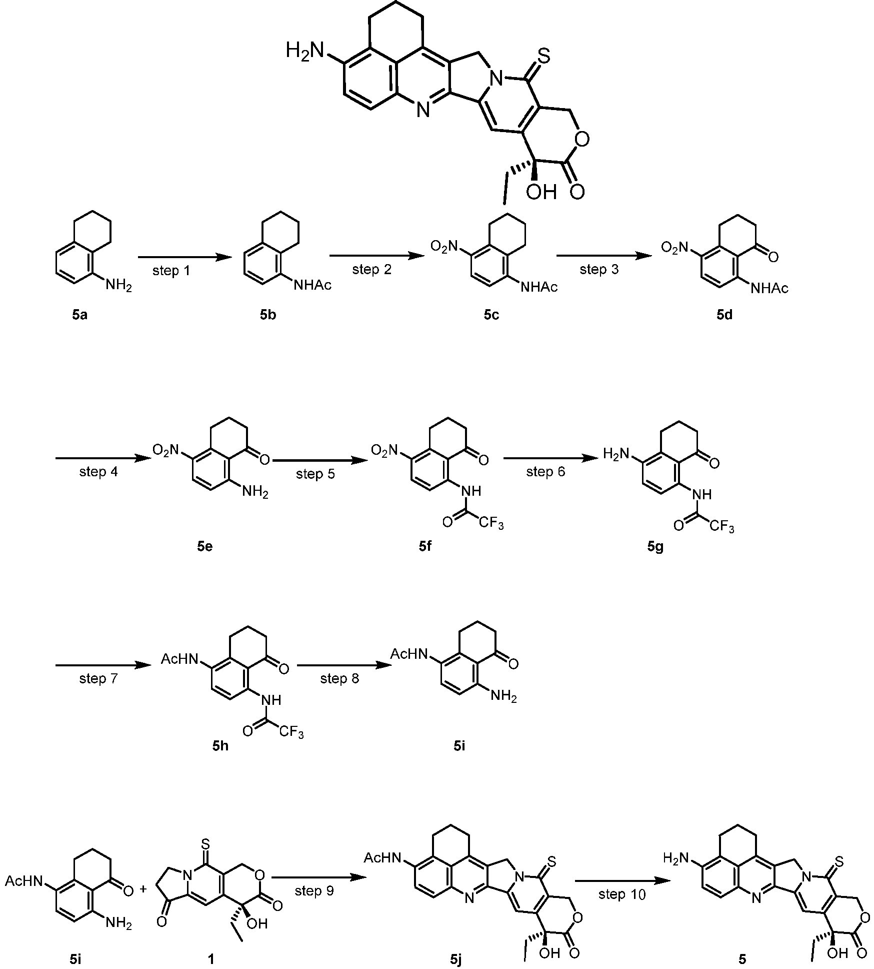

### Step 1

**5a** (2.50 g, 17.00 mmol) was dissolved in anhydrous dichloromethane (25 mL), the solution was cooled to 0°C followed by the addition of triethylamine (2.60 g, 20.00 mmol) and acetyl chloride (1.40 g, 18.00 mmol) successively, and the resulting mixture was stirred at 0°C for 1 h. The reaction mixture was diluted with dichloromethane (100 mL), washed with brine (25 mL x 3), dried over anhydrous sodium sulfate and filtered. The filtrate was concentrated under reduced pressure and the residue was triturated with methyl *tert*-butyl ether (50 mL) to give **5b** (2.80 g) with 87% yield.

MS-ESI calc. for [M+H]⁺ 190, found 190.

### Step 2

**5b** (2.00 g, 10.60 mmol) was added to concentrated sulphuric acid (35 mL) in batches at -20°C, after dissolution, potassium nitrate (1.07 g, 10.58 mmol) in batches was added, and the resulting mixture was stirred at -20°C for 1 h. The reaction mixture was slowly added to ice-water (100 mL), and the aqueous phase was extracted with dichloromethane (500 mL). The organic phase was dried over anhydrous sodium sulfate and filtered. The filtrate was concentrated and the residue was triturated with methyl *tert*-butyl ether (25 mL) to give the first batch of **5c** (1.00 g), and the mother liquor was concentrated under reduced pressure and the residue was triturated with methyl *tert*-butyl ether (15 mL) to give the second batch of **5c** (1.20 g) with 87% overall yield.

MS-ESI calc. for [M+H]⁺ 235, found 235.

### Step 3

Aqueous magnesium sulphate (1.51 g, 12.56 mmol, 1.5 M) was added to a solution of **5c** (2.10 g, 8.97 mmol) in acetone (60 mL), the mixture was cooled to 0°C followed by the addition of potassium permanganate (5.95 g, 37.68 mmol) in batches, the resulting mixture was stirred at 0°C for 1.5 h. The reaction mixture was filtered to remove insoluble solids, and the filter cake was washed with dichloromethane (200 mL). The collected organic phase was washed with brine (50 mL x 3), dried over anhydrous sodium sulfate and filtered. The filtrate was concentrated under reduced pressure and the residue was purified by flash column chromatography on silica gel (ethyl acetate: hexanes= 0-100%) to give **5d** (400 mg) with 18% yield.

MS-ESI calc. for [M+H]⁺ 249, found 249.

¹H NMR (400 MHz, DMSO-*d₆*) δ 12.28 (s, 1H), 8.64 (d, *J =* 9.2 Hz, 1H), 8.19 (d, *J =* 9.6 Hz, 1H), 3.12 (t, *J =* 6.0 Hz, 2H), 2.75 (t, *J =* 6.4 Hz, 2H), 2.21 (s, 3H), 2.06-1.95 (m, 2H).

### Step 4

**5d** (400 mg, 1.61 mmol) was dissolved in HCl_{(aq)} (6 N, 6 mL), the resulting mixture was stirred at 80°C for 2.5 h. The reaction mixture was diluted with water (25 mL) followed by the addition of aqueous sodium bicarbonate to adjust pH to ~9. The aqueous phase was extracted with dichloromethane (100 mL x 2), and the combined organic phases were dried over anhydrous sodium sulfate and filtered. The filtrate was concentrated under reduced pressure to afford crude **5e** (350 mg).

MS-ESI calc. for [M+H]⁺ 207, found 207.

### Step 5

Pyridine (254 mg, 3.22 mmol) and trifluoroacetic anhydride (676 mg, 3.22 mmol) was added successively to the solution of crude **5e** (350 mg) in anhydrous dichloromethane (25 mL) under an ice-bath, the resulting mixture was stirred at 0°C for 0.5 h. The reaction mixture was diluted with dichloromethane (50 mL), washed with brine (25 mL x 2), dried over anhydrous sodium sulfate and filtered. The filtrate was concentrated under reduced pressureand the residue was purified by flash column chromatography on silica gel (ethyl acetate: hexanes= 0-100%) to give **5f** (410 mg) with 84% yield over 2 steps.

MS-ESI calc. for [M+H]⁺ 303, found 303.

¹H NMR (400 MHz, CDCl₃) δ 13.64 (s, 1H), 8.76 (d, *J =* 9.2 Hz, 1H), 8.14 (d, *J =* 9.6 Hz, 1H), 3.27 (t, *J =* 6.0 Hz, 2H), 2.82 (t, *J =* 6.4 Hz, 2H), 2.21-2.11 (m, 2H).

### Step 6

Zinc powder (1.74 g, 27.20 mmol) was added in batches to a solution of **5f** (410 mg, 1.36 mmol) in a solvent mixture of methanol (50 mL), water (4 mL) and formic acid (4 mL) under an ice bath, the resulting mixture was stirred at 0°C for 0.5 h. The reaction mixture was filtered to remove insoluble solid and the filter cake was washed sequentially with ethyl acetate (200 mL) and water (50 mL) followed by the addition of aqueous sodium bicarbonate to adjust the pH to 8-9. The layers were separated, and the organic phase was dried over anhydrous sodium sulfate and filtered. The filtrate was concentrated under reduced pressure to give crude **5g** (330 mg).

MS-ESI calc. for [M+H]⁺ 273, found 273.

### Step 7

The above crude **5g** (330 mg) was dissolved in anhydrous dichloromethane (25 mL), the solution was cooled to 0°C followed by the addition of triethylamine (260 mg, 2.57 mmol) and acetyl chloride (200 mg, 2.57 mmol) successively, and the resulting mixture was stirred at 0°C for 1 h. The reaction mixture was diluted with dichloromethane (50 mL), washed with brine (25 mL x 2), dried over anhydrous sodium sulfate and filtered. The filtrate was concentrated under reduced pressure and the residue was purified by flash column chromatography on silica gel (methanol: dichloromethane= 0-100%) to give 5h (310 mg) with 72% yield over 2 steps.

MS-ESI calc. for [M+H]⁺ 315, found 315.

### Step 8

Potassium carbonate (550 mg, 3.99 mmol) was added to a solution of 5h (310 mg, 0.99 mmol) in a solvent mixture of methanol (22 mL) and water (1.5 mL), the resulting mixture was stirred at 50°C for 1 h. The reaction mixture was diluted with dichloromethane (100 mL) and water (25 mL), and the layers were separated. The aqueous phase was extracted with dichloromethane/methanol mixed solvent (V_{dichloromethane}: Vₘₑₜₕₐₙₒₗ = 4:1, 50 mL), and the combined organic phases were dried over anhydrous sodium sulfate and filtered. The filtrate was concentrated under reduced pressure and the residue was purified by flash column chromatography on silica gel (methanol: dichloromethane= 0-100%) to give **5i** (205 mg) with 95% yield.

MS-ESI calc. for [M+H]⁺ 219, found 219.

### Step 9

To a Schlenk tube (100 mL) was added **5i** (200 mg, 0.92 mmol), **1** (257 mg, 0.92 mmol) and *p*-toluenesulfonic acid (158 mg, 0.92 mmol) successively followed by addition of anhydrous toluene (10 mL), the resulting mixture was stirred at 110°C for 6 h. The reaction mixture was concentrated under reduced pressure and the residue was purified by flash column chromatography on silica gel (methanol: dichloromethane= 0-100%) to give crude **5j** (170 mg) directly for the next step.

MS-ESI calc. for [M+H]⁺ 462, found 462.

### Step 10

The crude product **5j** (170 mg) was dissolved in HCl_{(aq)} (6N, 2 mL), the resulting mixture was stirred at 80°C for 4 h. The reaction mixture was diluted with dichloromethane/methanol mixed solvent (V_{dichloromethane}: Vₘₑₜₕₐₙₒₗ = 10:1, 100 mL) and water (20 mL) followed by the addition of aqueous sodium bicarbonate to adjust pH to ~9. The layers were separated, and the aqueous phase was extracted with dichloromethane/methanol mixed solvent (V_{dichloromethane}: Vₘₑₜₕₐₙₒₗ = 10:1, 100 mL). the combined organic phases were dried over anhydrous sodium sulfate and filtered. The filtrate was concentrated under reduced pressure and the residue was purified by flash column chromatography on silica gel (methanol: dichloromethane= 0-100%) to give **5** (100 mg) with 26% yield over 2 steps.

MS-ESI calc. for [M+H]⁺ 420, found 420.

¹H NMR (400 MHz, DMSO-*d₆*) δ 7.76 (d, *J =* 8.8 Hz, 1H), 7.67 (s, 1H), 7.32 (d, *J =* 9.2 Hz, 1H), 6.62 (s, 1H), 5.91 (d, *J =* 16.4 Hz, 1H), 5.72 (s, 2H), 5.49 (d, *J =* 16.8 Hz, 1H), 5.42 (s, 2H), 3.09 (t, *J =* 5.6 Hz, 2H), 2.76 (t, *J =* 6.0 Hz, 2H), 2.07-1.98 (m, 2H), 1.88 (q, *J =* 7.2 Hz, 2H), 0.86 (t, *J =* 7.2 Hz, 3H).

### Example 6

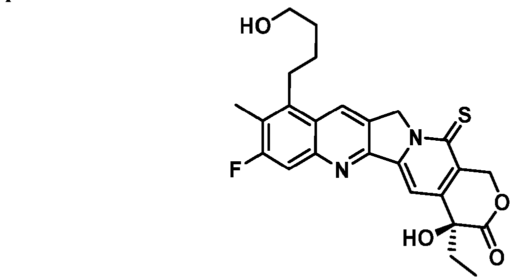

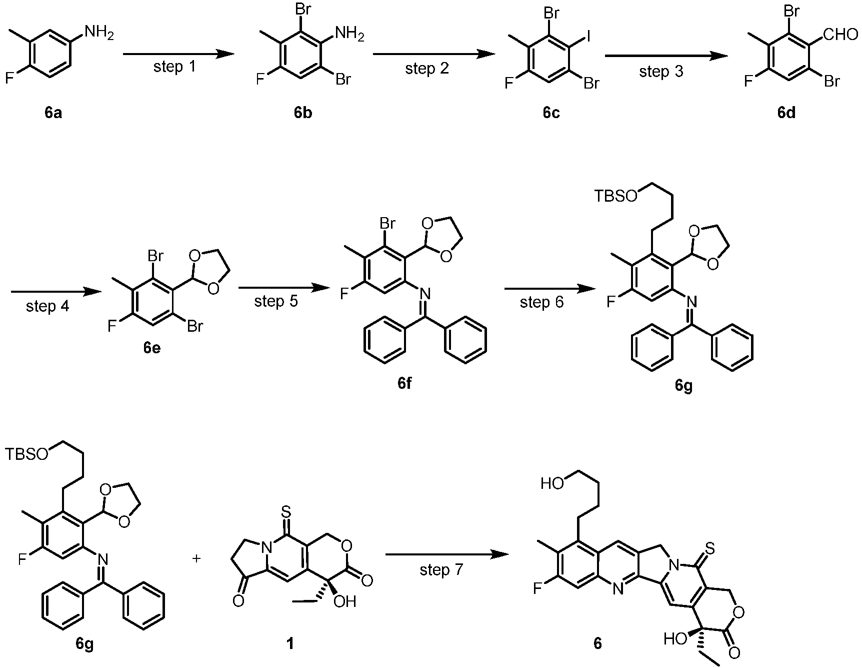

### Step 1

**6a** (20.00 g, 159.80 mmol) was dissolved in a dichloromethane/methanol mixed solvent mixture (V_{dichloromethane}: Vₘₑₜₕₐₙₒₗ = 1:1, 350 mL), and the solution was cooled down to 0 °C. A solution of liquid bromine (63.84 g, 399.50 mmosl) in dichloromethane/methanol mixed solvent (V_{dichloromethane}: Vₘₑₜₕₐₙₒₗ = 1:1, 50 mL) was added dropwise to the above solution, the resulting mixture was stirred at 25°C for 4 h. The reaction solution was concentrated under reduced pressure to give a residue, and the residue was diluted with aqueous sodium thiosulfate (1 M, 350 mL) and ethyl acetate (350 mL). After stirring for 10 min, aqueous sodium carbonate solution (1M, 50 mL) was added, and the layers were then separated. The organic phase was washed sequentially with aqueous sodium thiosulfate (1M, 230 mL x 1) and brine (230 mL x 1), dried over anhydrous sodium sulfate and filtered. The filtrate was concentrated under reduced pressure and the residue was purified by flash column chromatography on silica gel (ethyl acetate: hexanes= 0-100%) to give **6b** (40 g) with 89% yield.

¹H NMR (400 MHz, CDCl₃) δ 7.16 (d, *J =* 8.4 Hz, 1H), 4.40 (br s, 2H), 2.27 (d, *J* = 2.4 Hz, 3H).

### Step 2

*P*-toluenesulfonic acid (1.83 g, 10.63 mmol) was dissolved in anhydrous acetonitrile (15 mL), the mixture was stirred to dissolve and then cooled down to 0°C followed by the addition of a solution of potassium iodide (1.47 g, 8.86 mmol) and sodium nitrite (480 mg, 7.10 mmol) in water (2 mL). The resulting mixture was stirred at 0°C for 10 min, then warmed to room temperature and stirred for additional 1 h. The reaction mixture was added to water (8 mL), and aqueous sodium bicarbonate was added to adjust pH to ~9 followed with aqueous sodium thiosulphate (2.5 mL, 2M). The aqueous phase was extracted with ethyl acetate (30 mL x 3). The combined organic phases were dried over anhydrous sodium sulfate and filtered. The filtrate was concentrated under reduced pressure and the residue was purified by flash column chromatography on silica gel (ethyl acetate: hexanes= 0-100%) to give **6c** (500 mg) with 36% yield.

### Step 3

**6c** (9.00 g, 22.85 mmol) was dissolved in anhydrous toluene (54 mL) under N₂ atomosphere, the mixture was then cooled down to -30°C followed by the dropwise addition of a solution of isopropylmagnesium chloride in tetrahydrofuran (2.82 g, 27.42 mmol, 2M), the reaction was stirred at -30°C for 1.5 h, N,N-dimethylformamide (5.51 g, 75.42 mmol) was was then added dropwise. The resulting mixture was warmed up to 20°C and stirred for an additional 2 h. The reaction solution was diluted with saturated aqueous ammonium chloride (150 mL), and the aqueous was extracted with ethyl acetate (150 mL x 3). The combined organic phases were dried over anhydrous sodium sulfate and filtered. The filtrate was concentrated under reduced pressure and the residue was purified by flash column chromatography on silica gel (ethyl acetate: hexanes= 0-100%) to give **6d** (4.2 g) with 62% yield.

¹H NMR (400 MHz, CDCl₃) δ 10.20 (s, 1H), 7.38 (d, *J =* 8.8 Hz, 1H), 2.35 (d, *J =* 2.4 Hz, 3H).

### Step 4

Ethylene glycol (4.43 g, 71.45 mmol), triethyl orthoformate (2.31 g, 5.61 mmol) and p-toluenesulfonic acid (244.40 mg, 1.42 mmol) were added to a solution of **6d** (4.20 g, 14.19 mmol) in anhydrous 1,2-dichloroethane (35 mL), the resulting mixture was stirred at 80°C for 12h. The reaction mixture was washed with saturated aqueous sodium carbonate (80 mL) and saturated aqueous ammonium chloride (80 mL). The organic phase was dried over anhydrous sodium sulfate and filtered. The filtrate was concentrated under reduced pressure and the residue was purified by flash column chromatography on silica gel (ethyl acetate: hexanes= 0-100%) to give **6e** (3.9 g) with 81% yield.

¹H NMR (400 MHz, CDCl₃) δ 7.33 (d, *J =* 8.8 Hz, 1H), 6.43 (s, 1H), 4.36-4.30 (m, 2H), 4.11-4.04 (m, 2H), 2.32 (d, *J =* 1.2 Hz, 3H).

### Step 5

**6e** (1.5 g, 4.41 mmol), benzophenone imine (880 mg, 4.85 mmol), palladium acetate (99 mg, 0.44 mmol), (255 mg, 0.44 mmol), sodium *tert*-butoxide (850 mg, 8.82 mmol) were dissolved in anhydrous toluene (15 mL) under N₂ atomosphere, the resulting mixture was stirred at 100°C for 12 h. The reaction mixture was concentrated under reduced pressure and the residue was purified by flash column chromatography on silica gel (ethyl acetate: hexanes = 0-100%) to give **6f** (300 mg) with 15% yield.

¹H NMR (400 MHz, CDCl₃) δ 7.76 (d, *J =* 7.2 Hz, 2H), 7.52-7.44 (m, 1H), 7.44-7.36 (m, 2H), 7.34-7.27 (m, 3H), 7.26-7.21 (m, 2H), 6.47 (s, 1H), 5.91 (d, *J* = 10.4 Hz, 2H), 4.10-4.04 (m, 2H), 3.96-3.90 (m, 2H), 2.21 (d, *J =* 2.0 Hz, 3H).

### Step 6

(But-3-en-1-yloxy)(*tert*-butyl)dimethylsilane (30 mg, 0.16 mmol) was dissolved in anhydrous toluene (1 mL) under N₂ atomosphere, and the solution was cooled to 0°C followed by the addition of 9-borabicyclo[3.3.1]nonane (24 mg, 0.19 mmol), the resulting mixture was heated at 80°C for 20 min and then cooled to 0°C, a solution of sodium hydroxide (13 mg, 0.32 mmol) in water (1 mL) was added dropwise. After stirring for 10 min at 0°C, **6f** (57 mg, 0.13 mmol), tetrabutylammonium iodide (3 mg, 0.008 mmol) and 1,1'-bis(diphenylphosphino)ferrocene palladium chloride (3 mg, 0.003 mmol) were added, and the resulting mixture was stirred at 80°C for 12 h. The reaction mixture was concentrated under reduced pressure and the residue was purified by prep-TLC to give **6g** (40 mg) with 56% yield.

### Step 7

**1** (62 mg, 0.22 mmol) and concentrated HCl_{(aq)} (52.56 mg, 1.44 mmol, 12 N) were added to a solution of **6g** (120 mg, 0.22 mmol) in anhydrous ethanol (4 mL) and the resulting mixture was stirred at 80°C for 2 h. The reaction mixture was concentrated under reduced pressure and the residue was purified by prep-TLC to give **6** (7 mg) with 7% yield.

¹H NMR (500 MHz, DMSO-*d₆*) δ 8.98 (s, 1H), 7.80 (d, *J =* 10.5 Hz, 1H), 7.79 (s, 1H), 5.92 (d, *J =* 16.5 Hz, 1H), 5.50 (d, *J =* 16.5 Hz, 1H), 5.497 (s, 2H), 3.19-3.12 (m, 2H), 2.43 (d, *J* = 2.0 Hz, 3H), 1.92-1.84 (m, 2H), 1.68-1.55 (m, 4H), 0.86 (t, *J =* 7.0 Hz, 3H).

### Example 7

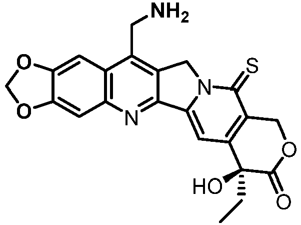

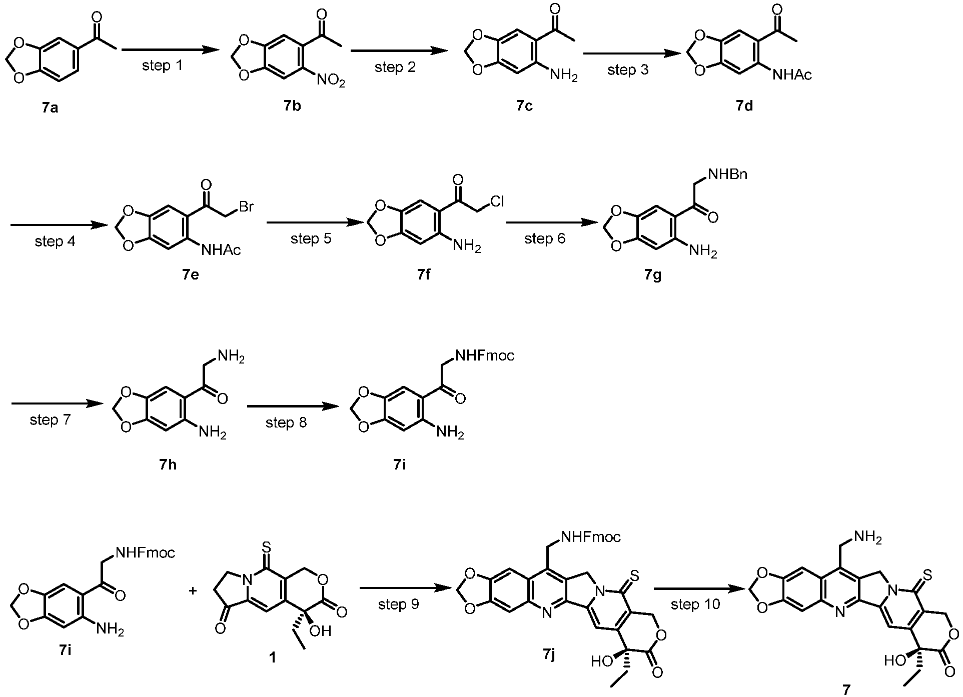

### Step 1

Sodium nitrite (13.78 g, 199.72 mmol) was added in batches to a solution of **7a** (11.00 g, 67.00 mmol) in trifluoroacetic acid (165 mL), the resulting mixture was stirred at 25°C for 12 h. The reaction mixture was diluted with water (100 mL), extracted with dichloromethane (100 mL x 3). The combined organic phses were washed with aqueous aqueous sodium bicarbonate (100 mL x 3), dried over anhydrous sodium sulfate and filtered. The filtrate was concentrated under reduced pressure and the residue was purified by flash column chromatography on silica gel (ethyl acetate: hexanes= 0-100%) to give **7b** (7.20 g) with 51% yield.

MS-ESI calc. for [M+H]⁺210, found 210.

### Step 2

Wet Pd/C (700 mg, 6.58 mmol, 10% w/w) was added to a solution of **7b** (7.20 g, 34.42 mmol) in tetrahydrofuran/water mixed solvent (V_{tetrahydrofuran}: V_{water} = 3:1, 35 mL), and the resulting mixture was stirred at room temperature for 12 h under H₂ atomosphere.The reaction mixture was filtered to remove insoluble solid and the filter cake was washed with dichloromethane. The filtrate was concentrated under reduced pressure to obtain the residue, which was dried over anhydrous sodium sulphate and filtered. The filtrate was concentrated under reduced pressure and the residue was purified by silica gel column chromatography (ethyl acetate: hexanes = 0-100%) to give **7c** (5.60 g) with 91% yield.

MS-ESI calc. for [M+H]⁺180, found 180.

¹H NMR (400 MHz, DMSO-*d₆*) δ 7.16 (s, 1H), 6.30 (s, 1H), 5.92 (s, 2H), 2.38 (s, 3H).

### Step 3

**7c** (5.50 g,30.70 mmol) was dissolved in anhydrous dichloromethane (110 mL), the solution was cooled to 0°C followed by the addition of *N,N-*diisopropylethylamine (5.95 g, 46.04 mmol) and acetyl chloride (3.61 g, 46.04 mmol) successively, and the resulting mixture was stirred at room temperature for 1 h. The reaction mixture was quenched with methanol (5 mL). The mixture was then concentrated under reduced pressure and the residue was purified by silica gel column chromatography (ethyl acetate: hexanes = 0-100%) to give **7d** (6.60 g) with 96% yield.

MS-ESI calc. for [M+H]⁺222, found 222.

¹H NMR (400 MHz, CDCl₃) δ 12.06 (br s, 1H), 8.35 (s, 1H), 7.24 (s, 1H), 6.01 (s, 2H), 2.55 (s, 3H), 2.19 (s, 3H).

### Step 4

**7d** (6.60 g, 29.84 mmol) was dissolved in glacial acetic acid (105 mL) and the solution was cooled down to 0°C, liquid bromine (4.77 g, 29.84 mmol) and hydrobromic acid acetic acid solution (12 M, 4.83 g, 59.68 mmol) were added and the resulting mixture was stirred at room temperature for 1 h. The reaction mixture was poured into ice-water (100 mL) to give a solid, which was collected. The collected filter cake was dissolved with dichloromethane (100 mL), dried over anhydrous sodium sulphate and filtered. The filtrate was concentrated under reduced pressure and the residue was purified by silica gel column chromatography (ethyl acetate: hexanes = 0-100%) to give **7e** (7.00 g) with 78% yield.

MS-ESI calc. for [M+H]⁺ 300, 302, found 300, 302.

### Step 5

Concentrated HCl_{(aq)} (12 M, 5.0 mL, 60.00 mmol) was added to a solution of **7e** (5.90 g, 19.67 mmol) in anhydrous ethanol (60 mL), the resulting mixture was stirred at 80°C for 4 h. The reaction mixture was concentrated under reduced pressure and the residue was purified by silica gel column chromatography (ethyl acetate: hexanes = 0-100%) to give crude **7f** (4.56 g).

MS-ESI calc. for [M+H]⁺214, 216, found 214, 216.

### Step 6

Benzylamine (10 mL) was added to a solution of **7f** (4.0 g, 18.70 mmol) in anhydrous dichloromethane (40 mL), the resulting mixture was stirred at room temperature for 4 h. The reaction mixture was concentrated under reduced pressure and the residue was purified by silica gel column chromatography (ethyl acetate: hexanes = 0-100%) to give **7g** (2.5 g) with 47% over 2 steps.

MS-ESI calc. for [M+H]⁺285, found 285.

### Step 7

Wet Pd/C (200 mg, 10% w/w) was added to a solution of **7g** (2.0 g, 7.04 mmol) in anhydrous tetrahydrofuran (25 mL), and the resulting mixture was stirred at 50 °C for 20 h under H₂ atomosphere. The reaction mixture was filtered to remove insoluble solid and the filtrate was concentrated under reduced pressure to give crude **7h** (1.3 g).

MS-ESI calc. for [M+H]⁺195, found 195.

### Step 8

Potassium carbonate (1.85 g, 13.40 mmol) and 9-fluorenylmethyl chloroformate (1.38 g, 5.36 mmol) was added to a solution of crude **7h** (1.3 g) in tetrahydrofuran/water mixed solvent (V_{tetrahydrofuran}: V_{water} = 2:1, 24 mL), the reaction mixture was stirred at 0°C for 1 h. The reaction mixture was diluted with water (20 mL) and ethyl acetate (100 mL) and the layers were separated. The organic phase was washed with brine (250 mL x 2), dried over anhydrous sodium sulfate and filtered. The filtrate was concentrated under reduced pressure and the residue was purified by flash column chromatography on silica gel (ethyl acetate: hexanes= 0-100%) to give **7i** (820 mg) with 28% yield over 2 steps.

MS-ESI calc. for [M+H]⁺417, found 417.

### Step 9

To a Schlenk tube (100 mL) was added **7i** (200 mg, 0.48 mmol), **1** (107 mg, 0.38 mmol) and *p*-toluenesulfonic acid (34 mg, 0.20 mmol) successively followed by addition of anhydrous toluene (10mL), the resulting mixture was stirred at 110°C for 6 h. The reaction mixture was concentrated under reduced pressure and the residue was purified by flash column chromatography on silica gel (ethyl acetate: hexanes= 0-100%) to give crude **7j** (200 mg) directly for the next step.

MS-ESI calc. for [M+H]⁺ 660, found 660.

### Step 10

Piperidine (255 mg, 3.00 mmol) was added to a solution of **7j** (200 mg, 0.30 mmol) in anhydrous tetrahydrofuran (5 mL), the reaction mixture was stirred at room temperature for 3.5 h. The reaction mixture was concentrated under reduced pressure and the residue was purified by flash column chromatography on silica gel (methanol: dichloromethane= 0-100%) to give **7** (80 mg) with 61% yield.

MS-ESI calc. for [M+H]⁺438, found 438.

¹H NMR (500 MHz, DMSO-*d₆*) δ 7.72 (s, 1H), 7.66 (s, 1H), 7.51 (s, 1H), 6.29 (d, *J =* 2.0 Hz, 2H), 5.91 (d, *J =* 16.5 Hz, 1H), 5.48 (d, *J =* 16.5 Hz, 1H), 4.28 (s, 2H),1.92-1.84 (m, 2H), 0.86 (t, *J =* 7.0 Hz, 3H).

### Example 8

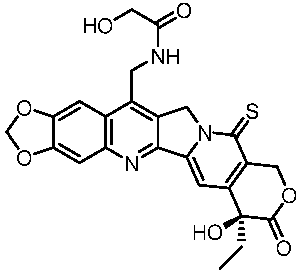

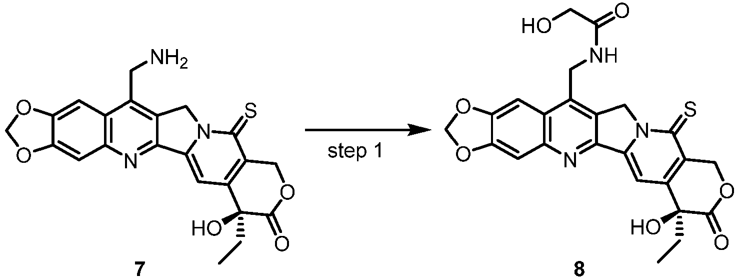

### Step 1

To a single neck flask (25 mL) was added **7** (30 mg, 0.07 mmol), glycolic acid (10.6 mg, 0.14 mmol) and anhydrous dichloromethane (1.0 mL) successively. The reaction mixture was cooled to 0°C followed by the addition of *N,N*-diisopropylethylamine (18 mg, 0.14 mmol) and O-(7-azabenzotriazol-1-yl)-N,N,N',N'-tetramethyluronium hexafluorophosphate (24 mg, 0.06 mmol), the resulting mixture was stirred at 0°C for 0.5 h. The reaction mixture was concentrated under reduced pressure and the residue was purified by flash column chromatography on silica gel (methanol: dichloromethane = 0-100%) to give **8** (12 mg) with 35% yield.

MS-ESI calc. for [M+H]⁺ 496, found 496.

### Example 9

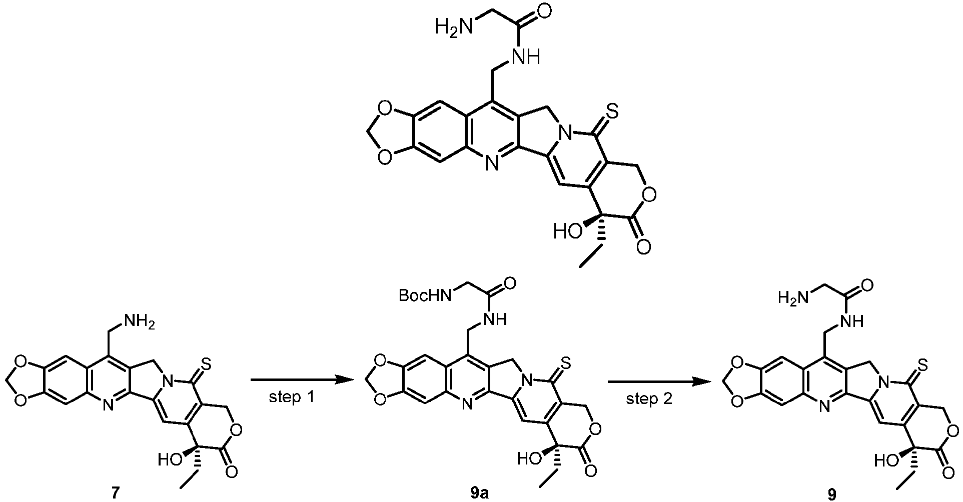

### Step 1

To a single neck flask (25 mL) was added **7** (65 mg, 0.15 mmol), N-Boc-glycine (52 mg, 0.30 mmol) and anhydrous dichloromethane (2 mL) successively. The reaction mixture was cooled to 0°C followed by the addition of *N,N*-diisopropylethylamine (39 mg, 0.38 mmol) and O-(7-azabenzotriazol-1-yl)-N,N,N',N'-tetramethyluronium hexafluorophosphate (54 mg, 0.14 mmol), the resulting mixture was stirred at 0°C for 0.5 h. The reaction mixture was concentrated under reduced pressure and the residue was purified by flash column chromatography on silica gel (methanol: dichloromethane = 0-100%) to give **9a** (80 mg) with 90% yield.

MS-ESI calc. for [M+H]⁺595, found 595.

### Step 2

Trifluoroacetic acid (0.4 mL) was added to a solution of **9a** (60 mg, 0.10 mmol) in anhydrous dichloromethane (4 mL) under ice-bath, the resulting mixture was stirred at room temperature for 2.5 h. The reaction mixture was concentrated under reduced pressure and the residue was purified by flash column chromatography on silica gel (methanol: dichloromethane = 0-100%) to give **9** (15 mg) with 30% yield.

MS-ESI calc. for [M+H]⁺495, found 495.

¹H NMR (500 MHz, DMSO-*d₆*) δ 7.74 (s, 2H), 7.58 (s, 1H), 6.32 (d, *J =* 2.0 Hz, 2H), 5.92 (d, *J* = 16.5 Hz, 1H), 5.73 (s, 2H), 5.50 (d, *J* = 16.5 Hz, 1H), 4.85 (s, 2H), 3.56 (s, 2H), 1.92-1.84 (m, 2H), 0.85 (t, *J =* 7.0 Hz, 3H).

### Example 10

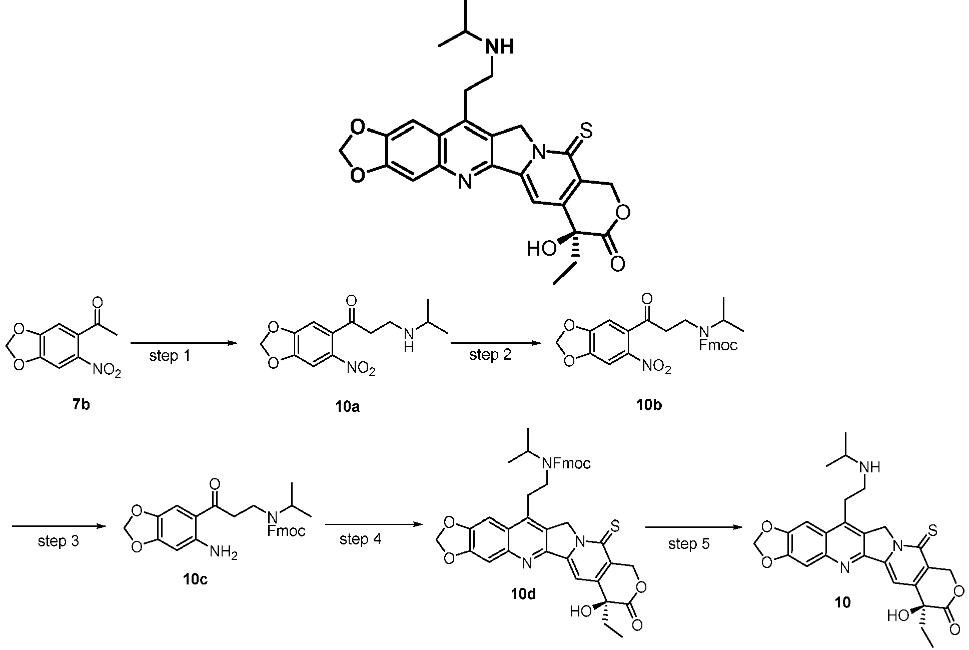

### Step 1

Paraformaldehyde (1.08 g, 35.64 mmol) and isopropylamine (3.43 g, 35.89 mmol) was added to a solution of **7b** (500 mg, 2.39 mmol) in ethanol (2 mL), the resulting mixture was stirred at 100°C for 72 h. The reaction mixture was concentrated under reduced pressure. The residue was diluted with water (10 mL) followed by the addition of saturated aqueous sodium bicarbonate to adjust pH to 7. The aqueous phase was extracted with dichloromethane (30 mL x 3), and the combined organic phases were dried over anhydrous sodium sulfate and filtered. The filtrate was concentrated under reduced pressure and the residue was purified by flash column chromatography on silica gel (methanol: dichloromethane = 0-100%) to give **10a** (80 mg) with 12% yield.

MS-ESI calc. for [M+H]⁺281, found 281.

¹H NMR (400 MHz, CDCl₃) δ 7.73 (s, 1H), 7.29 (s, 1H), 6.30 (s, 2H), 3.40-3.20 (m, 5H), 1.24 (d, *J =* 6.8 Hz, 6H).

### Step 2

9-Fluorenylmethyl chloroformate (74 mg, 0.29 mmol) and sodium bicarbonate (24 mg, 0.29 mmol) was added to a solution of **10a** (80 mg, 0.29 mmol) in tetrahydrofuran/water mixed solvent (V_{tetrahydrofuran}: V_{water} = 3:1, 2 mL), the resulting mixture was stirred at 25°C for 12 h. The reaction mixture was concentrated under reduced pressure. The residue was diluted with water (10 mL), which was extracted with dichloromethane (20 mL x 3). The combined organic phases were dried over anhydrous sodium sulfate and filtered. The filtrate was concentrated under reduced pressure and the residue was purified by flash column chromatography on silica gel (ethyl acetate: hexanes = 0-100%) to give **10b** (70 mg) with 78% yield.

MS-ESI calc. for [M+H]⁺503, found 503.

### Step 3

Wet Pd/C (10% w/w, 54 mg) was added to a solution of **10b** (540 mg, 1.07 mmol) in ethanol (10 mL), and the resulting mixture was stirred at 40 °C for 4 h under H₂ atomosphere. The reaction mixture was filtered to remove insoluble solid and the filtrate was concentrated under reduced pressureand the residue was purified by flash column chromatography on silica gel (ethyl acetate: hexanes = 0-100%) to give **10c** (360 mg) with 71% yield.

MS-ESI calc. for [M+H]⁺473, found 473.

### Step 4

**1** (89 mg, 0.32 mmol) and p-toluenesulfonic acid (28 mg, 0.16 mmol) was added to a solution of **10c** (150 mg, 0.32 mmol) in anhydrous toluene (4 mL), the resulting mixture was stirred at 120°C for 4 h. The reaction mixture was concentrated under reduced pressure and the residue was purified by flash column chromatography on silica gel (methanol: dichloromethane = 0-100%) to give **10d** (70 mg) with 31% yield.

MS-ESI calc. for [M+H]⁺716, found 716.

### Step 5

Piperidine (19 mg, 0.22 mmol) was added to a solution of **10d** (80 mg, 0.11 mmol) in tetrahydrofuran (2 mL), the reaction mixture was stirred at 40°C for 12 h. The reaction mixture was concentrated under reduced pressure and the residue was purified by prep-TLC to give **10** (30 mg) with 55% yield.

MS-ESI calc. for [M+H]⁺ 494, found 494.

¹H NMR (400 MHz, DMSO-*d₆*) δ 7.71 (s, 1H), 7.63 (s, 1H),7.49 (s, 1H), 6.28 (s, 2H), 5.91 (d, *J =* 16.4 Hz, 1H), 5.47 (d, *J =* 16.8 Hz, 1H), 5.45-5.40 (m, 1H), 3.36-3.28 (m, 2H), 3.19-3.10 (m, 1H), 3.09-3.00 (m, 2H), 1.93-1.82 (m, 2H), 1.14 (d, *J =* 6.4 Hz, 6H), 0.85 (t, *J= 7.2* Hz, 3H).

### Example 11

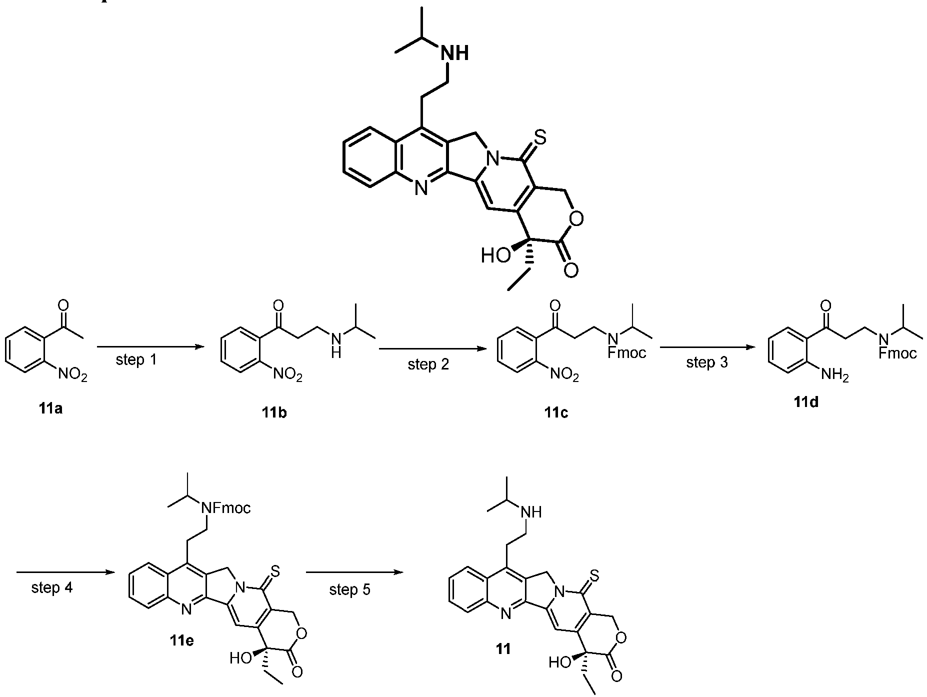

### Step 1

Paraformaldehyde (6.82 g, 227.11 mmol) andisopropylamine (21.70 g, 227.06 mmol) was added to a solution of **11a** (2.5 g, 215.14 mmol) in ethanol (30 mL), the resulting mixture was stirred at 100°C for 72 h. The reaction mixture was concentrated under reduced pressure. The residue was diluted with water (30 mL) followed by the addition of saturated aqueous sodium bicarbonate to adjust pH to ~7. The aqueous phase was extracted with dichloromethane (100 mL x 3), and the combined organic phases were dried over anhydrous sodium sulfate and filtered. The filtrate was concentrated under reduced pressure and the residue was purified by flash column chromatography on silica gel (methanol: dichloromethane = 0-100%) to give **11b** (2.20 g) with 61% yield.

MS-ESI calc. for [M+H]⁺237, found 237.

### Step 2

9-Fluorenylmethyl chloroformate (3.75 g, 14.50 mmol) and sodium bicarbonate (1.26 g, 14.50 mmol) was added to a solution of **11b** (2.20 g, 9.65 mmol) in tetrahydrofuran/water mixed solvent (V_{tetrahydrofuran}: V_{water} = 3:1, 13 mL), the resulting mixture was stirred at 25°C for 12 h. The reaction mixture was concentrated under reduced pressure. The residue was diluted with water (30 mL), which was extracted with dichloromethane (20 mL x 3). The combined organic phases were dried over anhydrous sodium sulfate and filtered. The filtrate was concentrated under reduced pressure and the residue was purified by flash column chromatography on silica gel (ethyl acetate: hexanes = 0-100%) to give **11c** (345 mg) with 8% yield.

MS-ESI calc. for [M+H]⁺ 459, found 459.

### Step 3

Wet Pd/C (10% w/w, 35 mg) was added to a solution of **11c** (345 mg, 0.72 mmol) in ethanol (10 mL), and the resulting mixture was stirred at 40 °C for 4 h under H₂ atomosphere. The reaction mixture was filtered to remove insoluble solid and the filtrate was concentrated under reduced pressureand the residue was purified by flash column chromatography on silica gel (ethyl acetate: hexanes = 0-100%) to give **11d** (220 mg) with 71% yield.

MS-ESI calc. for [M+H]⁺429, found 429.

### Step 4

1 (66 mg, 0.24 mmol) and p-toluenesulfonic acid (20 mg, 0.12 mmol) was added to a solution of **11d** (100 mg,0.23 mmol) in anhydrous toluene (4 mL), the resulting mixture was stirred at 120°C for 4 h. The reaction mixture was concentrated under reduced pressure and the residue was purified by flash column chromatography on silica gel (methanol: dichloromethane = 0-100%) to give **11e** (100 mg) with 64% yield.

MS-ESI calc. for [M+H]⁺672, found 672.

### Step 5

Piperidine (21 mg, 0.25 mmol) was added to a solution of **11e** (80 mg, 0.12 mmol) in tetrahydrofuran (2 mL), the reaction mixture was stirred at 40°C for 12 h. The reaction mixture was concentrated under reduced pressure and the residue was purified by prep-TLC to give **11** (6 mg) with 11% yield.

MS-ESI calc. for [M+H]⁺450, found 450.

### Example 12

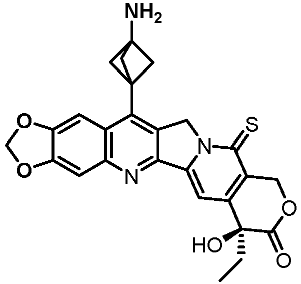

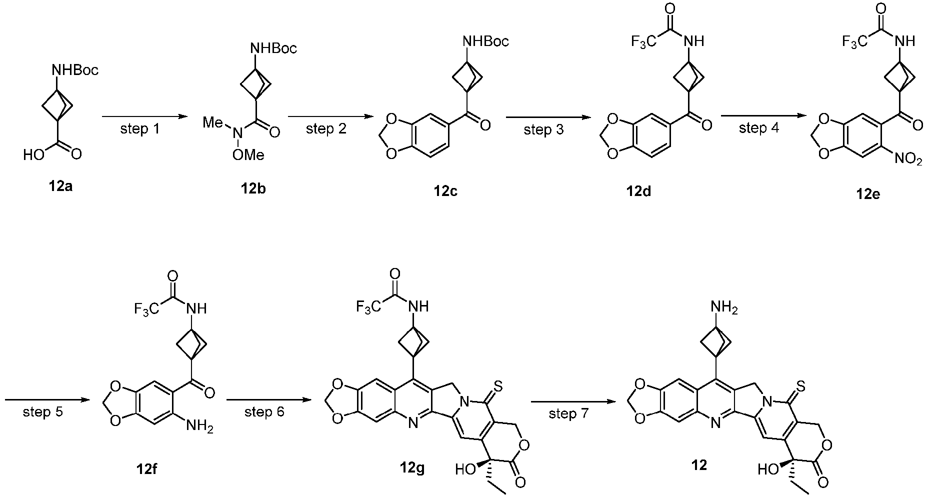

### Step 1

O-(7-Azabenzotriazol-1-yl)-N,N,N',N'-tetramethyluroniumhexafluorophosphate (12.55 g, 33.00 mmol), N,N-diisopropylethylamine (5.69 g, 44.00 mmol), N-methyl-N-methoxymethylamine hydrochloride (1.41 g, 33.00 mmol) were added to a solution of **12a** (5.00 g, 22.00 mmol) in dichloromethane (450 mL), the resulting mixture was stirred at 25°C for 12 h. The reaction mixture was diluted with dichloromethane (200 mL) followed by the addition of HCl_{(aq)} (1N, 50 mL), and the layers were separated. The organic phase was washed sequentially with saturated aqueous sodium bicarbonate (50 mL x 1) and brine (50 mL x 1), dried over anhydrous sodium sulfate and filtered. The filtrate was concentrated under reduced pressure and the residue was purified by flash column chromatography on silica gel (ethyl acetate: hexanes= 0-100%) to give **12b** (5.30 g) with 89% yield.

### Step 2

4-Bromo-1,2-methylenedioxybenzene (9.37 g, 46.61 mmol) was dissolved in tetrahydrofuran (100 mL), and the solution was then cooled to -65°C followed by dropwise addition of n-butyllithium (1.6 M hexane solution, 29 mL), the resulting mixtue was stirred for 0.5 h at -65°C, a solution of **12b** (5.04 g, 18.64 mmol) in anhydrous tetrahydrofuran (40 mL) was added. After the addition, the reaction was kept stirring at -65°C for 3 h. The reaction mixture was quenched with saturated aqueous ammonium chloride solution (500 mL), extracted with ethyl acetate (500 mL x 3). The combined organic phases were washed with brine (500 mL x 1), dried over anhydrous sodium sulfate and filtered. The filtrate was concentrated under reduced pressure and the residue was purified by flash column chromatography on silica gel (ethyl acetate: hexanes= 0-100%) to give **12c** (4.00 g) with 45% yield.
¹H NMR (400 MHz, CDCl₃) δ 7.63 (d, *J =* 8.0 Hz, 1H), 7.44 (s, 1H), 6.82 (d, *J =* 8.4 Hz, 1H), 6.03 (s, 2H), 2.50 (s, 6H), 1.46 (s, 9H).

### Step 3

**12c** (3.00 g, 9.05 mmol) was dissolved in dichloromethane (100 mL), and the solution was then cooled to 0°C followed by the addition of trifluoroacetic acid (27.61 g, 242.15 mmol), the resulting mixtue was stirred at 25°C for 3 h. The reaction mixture was concentrated under reduced pressure and the residue was dissolved in dichloromethane (40 mL), and the above solution was cooled to 0°C followed by dropwise addition of N,N-diisopropylethylamine (7.42 g, 57.41 mmol) and trifluoroacetic anhydride (3.85 g, 18.33 mmol), the resulting mixtue was warmed to 25°C and stirred for 4 h.The reaction mixture was quenched with water (200 mL), extracted with ethyl acetate (200 mL x 3). The combined organic phases were washed with brine (200 mL x 1), dried over anhydrous sodium sulfate and filtered. The filtrate was concentrated under reduced pressure and the residue was purified by flash column chromatography on silica gel (ethyl acetate: hexanes= 0-100%) to give **12d** (2.50 g) with 84% yield.

### Step 4

**12d** (2.50 g, 7.64 mmol) was dissolved in acetic anhydride (5.8 mL), and the solution was then cooled to 0°C followed by the addition of copper nitrate (860 mg, 4.59 mmol) in batches, the resulting mixtue was kept stirring at 0°C for 3 h. The reaction mixture was quenched with water (30 mL), extracted with ethyl acetate (30 mL x 3). The combined organic phases were dried over anhydrous sodium sulfate and filtered. The filtrate was concentrated under reduced pressure and the residue was purified by flash column chromatography on silica gel (ethyl acetate: hexanes= 0-100%) to give **12e** (900 mg) with 75% yield.

MS-ESI calc. for [M+H]⁺373, found 373.

¹H NMR (400 MHz, CDCl₃) δ 7.60 (s, 1H), 6.66 (br s, 1H), 6.64 (s, 1H), 6.21 (s, 2H), 2.43 (s, 6H).

### Step 5

Wet Pd/C (10% w/w, 40 mg) was added to a solution of **12e** (400 mg, 1.07 mmol) in ethanol (10 mL), and the resulting mixture was stirred at 40°C for 4 h. The reaction mixture was concentrated under reduced pressure and the residue was purified by silica gel column chromatography (ethyl acetate: hexanes = 0-100%) to give **12f** (220 mg) with 60% yield.

MS-ESI calc. for [M+H]⁺343, found 343.

### Step 6

**1** (82 mg, 0.32 mmol) and *p*-toluenesulfonic acid (26 mg, 0.15 mmol) was added to a solution of **12f** (100 mg, 0.29 mmol) in anhydrous toluene (4 mL), the resulting mixture was stirred at 120°C for 4 h. The reaction mixture was concentrated under reduced pressure and the residue was purified by flash column chromatography on silica gel (methanol: dichloromethane = 0-100%) to give **12g** (80 mg) with 47% yield.

### Step 7

HCl_{(aq)} (1.6 mL, 4M) were added to a solution of **12g** (80 mg, 0.14 mmol) in methanol (4 mL) and the resulting mixture was stirred at 65°C for 4 h. The reaction mixture was concentrated under reduced pressure and the residue was purified by flash column chromatography on silica gel (methanol: dichloromethame = 0-100%) to give **12** (14 mg) with 21% yield.

MS-ESI calc. for [M+H]⁺490, found 490.

¹H NMR (400 MHz, DMSO-*d₆*) δ 7.74 (s, 1H), 7.64 (s, 1H), 7.58 (s, 1H), 6.32 (s, 2H), 5.92 (d, *J =* 16.4 Hz, 1H), 5.61 (s, 2H), 5.50 (d, *J =* 16.4 Hz, 1H), 2.81 (s, 6H), 1.93-1.84 (m, 2H), 0.84 (t, *J =* 7.2 Hz, 3H).

### Example 13

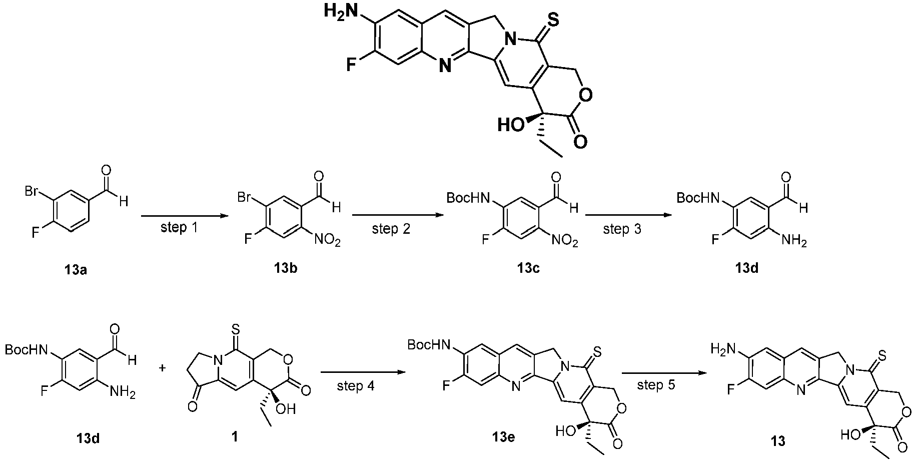

### Step 1

**13a** (15 g, 73.90 mmol) was added in batches to a solvent mixture of concentrated sulfuric acid (45 mL) and concentrated nitric acid (12 mL) under ice-bath, the resulting mixture was slowly warmed to room temperature and stirred for 5 h. The reaction mixture was poured into ice-water (1L) to give a yellow solid, which was collected and dried to give **13b** (22 g).

### Step 2

To a schlenk tube was added **13b** (750 mg, 3.02 mmol), *tert*-butyl carbamate (424 mg, 3.62 mmol), Pd₂(dba)₃ (138 mg, 0.15 mmol), XPhos (288 mg, 0.60 mmol), and cesium carbonate (1.96 g, 6.04 mmol) sequentially, and the Schlenk tube was evacuated and backfilled with nitrogen for 3 times followed by the addition of anhydrous toluene (8 mL), the Schlenk tube was sealed and the resulting mixture was stirred at 90°C overnight. The reaction mixture was diluted with ethyl acetate (100 mL), washed successively with water (25mL x 1) and brine (25 mL x 2). The organic phase was dried and filtered. The filtrate was concentrated under reduced pressure and the residue was purified by flash column chromatography on silica gel (ethyl acetate: hexanes= 0-100%) to give **13c** (550 mg) with 64% yield.

¹H NMR (400 MHz, DMSO-*d₆*) δ 10.44 (s, 1H), 8.75 (d, *J =* 6.0 Hz, 1H), 7.92 (d, *J =* 8.4 Hz, 1H), 7.05 (s, 1H), 1.55 (s, 9H).

### Step 3

**13c** (500 mg, 1.76 mmol) and ammonium chloride (156 mg, 2.94 mmol) was dissolved in a mixed solvent of ethanol (8 mL) and water (2 mL), iron powder (394 mg, 7.04 mmol) was added in batches, the resulting mixture was heated to 60°C and stirred for 2 h. The reaction solution was diluted by ethyl acetate (100 mL), the organic phase was washed by water (25 mL x 1) and brine (25 mL x 1) sequentially, dried and filtered. The filtrate was concentrated under reduced pressure and the residue was purified by flash column chromatography on silica gel (ethyl acetate: hexanes = 0-100%) to give **13d** (310 mg) with 69% yield.

MS-ESI calc. for [M+H]⁺255, found 255.

### Step 4

To a Schlenk tube (100 mL) was added **13d** (250 mg, 0.98 mmol), 1 (167 mg, 0.60 mmol) and *p*-toluenesulfonic acid (26 mg, 0.15 mmol) successively followed by addition of anhydrous toluene (3 mL), the resulting mixture was stirred at 110°C for 2 h. The reaction mixture was concentrated under reduced pressure and the residue was purified by flash column chromatography on silica gel (methanol: dichloromethane = 0-100%) to give crude **13e** (180 mg) directly for the next step.

MS-ESI calc. for [M+H]⁺498, found 498.

### Step 5

**13e** (45 mg, 0.09 mmol) was dissolved in anhydrous dichloromethane (1 mL), and the solution was then cooled to 0°C followed by the addition of trifluoroacetic acid (0.3 mL), the resulting mixtue was stirred at roomtemperature for 5 h. The reaction mixture was concentrated under reduced pressure and the residue was purified by flash column chromatography on silica gel (methanol: dichloromethane = 0-100%) to give **9** (20 mg) with 30% yield.

MS-ESI calc. for [M+H]⁺398, found 398.

¹H NMR (400 MHz, DMSO-*d₆*) δ 8.39 (s, 1H), 7.80 (d, *J* = 12.4 Hz, 1H), 7.70 (s, 1H),7.16 (d, *J =* 9.6 Hz, 1H), 5.90 (d, *J =* 16.4 Hz, 1H), 5.46 (d, *J =* 16.4 Hz, 1H), 5.43 (s, 2H), 1.93-1.80 (m, 2H), 0.85 (t, *J =* 7.2 Hz, 3H).

### Example 14

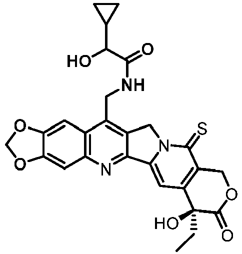

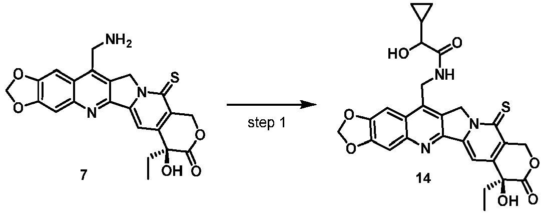

### Step 1

To a single neck flask (25 mL) was successively added 7 (20 mg, 0.05 mmol) and 2-cyclopropyl-2-hydroxyacetic acid (12 mg, 0.10 mmol) followed by the addition of anhydrous dichloromethane (1.0 mL), the mixture was cooled to 0°C, triethylamine (10 mg, 0.10 mmol) and O-(7-azabenzotriazol-1-yl)-N,N,N',N'-tetramethyluronium hexafluorophosphate (23 mg, 0.06 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **14** (12 mg) with 49% yield.

MS-ESI calc. for [M+H]⁺536, found 536.

¹H NMR (400 MHz, DMSO-*d₆*) δ 8.66 (t, *J =* 5.6 Hz, 1H), 7.87 (s,1H), 7.74 (s, 1H), 7.54 (s, 1H), 6.30 (s, 2H), 5.92 (d, *J =* 16.4 Hz, 1H), 5.74 (s, 2H), 5.49 (d, *J =* 16.4 Hz, 1H), 4.75 (d, *J =* 5.2 Hz, 2H), 3.56-3.51 (m, 1H), 1.94-1.83 (m, 2H), 1.07-0.97 (m, 1H), 0.85 (t, *J =* 7.2 Hz, 3H), 0.39-0.29 (m, 2H), 0.28-0.18 (m, 2H).

### Example 15

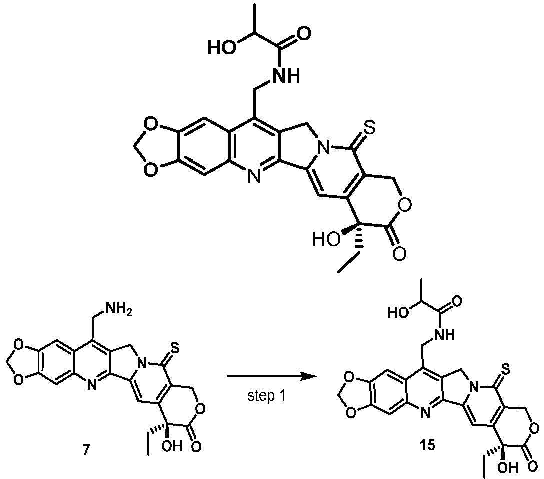

### Step 1

To a single neck flask (25 mL) was successively added 7 (20 mg, 0.05 mmol) and lactic acid (9 mg, 0.10 mmol) followed by the addition of anhydrous dichloromethane (1.0 mL), the mixture was cooled to 0°C, N,N-diisopropylethylamine (20 mg, 0.15 mmol) and O-(7-azabenzotriazol-1-yl)-N,N,N',N'-tetramethyluronium hexafluorophosphate (20 mg, 0.05 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **15** (10 mg) with 43% yield.

MS-ESI calc. for [M+H]⁺510, found 510.

¹H NMR (400 MHz, DMSO-*d₆*) δ 8.74 (t, *J =* 5.2 Hz, 1H), 7.89 (s, 1H), 7.76 (s, 1H), 7.57 (s, 1H), 6.32 (s, 2H), 5.94 (d, *J =* 16.8 Hz, 1H), 5.74 (s, 2H), 5.51 (d, *J =* 16.4 Hz, 1H), 4.77 (d, *J =* 5.6 Hz, 2H), 4.05-3.96 (m, 1H), 1.94-1.82 (m, 2H), 0.88 (t, *J =* 7.2 Hz, 3H).

### Example 16

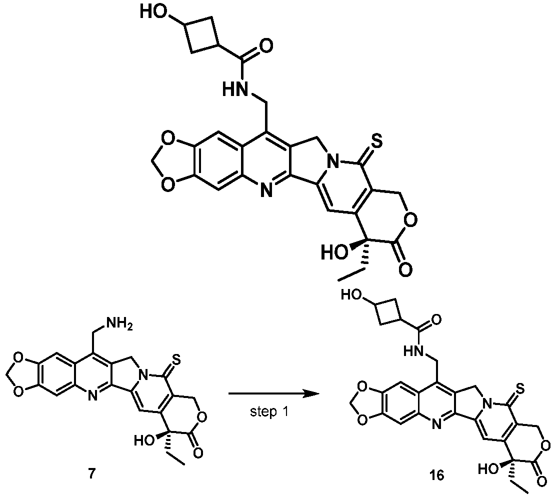

### Step 1

To a single neck flask (25 mL) was successively added 7 (20 mg, 0.05 mmol) and 3-hydroxycyclobutanecarboxylic acid (12 mg, 0.10 mmol) followed by the addition of anhydrous dichloromethane (1.0 mL), the mixture was cooled to 0°C, N,N-diisopropylethylamine (17 mg, 0.13 mmol) and O-(7-azabenzotriazol-1-yl)-N,N,N',N'-tetramethyluronium hexafluorophosphate (23 mg, 0.06 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **16** (15 mg) with 61% yield.

MS-ESI calc. for [M+H]⁺536, found 536.

¹H NMR (400 MHz, DMSO-*d₆*) δ 7.72 (s, 1H), 7.69 (s, 1H), 7.52 (s, 1H), 6.29 (s, 2H), 5.91 (d, *J* = 16.4 Hz, 1H), 5.58 (s, 2H), 5.48 (d, *J* = 16.8 Hz, 1H), 4.77-4.69 (m, 2H), 3.97-3.82 (m, 1H), 2.45-2.35 (m, 1H), 2.30-2.22 (m, 2H), 2.00-1.82 (m, 4H), 0.85 (t, *J =* 7.2 Hz, 3H).

### Example 17

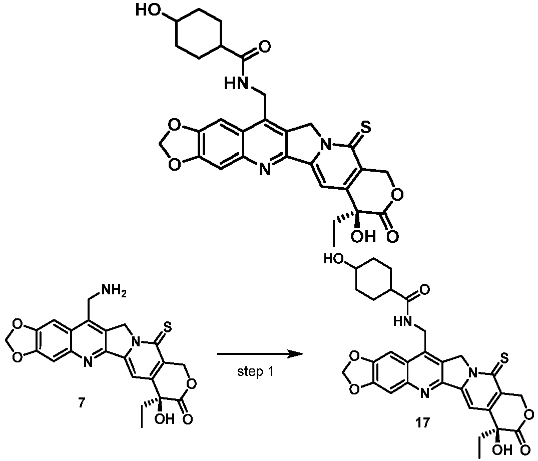

### Step 1

To a single neck flask (25 mL) was successively added **7** (20 mg, 0.05 mmol) and 4-hydroxycyclohexanecarboxylic acid (14 mg, 0.10 mmol) followed by the addition of anhydrous dichloromethane (1.0 mL), the mixture was cooled to 0°C, N,N-diisopropylethylamine (17 mg, 0.13 mmol) and O-(7-azabenzotriazol-1-yl)-N,N,N',N'-tetramethyluronium hexafluorophosphate (23 mg, 0.06 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **17** (9 mg) with 35% yield.

MS-ESI calc. for [M+H]⁺564, found 564.

¹H NMR (400 MHz, DMSO-*d₆*) δ 8.60 (s, 1H), 7.73(s, 1H), 7.71 (s, 1H), 7.54 (s, 1H), 6.30 (s, 2H), 5.92 (d, *J* = 16.8 Hz, 1H), 5.67-5.57 (m, 2H), 5.48 (d, *J* = 16.4 Hz, 1H), 4.79-4.70 (m, 2H), 3.35-3.22 (m, 1H), 2.12-2.00 (m, 1H), 1.92-1.84 (m, 2H), 1.84-1.77 (m, 2H), 1.77-1.67 (m, 2H), 1.42-1.32 (m, 2H), 1.15-1.02 (m, 2H), 0.85 (t, *J =* 7.2 Hz, 3H).

### Example 18

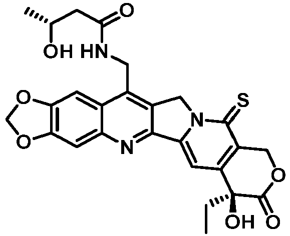

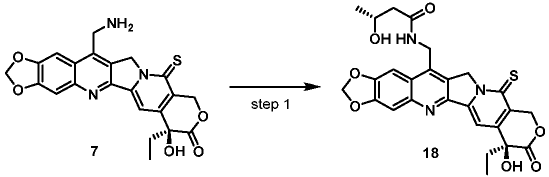

### Step 1

To a single neck flask (25 mL) was successively added **7** (20 mg, 0.05 mmol) and (R)-3-hydroxybutyric acid (10 mg, 0.10 mmol) followed by the addition of anhydrous dichloromethane (1.0 mL), the mixture was cooled to 0°C, N,N-diisopropylethylamine (20 mg, 0.15 mmol) and O-(7-azabenzotriazol-1-yl)-N,N,N',N'-tetramethyluronium hexafluorophosphate (23 mg, 0.06 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **18** (17 mg) with 71% yield.

MS-ESI calc. for [M+H]⁺524, found 524.

¹H NMR (400 MHz, DMSO-*d₆*) δ 7.730 (s, 1H), 7.726 (s, 1H),7.53 (s, 1H), 6.29 (d, *J* = 2.0 Hz, 2H), 5.92 (d, *J =* 16.4 Hz, 1H), 5.64 (s, 2H), 5.49 (d, *J =* 16.4 Hz, 1H), 4.81-4.65 (m, 2H), 4.00-3.89 (m, 1H), 2.29-2.20 (m, 1H), 2.17-2.09 (m, 1H), 1.94-1.82 (m, 2H), 0.99 (d, *J =* 6.4 Hz, 3H), 0.85 (t, *J =* 7.2 Hz, 3H).

### Example 19

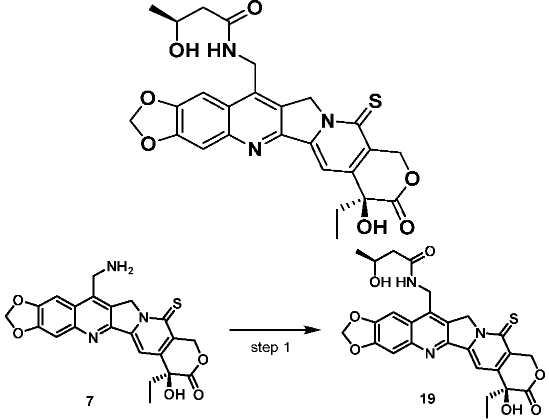

### Step 1

To a single neck flask (25 mL) was successively added **7** (20 mg, 0.05 mmol) and (S)-3-hydroxybutyric acid (10 mg, 0.10 mmol) followed by the addition of anhydrous dichloromethane (1.0 mL), the mixture was cooled to 0°C, N,N-diisopropylethylamine (20 mg, 0.15 mmol) and O-(7-azabenzotriazol-1-yl)-N,N,N',N'-tetramethyluronium hexafluorophosphate (23 mg, 0.06 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **19** (15 mg) with 63% yield.

MS-ESI calc. for [M+H]⁺524, found 524.

¹H NMR (400 MHz, DMSO-*d₆*) δ 8.66 (t, *J =* 5.6 Hz, 1H), 7.73 (s, 2H), 7.54 (s, 1H), 6.30 (s, 2H), 5.92 (d, *J* = 16.4 Hz, 1H), 5.68-5.62 (m, 2H), 5.49 (d, *J* = 16.4 Hz, 1H), 4.77-4.71 (m, 2H), 4.00-3.89 (m, 1H), 2.28-2.21 (m, 1H), 2.17-2.10 (m, 1H), 1.93-1.84 (m, 2H), 1.00 (d, *J =* 6.4 Hz, 3H), 0.85 (t, *J =* 7.2 Hz, 3H).

### Example 20

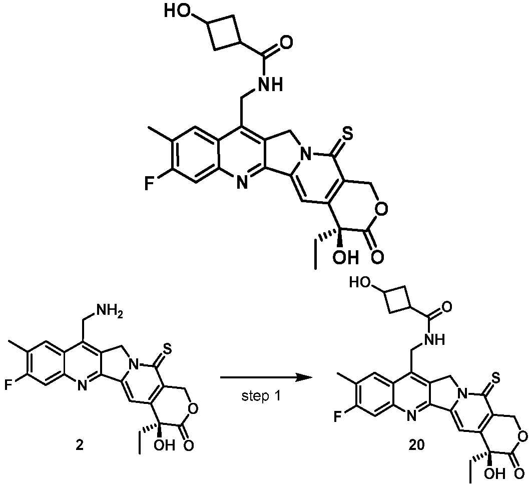

### Step 1

To a single neck flask (25 mL) was successively added **2** (20 mg, 0.05 mmol) and 3-hydroxycyclobutanecarboxylic acid (12 mg, 0.10 mmol) followed by the addition of anhydrous dichloromethane (1.0 mL), the mixture was cooled to 0°C, N,N-diisopropylethylamine (20 mg, 0.15 mmol) and O-(7-azabenzotriazol-1-yl)-N,N,N',N'-tetramethyluronium hexafluorophosphate (23 mg, 0.06 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **20** (15 mg) with 61% yield.

MS-ESI calc. for [M+H]⁺ 524, found 524.

¹H NMR (400 MHz, DMSO-*d₆*) δ 8.30 (d, *J =* 8.0 Hz, 1H), 7.89 (d, *J =* 10.8 Hz, 1H), 7.79 (s, 1H), 5.91 (d, *J =* 16.4 Hz, 1H), 5.61 (s, 2H), 5.49 (d, *J =* 16.8 Hz, 1H), 4.89-4.81 (m, 2H), 3.98-3.85 (m, 1H), 2.46-2.38 (m, 1H), 2.45-2.35 (m, 2H), 1.98-1.82 (m, 4H), 0.85 (t, *J =* 7.2 Hz, 3H).

### Example 21

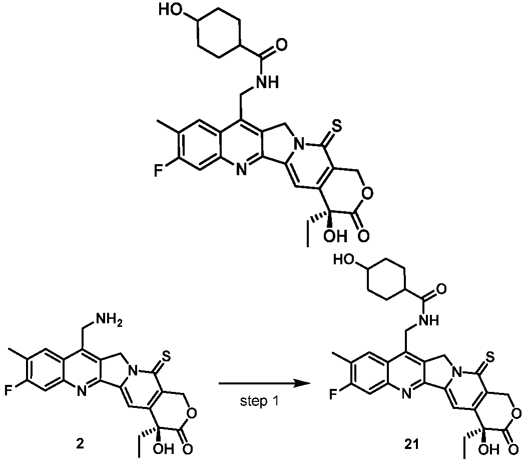

### Step 1

To a single neck flask (25 mL) was successively added 2 (20 mg, 0.05 mmol) and 4-hydroxycyclohexanecarboxylic acid (14 mg, 0.10 mmol) followed by the addition of anhydrous dichloromethane (1.0 mL), the mixture was cooled to 0°C, N,N-diisopropylethylamine (20 mg, 0.15 mmol) and O-(7-azabenzotriazol-1-yl)-N,N,N',N'-tetramethyluronium hexafluorophosphate (23 mg, 0.06 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **20** (22 mg) with 89% yield.

MS-ESI calc. for [M+H]⁺ 552, found 552.

¹H NMR (400 MHz, DMSO-*d₆*) δ 8.30 (d, *J =* 8.0 Hz, 1H), 7.91 (d, *J =* 10.4 Hz, 1H), 7.79 (s, 1H), 5.92 (d, *J* = 16.8 Hz, 1H), 5.66-5.60 (m, 2H), 5.49 (d, *J* = 16.4 Hz, 1H), 3.36-3.25 (m, 1H), 2.14-2.04 (m, 1H), 1.92-1.85 (m, 2H), 1.85-1.68 (m, 4H), 1.55-1.35 (m, 2H), 1.16-1.02 (m, 2H), 0.86 (t, *J =* 7.2 Hz, 3H).

### Example 22

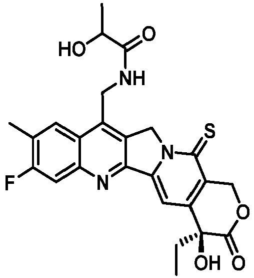

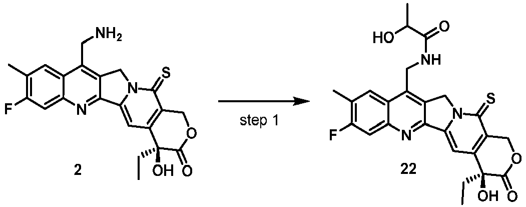

### Step 1

To a single neck flask (25 mL) was successively added **2** (25 mg, 0.06 mmol) and lactic acid (11 mg, 0.10 mmol) followed by the addition of anhydrous dichloromethane (1.0 mL), the mixture was cooled to 0°C, N,N-diisopropylethylamine (23 mg, 0.18 mmol) and O-(7-azabenzotriazol-1-yl)-N,N,N',N'-tetramethyluronium hexafluorophosphate (27 mg, 0.07 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **22** (28 mg) with 96% yield.

MS-ESI calc. for [M+H]⁺498, found 498.

¹H NMR (400 MHz, DMSO-*d₆*) δ 8.74 (t, *J =* 6.0 Hz, 1H), 8.42 (d, *J =* 8.0 Hz, 1H), 7.92 (d, *J =* 10.8 Hz, 1H), 7.79 (s, 1H), 5.92 (d, *J =* 16.4 Hz, 1H), 5.78-5.68 (m, 2H), 5.50 (d, *J =* 16.4 Hz, 1H), 5.00-4.76 (m ,2H), 4.06-3.95 (m, 1H), 1.94-1.82 (m, 2H), 0.86 (t, *J=* 7.2 Hz, 3H).

### Example 23

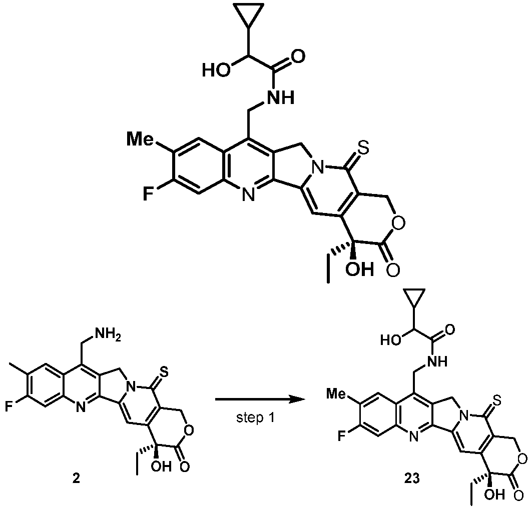

### Step 1

To a single neck flask (25 mL) was successively added **2** (25 mg, 0.06 mmol) and 2-cyclopropyl-2-hydroxyacetic acid (14 mg, 0.12 mmol) followed by the addition of anhydrous dichloromethane (1.0 mL), the mixture was cooled to 0°C, N,N-diisopropylethylamine (23 mg, 0.18 mmol) and O-(7-azabenzotriazol-1-yl)-N,N,N',N'-tetramethyluronium hexafluorophosphate (34 mg, 0.09 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **23** (28 mg) with 91% yield.

MS-ESI calc. for [M+H]⁺524, found 524.

¹H NMR (400 MHz, DMSO-*d₆*) δ 8.69 (t, *J =* 5.6 Hz, 1H), 8.43 (d, *J =* 7.6 Hz, 1H), 7.92 (d, *J =* 10.8 Hz, 1H), 7.80 (s, 1H), 5.92 (d, *J =* 16.4 Hz, 1H), 5.78-5.70 (m, 2H), 5.50 (d, *J =* 16.4 Hz, 1H), 5.00-4.79 (m, 2H), 3.57-3.52 (m, 1H), 1.94-1.82 (m, 2H), 1.09-0.97 (m, 1H), 0.86 (t, *J =* 6.8 Hz, 3H), 0.38-0.28 (m, 2H), 0.28-0.18 (m, 2H).

### Example 24

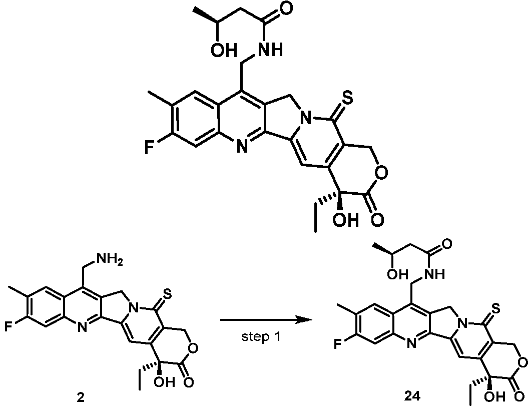

### Step 1

To a single neck flask (25 mL) was successively added **2** (20 mg, 0.05 mmol) and (S)-3-hydroxybutyric acid (10 mg, 0.10 mmol) followed by the addition of anhydrous dichloromethane (1.0 mL), the mixture was cooled to 0°C, N,N-diisopropylethylamine (19 mg, 0.15 mmol) and O-(7-azabenzotriazol-1-yl)-N,N,N',N'-tetramethyluronium hexafluorophosphate (23 mg, 0.06 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **24** (18 mg) with 75% yield.

MS-ESI calc. for [M+H]⁺512, found 512.

¹H NMR (400 MHz, DMSO-*d₆*) δ 8.32 (d, *J =* 8.4 Hz, 1H), 7.91 (d, *J =* 10.4 Hz, 1H), 7.79 (s, 1H), 5.92 (d, *J =* 16.8 Hz, 1H), 5.66 (s, 2H), 5.50 (d, *J =* 16.4 Hz, 1H), 4.91-4.76 (m, 2H), 4.00-3.90 (m, 1H), 2.31-2.22 (m, 1H), 2.20-2.10 (m, 1H), 1.93-1.84 (m, 2H), 0.99 (d, *J =* 6.4 Hz, 3H), 0.85 (d, *J =* 7.2 Hz, 3H).

### Example 25

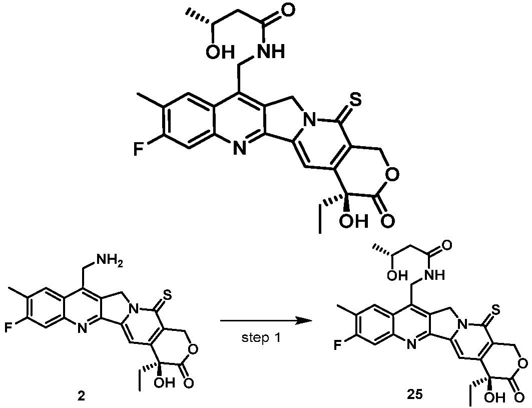

### Step 1

To a single neck flask (25 mL) was successively added **2** (25 mg, 0.06 mmol) and (R)-3-hydroxybutyric acid (13 mg, 0.12 mmol) followed by the addition of anhydrous dichloromethane (1.0 mL), the mixture was cooled to 0°C, N,N-diisopropylethylamine (23 mg, 0.18 mmol) and O-(7-azabenzotriazol-1-yl)-N,N,N',N'-tetramethyluronium hexafluorophosphate (27 mg, 0.07 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **25** (18 mg) with 60% yield.

MS-ESI calc. for [M+H]⁺512, found 512.

¹H NMR (400 MHz, DMSO-*d₆*) δ 8.30 (d, *J =* 8.4 Hz, 1H), 7.89 (d, *J =* 10.8 Hz, 1H), 7.78 (s, 1H), 5.91 (d, *J* = 16.4 Hz, 1H), 5.72-5.56 (m, 2H), 5.49 (d, *J* = 16.8 Hz, 1H), 4.92-4.76 (m, 2H), 4.00-3.90 (m, 1H), 2.30-2.22 (m, 1H), 2.20-2.10 (m, 1H), 1.94-1.82 (m, 2H), 0.99 (d, *J =* 6.4 Hz, 3H), 0.85 (d, *J =* 7.2 Hz, 3H).

### Example 26

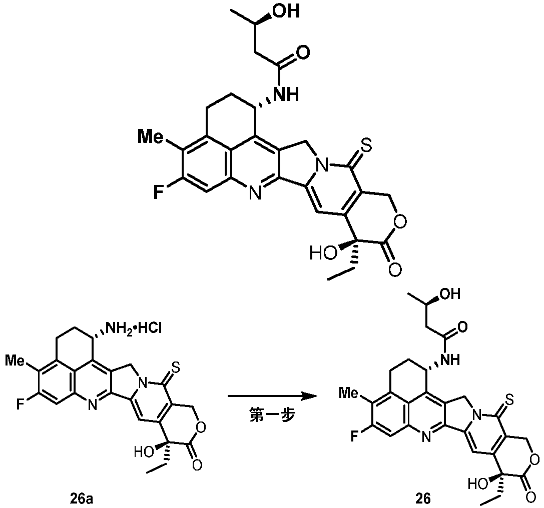

### Step 1

To a single neck flask (25 mL) was successively added **26a** (30 mg, 0.06 mmol) and (S)-3-hydroxybutyric acid (10 mg, 0.12 mmol) followed by the addition of anhydrous dichloromethane (1.0 mL), the mixture was cooled to 0°C, N,N-diisopropylethylamine (20 mg, 0.15 mmol) and O-(7-azabenzotriazol-1-yl)-N,N,N',N'-tetramethyluronium hexafluorophosphate (27 mg, 0.07 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **26** (22 mg) with 67% yield.

MS-ESI calc. for [M+H]⁺538, found 538.

¹H NMR (400 MHz, DMSO-*d₆*) δ 8.52 (d, *J =* 8.8 Hz, 1H), 7.793 (d, *J =* 10.8 Hz, 1H), 7.789 (s, 1H), 5.90 (d, *J =* 16.4 Hz, 1H), 5.63-5.54 (m, 1H), 5.52-5.54 (m, 2H), 5.41-5.32 (m, 1H), 4.15-4.04 (m, 1H), 3.28-3.03 (m, 2H), 2.39 (s, 3H), 2.38-2.25 (m, 2H), 2.22-2.07 (m, 2H), 1.93-1.84 (m, 2H), 1.12 (d, *J =* 6.4 Hz, 3H), 0.85 (t, *J =* 7.2 Hz, 3H).

### Example 27

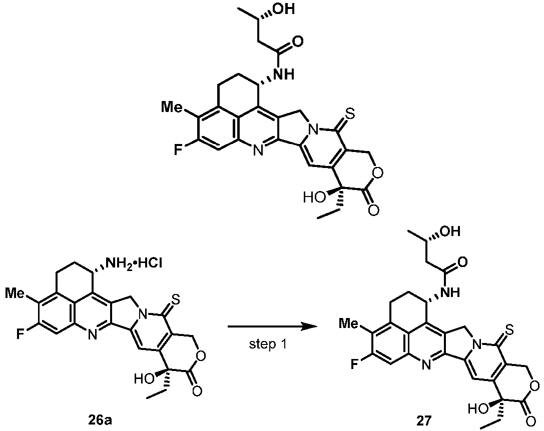

### Step 1

To a single neck flask (25 mL) was successively added **26a** (45 mg, 0.09 mmol) and (R)-3-hydroxybutyric acid (19 mg, 0.18 mmol) followed by the addition of anhydrous dichloromethane (1.0 mL), the mixture was cooled to 0°C, N,N-diisopropylethylamine (30 mg, 0.23 mmol) and O-(7-azabenzotriazol-1-yl)-N,N,N',N'-tetramethyluronium hexafluorophosphate (42 mg, 0.11 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **27** (45 mg) with 91% yield.

MS-ESI calc. for [M+H]⁺538, found 538.

¹H NMR (400 MHz, DMSO-*d₆*) δ 8.50 (d, *J =* 8.4 Hz, 1H), 7.80-7.72 (m, 2H), 5.90 (d, *J* = 16.4 Hz, 1H), 5.62-5.53 (m, 1H), 5.52-5.43 (m, 2H), 5.35 (d, *J* = 20.0 Hz, 1H), 4.12-4.00 (m, 1H), 3.27-3.05 (m, 2H), 2.44-2.39 (m, 1H), 2.38 (s, 3H), 2.28-2.21 (m, 1H), 2.20-2.08 (m, 2H), 1.93-1.83 (m, 2H), 1.10 (d, *J =* 6.0 Hz, 3H), 0.85 (t, *J =* 7.2 Hz, 3H).

### Example 28

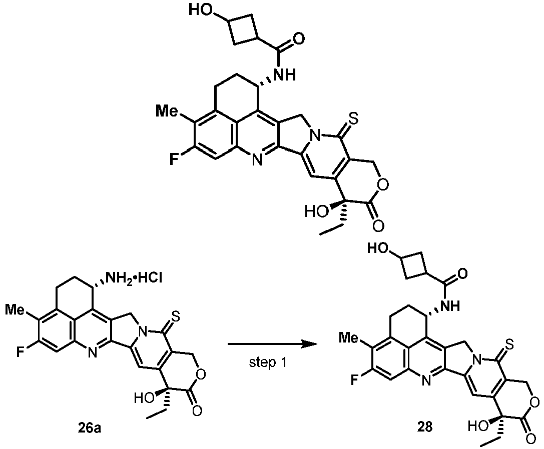

### Step 1

To a single neck flask (25 mL) was successively added **26a** (25 mg, 0.05 mmol) and 3-hydroxycyclobutanecarboxylic acid (12 mg, 0.10 mmol) followed by the addition of anhydrous dichloromethane (1.0 mL), the mixture was cooled to 0°C, N,N-diisopropylethylamine (17 mg, 0.13 mmol) and O-(7-azabenzotriazol-1-yl)-N,N,N',N'-tetramethyluronium hexafluorophosphate (23 mg, 0.06 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **28** (26 mg) with 92% yield.

MS-ESI calc. for [M+H]⁺550, found 550.

¹H NMR (400 MHz, DMSO-*d₆*) δ 7.81-7.75 (m, 2H), 5.90 (d, *J* = 16.4 Hz, 1H), 5.60-5.53 (m, 1H), 5.48 (d, *J =* 16.8 Hz, 1H), 5.41 (d, *J =* 20.0 Hz, 1H), 5.30 (d, *J =* 20.0 Hz, 1H), 4.00-3.89 (m, 1H), 3.30-3.05 (m, 2H), 2.38 (s, 3H), 2.25-2.00 (m, 4H), 1.94-1.83 (m, 2H), 0.85 (t, *J =* 7.2 Hz, 3H).

### Example 29

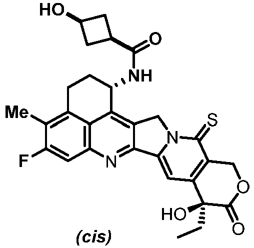

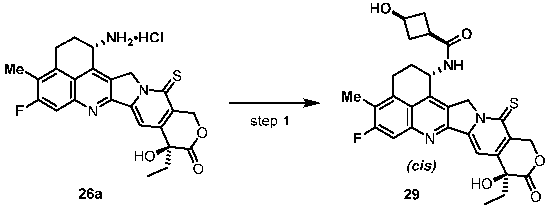

### Step 1

To a single neck flask (25 mL) was successively added **26a** (30 mg, 0.06 mmol) and cis-3-hydroxycyclobutanecarboxylic acid (14 mg, 0.12 mmol) followed by the addition of anhydrous dichloromethane (1.0 mL), the mixture was cooled to 0°C, N,N-diisopropylethylamine (20 mg, 0.15 mmol) and O-(7-azabenzotriazol-1-yl)-N,N,N',N'-tetramethyluronium hexafluorophosphate (27 mg, 0.07 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **29** (33 mg) with 99% yield.

MS-ESI calc. for [M+H]⁺550, found 550.

¹H NMR (400 MHz, DMSO-*d₆*) δ 8.49 (d, *J =* 8.8 Hz, 1H), 7.78 (s, 1H), 7.77 (d, *J* = 10.4 Hz, 1H), 5.90 (d, *J =* 16.4 Hz, 1H), 5.61-5.52 (m, 1H), 5.48 (d, *J =* 16.8 Hz, 1H), 5.40 (d, *J* = 20.0 Hz, 1H), 5.28 (d, *J =* 20.0 Hz, 1H), 4.01-3.89 (m, 1H), 3.30-3.05 (m, 2H), 2.38 (s, 3H), 2.25-2.00 (m, 4H), 1.94-1.82 (m, 2H), 0.85 (t, *J =* 7.2 Hz, 3H).

### Example 30

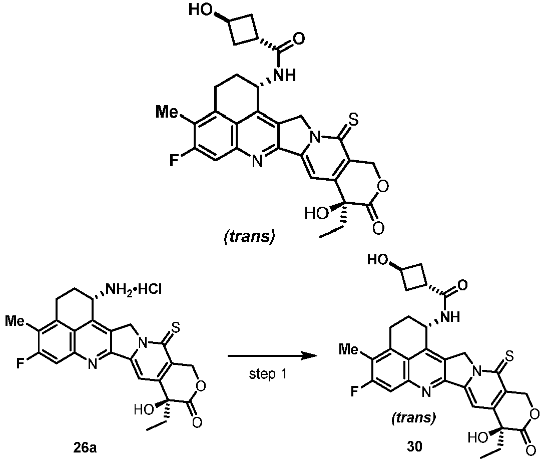

### Step 1

To a single neck flask (25 mL) was successively added **26a** (30 mg, 0.06 mmol) and trans-3-hydroxycyclobutanecarboxylic acid (14 mg, 0.12 mmol) followed by the addition of anhydrous dichloromethane (1.0 mL), the mixture was cooled to 0°C, and N,N-diisopropylethylamine (20 mg, 0.15 mmol) and O-(7-azabenzotriazol-1-yl)-N,N,N',N'-tetramethyluronium hexafluorophosphate (27 mg, 0.07 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **30** (22 mg) with 65% yield.

MS-ESI calc. for [M+H]⁺550, found 550.

¹H NMR (400 MHz, DMSO-*d₆*) δ 8.46 (d, *J =* 8.8 Hz, 1H), 7.79 (d, *J =* 10.4 Hz, 1H), 7.78 (s, 1H), 5.90 (d, *J* = 16.4 Hz, 1H), 5.62-5.53 (m, 1H), 5.47 (d, *J* = 16.4 Hz, 1H), 5.44-5.27 (m, 2H), 4.42-4.32 (m, 1H), 3.28-3.06 (m, 2H), 3.00-2.90 (m, 1H), 2.38 (s, 3H), 2.25-1.98 (m, 4H), 1.94-1.82 (m, 2H), 0.85 (t, *J =* 7.2 Hz, 3H).

### Example 31

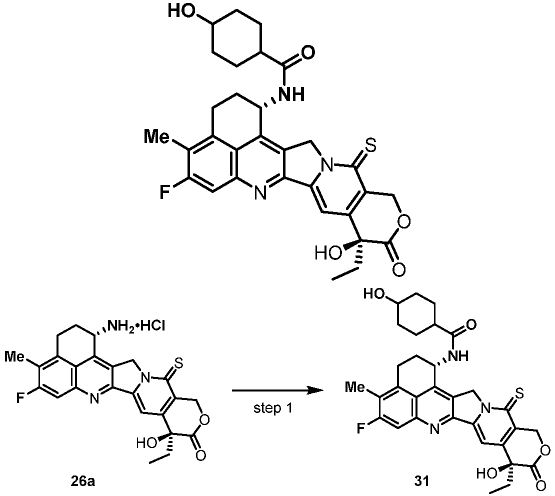

### Step 1

To a single neck flask (25 mL) was successively added **26a** (30 mg, 0.06 mmol) and3-hydroxycyclobutanecarboxylic acid (17 mg, 0.12 mmol) followed by the addition of anhydrous dichloromethane (1.0 mL), the mixture was cooled to 0°C, N,N-diisopropylethylamine (20 mg, 0.15 mmol) and O-(7-azabenzotriazol-1-yl)-N,N,N',N'-tetramethyluronium hexafluorophosphate (27 mg, 0.07 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **31** (32 mg) with 90% yield.

MS-ESI calc. for [M+H]⁺578, found 578.

¹H NMR (400 MHz, DMSO-*d₆*) δ 7.78 (s, 1H), 7.75 (d, *J =* 11.2 Hz, 1H), 5.89 (d, *J =* 16.8 Hz, 1H), 5.60-5.51 (m, 1H), 5.50-5.37 (m, 2H), 5.34-5.20 (m, 1H), 3.40-3.32 (m, 1H), 3.28-3.05 (m, 2H), 2.37 (s, 3H), 2.21-2.11 (m, 2H), 2.10-2.00 (m, 1H), 1.94-1.62 (m, 6H), 1.60-1.35 (m, 2H), 1.19-1.04 (m, 2H), 0.85 (t, *J =* 7.2 Hz, 3H).

### Example 32

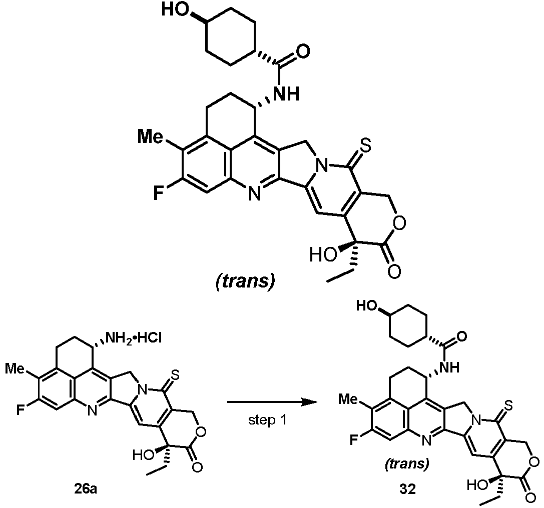

### Step 1

To a single neck flask (25 mL) was successively added **26a** (30 mg, 0.06 mmol) andtrans-4-hydroxycyclohexanecarboxylic acid (17 mg, 0.12 mmol) followed by the addition of anhydrous dichloromethane (1.0 mL), the mixture was cooled to 0°C, N,N-diisopropylethylamine (20 mg, 0.15 mmol) and O-(7-azabenzotriazol-1-yl)-N,N,N',N'-tetramethyluronium hexafluorophosphate (27 mg, 0.07 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **32** (25 mg) with 70% yield.

MS-ESI calc. for [M+H]⁺578, found 578.

¹H NMR (400 MHz, DMSO-*d₆*) δ 8.46 (d, *J =* 8.4 Hz, 1H), 7.80-7.73 (m, 2H), 5.90 (d, *J = 16.8* Hz, 1H), 5.59-5.51 (m, 1H), 5.47 (d, *J =* 16.4 Hz, 1H), 5.43 (d, *J =* 19.6 Hz, 1H), 5.27 (d, *J =* 19.6 Hz, 1H), 3.27-3.05 (m, 2H), 2.38 (s, 3H), 2.22-2.11 (m, 2H), 2.10-2.00 (m, 2H), 1.94-1.77 (m, 6H), 1.58-1.42 (m, 2H), 1.18-1.05 (m, 2H), 0.85 (t, *J* = 7.2 Hz, 3H).

### Example 33

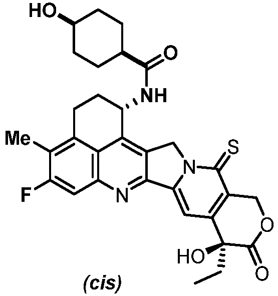

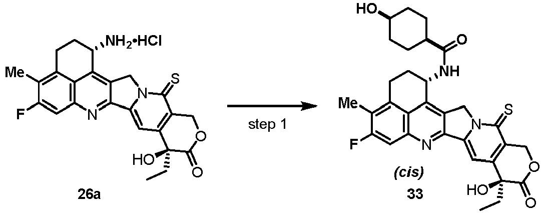

### Step 1

To a single neck flask (25 mL) was successively added **26a** (30 mg, 0.06 mmol) and cis-4-hydroxycyclohexanecarboxylic acid (17 mg, 0.12 mmol) followed by the addition of anhydrous dichloromethane (1.0 mL), the mixture was cooled to 0°C, N,N-diisopropylethylamine (20 mg, 0.15 mmol) and O-(7-azabenzotriazol-1-yl)-N,N,N',N'-tetramethyluronium hexafluorophosphate (27 mg, 0.07 mmol) was added sequentially, the reaction was kept strring at 0°C for 0.5 h. The reaction mixture was concentrated under reduced pressure and the residue was purified by flash column chromatography on silica gel (methanol: dichloromethane = 0-100%) to give **33** (25 mg) with 79% yield.

MS-ESI calc. for [M+H]⁺578, found 578.

¹H NMR (400 MHz, DMSO-*d₆*) δ 8.41 (d, *J =* 8.8 Hz, 1H), 7.78 (s, 1H), 7.77 (d, *J* = 10.8 Hz, 1H), 5.90 (d, *J =* 16.4 Hz, 1H), 5.61-5.52 (m, 1H), 5.47 (d, *J =* 16.4 Hz, 1H), 5.44 (d, *J* = 20.0 Hz, 1H), 5.32 (d, *J =* 20.0 Hz, 1H), 3.28-3.05 (m, 2H), 2.38 (s, 3H), 2.30-2.03 (m, 4H), 1.95-1.80 (m, 4H), 1.73-1.63 (m, 2H), 1.56-1.34 (m, 4H), 0.85 (t, *J* = 7.2 Hz, 3H).

### Biological activity test

### Cell proliferation inhibition test

KPL-4 Cells in the logarithmic phase of growth were collected and re-suspended with fresh RPMI1640 complete cell culture medium, and adjusted to 2×10⁴ cells/mL after cell counting. Cells were seeded into 96-well cell culture plates at 100 µL/well, and placed in a CO₂ incubator (37°C, 5% CO₂) overnight. In the next day, one of the cell culture plates were taken out, balanced to room temperature, and CellTiter-Glo reagent (Promega, USA), which was pre-balanced to room temperature and evenly mixed, was added into the plate at 100 µl. The plate was kept in dark for 30 min, and then luminescence value was read with a microplate reader (denoted as G₀ value). The other cell culture plates were taken out and added with different concentrations of test compounds or DMSO (final concentration 0.5%) in corresponding wells. After incubated for 72h in a CO₂ incubator, the cell culture plates were balanced to room temperature and tested for cell viabilities using CellTiter-Glo reagent as described above (denoted as G₃ value).

DXd was used as a positive reference compound with the following structure:

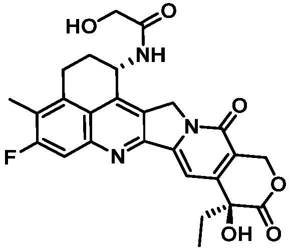

The cell proliferation rates were calculated using the following formula: Cell proliferation rate (%) = (mean of G_{3compound} - mean of G₀) / (mean of G_{3DMSO} - mean of G₀) ×100. Inhibition curves were fitted with GraphPad Prism and GI₅₀ values were obtained (shown in the table below).

**Table 1: Results of cell proliferation inhibition assay**

| Example | GI₅₀ (nM) |
|---|---|
| Example 2 | 2.82 |
| Example 3 | 6.27 |
| Example 4 | 2.23 |
| Example 5 | 1.84 |
| DXd | 45.4^{a} |

| | |
|---|---|
| Note: ^{a}the average result of 2 measurements. | |

**Table 2: Results of cell proliferation inhibition assay**

| Example | GI₅₀ (nM) |
|---|---|
| Example 6 | 3.45 |
| Example 7 | 1.94 |
| Example 8 | 12 |
| Example 9 | 12.2 |
| Example 10 | 2.24 |
| Example 11 | 6.48 |
| Example 12 | 2.98 |
| Example 13 | 0.63 |
| Example 14 | 3.49 |
| Example 15 | 4.13 |
| Example 16 | 14.5 |
| Example 18 | 4.35 |
| Example 19 | 4.27 |
| Example 20 | 8.88 |
| Example 21 | 16.8 |
| Example 22 | 2.81 |
| Example 23 | 1.73 |
| Example 24 | 2.81 |
| Example 25 | 2.21 |
| Example 26 | 3.24 |
| Example 27 | 2.93 |
| Example 28 | 10.8 |
| Example 29 | 5.96 |
| Example 30 | 11.3 |
| Example 31 | 8.62 |
| Example 32 | 8.47 |
| Example 33 | 6.34 |
| DXd | 22.8 |

The results indicated that, the compounds of the application showed high inhibitory activities against proliferation of the tumor cells, with higher potency than DXd. Because the compounds of the application have excellent inhibitory activity against tumor cell proliferation, they can be used as treatment for oncology, or as toxins for the preparation of antibody-drug conjugates for the treatment of tumors.

All documents referred to in the present application are incorporated by reference herein as if each document is individually incorporated by reference. Further, it should be understood that upon reading the above teaching of the present application, various modifications or modifications may be made to the present application by those skilled in the art, and those equivalents also fall within the scope defined by the appended claims of the present application.

## Claims

1. A compound as shown in formula (I), or a pharmaceutically acceptable salt or hydrate thereof: 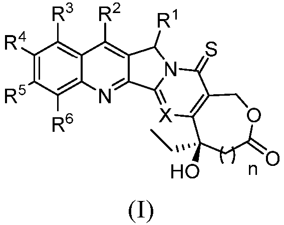 wherein, n is 0 or 1;
X is selected from the group consisting of N and CR⁰;
R⁰ is selected from the group consisting of hydrogen, deuterium, halogen, C₁-C₈ alkyl, C₁-C₈ alkoxy, OH, NH₂, N₃ and NO₂;
R¹ is selected from the group consisting of hydrogen, deuterium, halogen, C₁-C₈ alkyl, C₁-C₈ alkoxy, C₁-C₈ haloalkyl, C₁-C₈ haloalkoxy, N₃, NO₂, NH₂, NH-OH, -NR'R", -COOR', -CONR'R", -NHR‴NR'R"; wherein R', R" and R‴ are each independently selected from hydrogen, alkyl, aryl, arylalkyl, acyl, alkoxycarbonyl, and aryloxycarbonyl;
R², R³, R⁴, R⁵ and R⁶ are each independently selected from the group consisting of hydrogen, deuterium, halogen, hydroxyl, cyano, NH₂, NO₂, substituted or unsubstituted C₁-C₈ alkyl, substituted or unsubstituted C₁-C₈ alkoxy, substituted or unsubstituted C₁-C₈ alkylthio, substituted or unsubstituted C₁-C₈ deuterated alkyl, -(CH₂)ₘtri(C₁-C₄ alkyl)silyl, -(CH₂)ₘ(C₃-C₈ cycloalkyl), -(CH₂)ₘ(3-12 membered heterocyclyl), -(CH₂)ₘN(R⁷)2, -(CH₂)ₘS(CH₂)ₚR⁷, -(CH₂)ₘS(O)(CH₂)ₚR⁷, -(CH₂)ₘS(O)₂(CH₂)ₚR⁷, -(CH₂)ₘNH(CH₂)ₚR⁷, -(CH₂)ₘNHC(O)(CH₂)ₚR⁷, -(CH₂)ₘOC(O)(CH₂)ₚR⁷, -(CH₂)ₘC(O)(CH₂)ₚR⁷, and -CH=N(OtBu); wherein, m and p are each independently 0, 1, 2, 3, or 4;
or R² and R³ together with the carbon atoms to which they are attached form a substituted or unsubstituted C₅-C₈ carbocycle or a substituted or unsubstituted 5-12 membered heterocycle;
or R² and R³ together with the carbon atoms to which they are attached form a structure selected from the group consisting of a saturated or unsaturated 5-6 membered carbocycle that is unsubstituted or substituted by one or more R^{a}, and a saturated or unsaturated 5-6 membered heterocycle that is unsubstituted or substituted by one or more R^{a};
or R³ and R⁴, or R⁴ and R⁵*,* together with the carbon atoms to which they are attached form a structure selected from the group consisting of a saturated or unsaturated 5-12 membered carbocycle that is unsubstituted or substituted by one or more R^{a}, and a saturated or unsaturated 5-12 membered heterocycle that is unsubstituted or substituted by one or more R^{a}; wherein R^{a} is a substituted or unsubstituted substitutent and selected from the group consisting of hydrogen, deuterium, halogen, cyano, nitro, hydroxyl, amino, C₁-C₆ alkyl-NH-, (C₁-C₆ alkyl)₂N-, C₁-C₆ alkyl, C₂-C₆ alkenyl, C₂-C₆ alkynyl, C₁-C₆ alkoxy, allyl, benzyl, C₆-C₁₂ aryl, C₁-C₆ alkoxy-C₁-C₆ alkyl, C₁-C₆ alkoxy-carbonyl, phenoxycarbonyl, C₂-C₆ alkynyl-carbonyl, C₂-C₆ alkenyl-carbonyl, C₃-C₆ cycloalkyl-carbonyl, C₁-C₆ alkyl-sulfonyl, phenyl, 5-7 membered heteroaryl, C₃-C₈ cycloalkyl, 3-12 membered heterocyclyl, -(CH₂)ₘN(R⁷)₂, -(CH₂)ₘS(CH₂)ₚR⁷, -(CH₂)ₘS(O)(CH₂)ₚR⁷, -(CH₂)ₘS(O)₂(CH₂)ₚR⁷, -(CH₂)ₘNH(CH₂)ₚR⁷, -(CH₂)ₘNHC(O)(CH₂)ₚR⁷, -(CH₂)ₘOC(O)(CH₂)ₚR⁷, and -(CH₂)ₘC(O)(CH₂)ₚR⁷; wherein, m and p are each independently 0, 1, 2, 3 or 4, preferably 0, 1 or 2;
each R⁷ is independently selected from the group consisting of hydrogen, deuterium, halogen, substituted or unsubstituted C₁-C₈ alkyl, C₁-C₈ haloalkyl, C₁-C₈ deuterated alkyl, substituted or unsubstituted C₁-C₈ alkoxy, hydroxyl, amino, cyano, nitro, thiol, substituted or unsubstituted C₁-C₈ alkylene-OH, substituted or unsubstituted C₁-C₈ alkylene-NH₂, SO₂Me, -OC(O) (substituted or unsubstituted C₁-C₄ alkyl), -C(O)(substituted or unsubstituted C₁-C₄ alkyl), substituted or unsubstituted phenyl, substituted or unsubstituted 5-7 membered heteroaryl, substituted or unsubstituted C₃-C₈ cycloalkyl, and substituted or unsubstituted 3-12 membered heterocyclyl;
unless otherwise specified, each of the above groups can be substituted by substituents selected from the group consisting of deuterium, halogen, cyano, nitro, hydroxyl, amino, C₁-C₆ alkyl-NH-, (C₁-C₆ alkyl)₂N-, C₁-C₆ alkyl, C₂-C₆ alkenyl, C₂-C₆ alkynyl, C₁-C₆ alkoxy, halogenated C₁-C₆ alkyl, halogenated C₂-C₆ alkenyl, halogenated C₂-C₆ alkynyl, halogenated C₁-C₆ alkoxy, allyl, benzyl, C₆-C₁₂ aryl, C₁-C₆ alkoxy-C₁-C₆ alkyl, C₁-C₆ alkoxy-carbonyl, phenoxycarbonyl, C₂-C₆ alkynyl-carbonyl, C₂-C₆ alkenyl-carbonyl, C₃-C₆ cycloalkyl-carbonyl, C₁-C₆ alkyl-sulfonyl, phenyl, 5-7 membered heteroaryl, C₃-C₈ cycloalkyl, 3-12 membered heterocyclyl;
with the proviso that the compound is other than any structure selected from the group consisting of

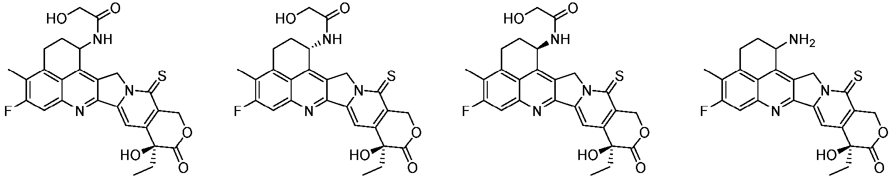

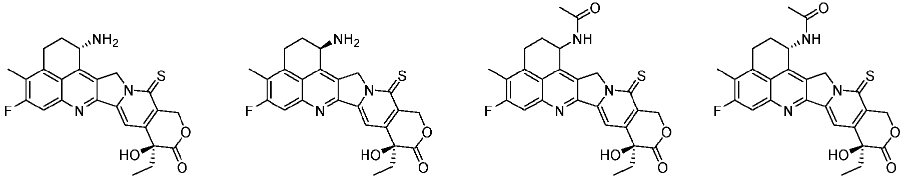

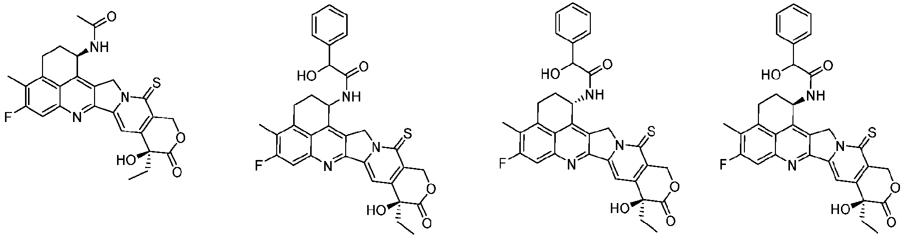

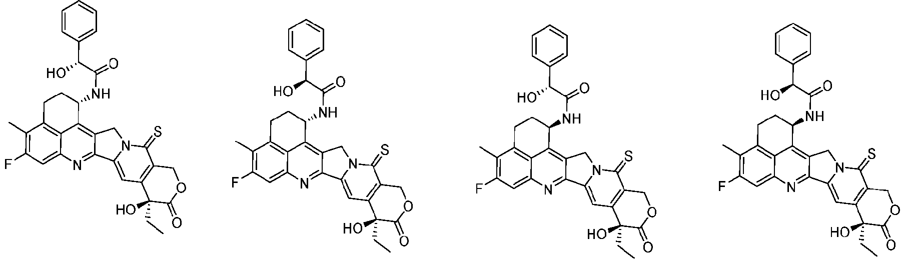

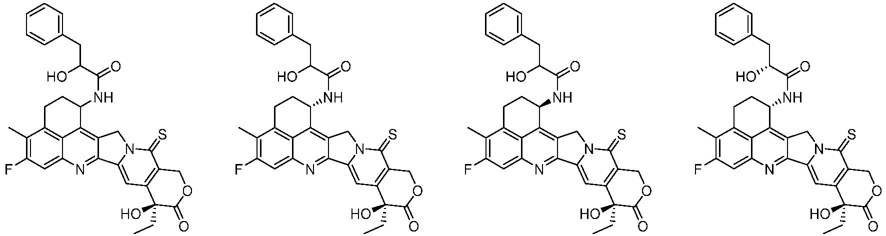

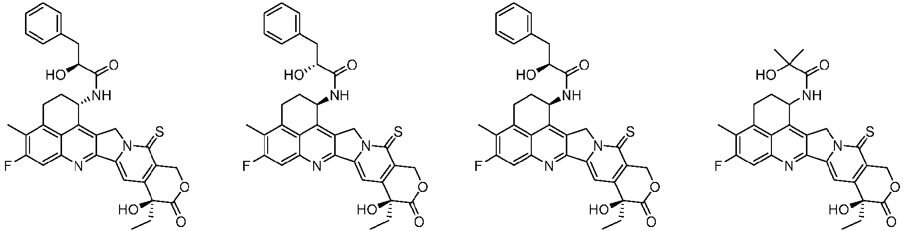

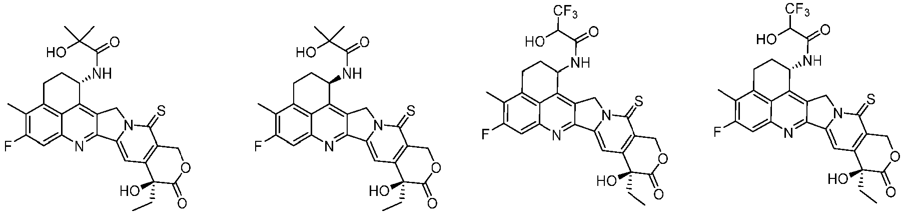

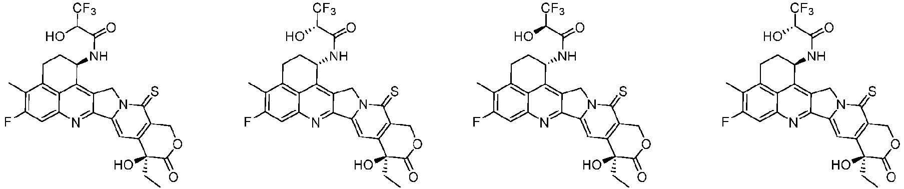

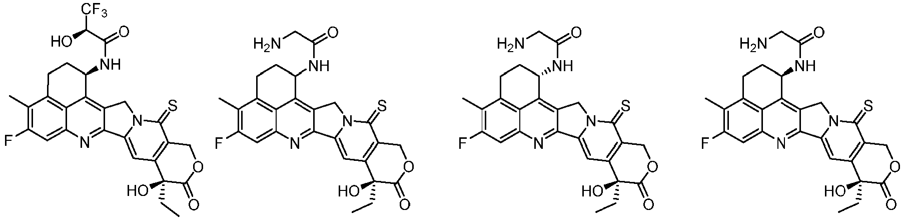

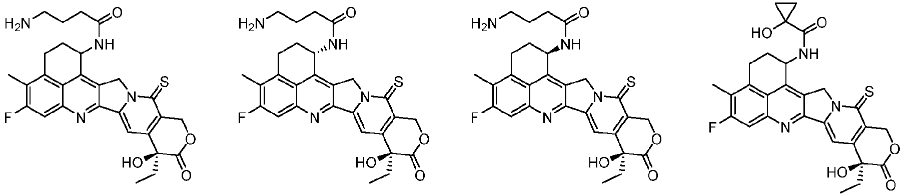

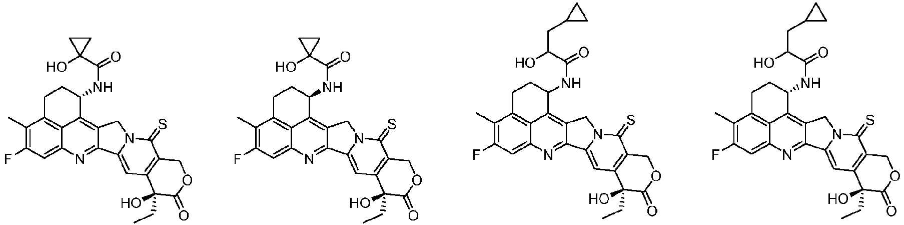

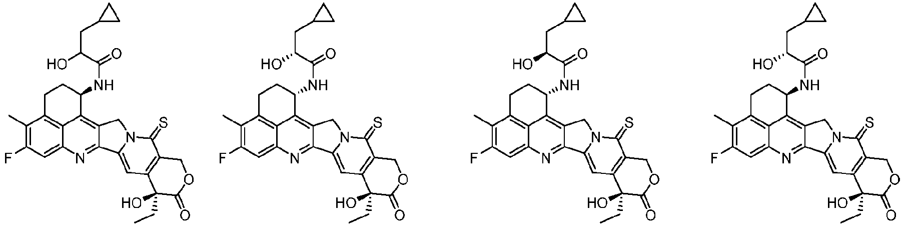

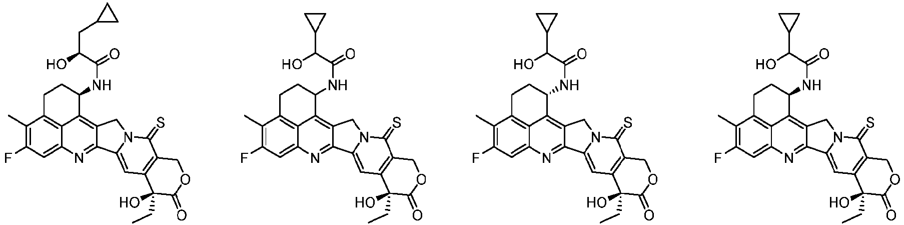

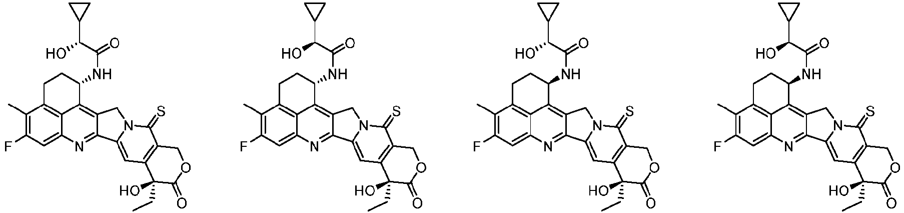

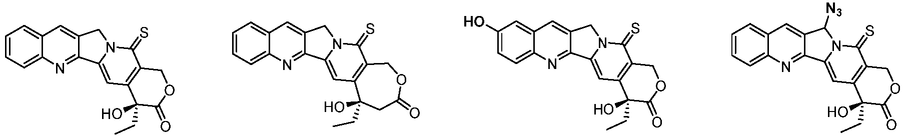

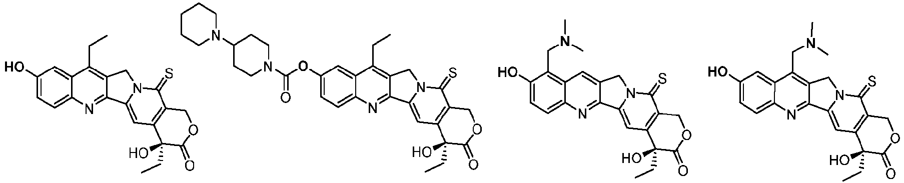

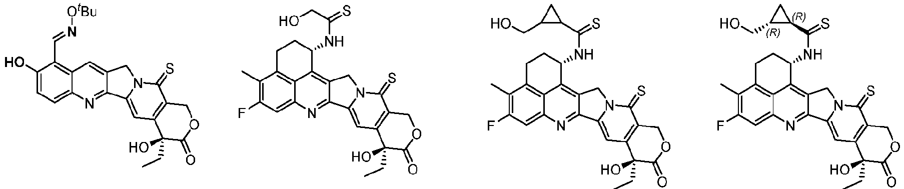

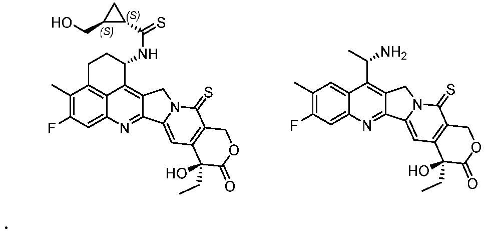

2. The compound according to claim 1, or a pharmaceutically acceptable salt or hydrate thereof, wherein the compound of formula (I) has a structure as shown in formula (II) or formula (III):

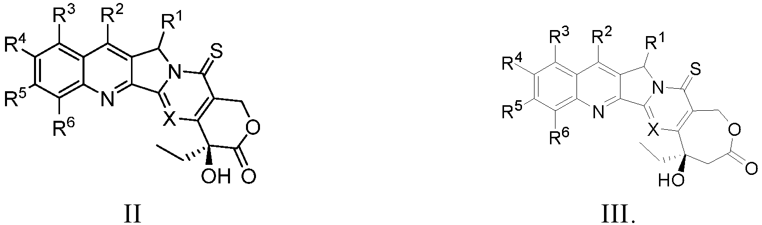

3. The compound according to any one of claims 1-2, or a pharmaceutically acceptable salt or hydrate thereof, wherein the compound of formula (I) has a structure as shown in formula (IV) or formula (V):

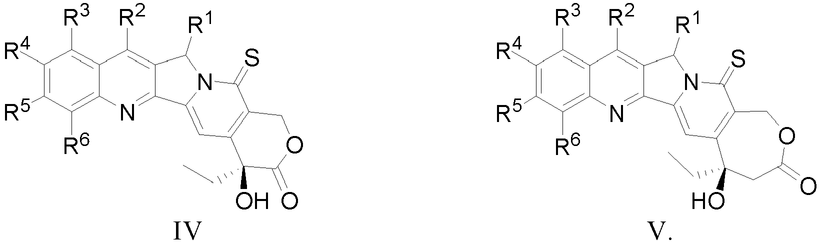

4. The compound according to any one of claims 1-3, or a pharmaceutically acceptable salt or hydrate thereof, wherein the compound of formula (I) has a structure as shown in formula (VI) or formula (VII):

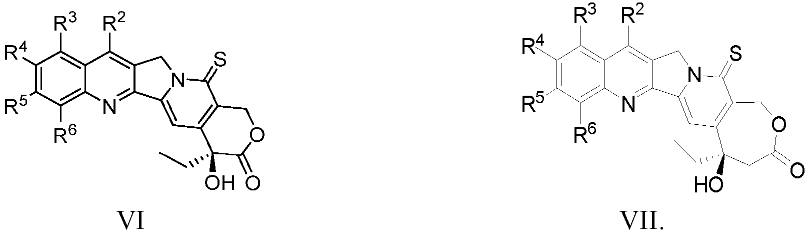

5. The compound according to any one of claims 1-4, or a pharmaceutically acceptable salt or hydrate thereof, wherein R⁴ is selected from the group consisting of hydrogen, deuterium, halogen, hydroxyl, cyano, NH₂, NO₂, substituted or unsubstituted C₁-C₈ alkyl, substituted or unsubstituted C₁-C₈ alkoxy, substituted or unsubstituted C₁-C₈ alkylthio, substituted or unsubstituted C₁-C₈ deuterated alkyl, -(CH₂)ₘ(C₃-C₈ cycloalkyl), -(CH₂)ₘ(3-12 membered heterocyclyl), -(CH₂)ₘN(R⁷)₂, -(CH₂)ₘS(O)(CH₂)ₚR⁷, -(CH₂)ₘS(O)₂(CH₂)ₚR⁷, and -(CH₂)ₘNH(CH₂)ₚR⁷; wherein, m and p are each independently 0, 1 or 2, and R⁷ is defined as described above;
R⁵ is selected from the group consisting of hydrogen, deuterium, halogen, NH₂, OH, substituted or unsubstituted C₁-C₈ alkyl, and substituted or unsubstituted C₁-C₈ alkoxy;
or R⁴ and R⁵ together with the carbon atoms to which they are attached form a structure selected from the group consisting of a saturated or unsaturated 5-6 membered carbocycle that is unsubstituted or substituted by one or more R^{a}, and a saturated or unsaturated 5-6 membered heterocycle that is unsubstituted or substituted by one or more R^{a}; wherein R^{a} is defined as described above.

6. The compound according to any one of claims 1-5, or a pharmaceutically acceptable salt or hydrate thereof, wherein R² and R³ are each independently selected from the group consisting of hydrogen, deuterium, halogen, NH₂, substituted or unsubstituted C₁-C₈ alkyl, substituted or unsubstituted C₁-C₈ deuterated alkyl, -(CH₂)ₘ(C₃-C₆ cycloalkyl), -(CH₂)ₘ(3-6 membered heterocyclyl), -(CH₂)ₘN(R⁷)₂, and -(CH₂)ₘOC(O)R⁷; wherein m is 0, 1, 2, 3, or 4, and R⁷ is defined as described above;
or, R² and R³ together with the carbon atoms to which they are attached form a structure selected from the group consisting of a saturated or unsaturated 5-6 membered carbocycle that is unsubstituted or substituted by one or more R^{a}, and a saturated or unsaturated 5-6 membered heterocycle that is unsubstituted or substituted by one or more R^{a}; wherein R^{a} is defined as described above.

7. The compound of any one of claims 1-6, or a pharmaceutically acceptable salt or hydrate thereof, wherein R¹ and R⁶ are each independently hydrogen.

8. The compound according to any one of claims 1-7, or a pharmaceutically acceptable salt or hydrate thereof, wherein R⁴ and R⁵ are each independently selected from the group consisting of hydrogen, deuterium, halogen, hydroxyl, NH₂, substituted or unsubstituted C₁-C₄ alkyl, substituted or unsubstituted C₁-C₄ alkoxy, and cyclopropyl;
or R⁴ and R⁵ together with the carbon atoms to which they are attached form an oxa-5-6 membered heterocycle that is unsubstituted or substituted by one or more R^{a}; wherein R^{a} is defined as described above.

9. The compound according to any one of claims 1-8, or a pharmaceutically acceptable salt or a hydrate thereof, wherein,
R² is selected from the group consisting of deuterium, halogen, NH₂, substituted or unsubstituted C₁-C₈ alkyl, substituted or unsubstituted C₁-C₈ deuterated alkyl, -(CH₂)ₘ(C₃-C₆ cycloalkyl), -(CH₂)ₘ(3-6 membered heterocyclyl), -(CH₂)ₘN(R⁷)₂, and -(CH₂)ₘOC(O)R⁷; wherein m is 0, 1, 2, 3, or 4;
R³ is selected from the group consisting of hydrogen, deuterium, halogen, substituted or unsubstituted C₁-C₈ alkyl, substituted or unsubstituted C₁-C₈ deuterated alkyl, -(CH₂)ₘ(C₃-C₆ cycloalkyl), -(CH₂)ₘ(3-6 membered heterocyclyl), -(CH₂)ₘN(R⁷)₂, and -(CH₂)ₘOC(O)R⁷; wherein m is 0, 1, 2, 3 or 4;
or, R² and R³ together with the carbon atoms to which they are attached form a structure selected from the group consisting of a saturated or unsaturated 5-6 membered carbocycle that is unsubstituted or substituted by one or more R^{a}, and a saturated or unsaturated 5-6 membered heterocycle that is unsubstituted or substituted by one or more R^{a};
R⁴ is selected from the group consisting of hydrogen, deuterium, halogen, hydroxyl, cyano, NH₂, NO₂, substituted or unsubstituted C₁-C₈ alkyl, and substituted or unsubstituted C₁-C₈ alkoxy;
R⁵ is selected from the group consisting of hydrogen, deuterium, halogen, and substituted or unsubstituted C₁-C₈ alkyl;
or R⁴ and R⁵ linked together to form a structure selected from the group consisting of -OCH₂O- and -O(CH₂)₂O-, and the structure is unsubstituted or substituted by one or more R^{a};
R⁷ is selected from the group consisting of hydrogen, deuterium, halogen, substituted or unsubstituted C₁-C₈ alkyl, hydroxyl, amino, cyano, nitro, and thiol;
wherein R^{a} is defined as described above.

10. The compound according to any one of claims 1-9, or a pharmaceutically acceptable salt or a hydrate thereof, wherein the compound has a structure as shown in the following formulas:

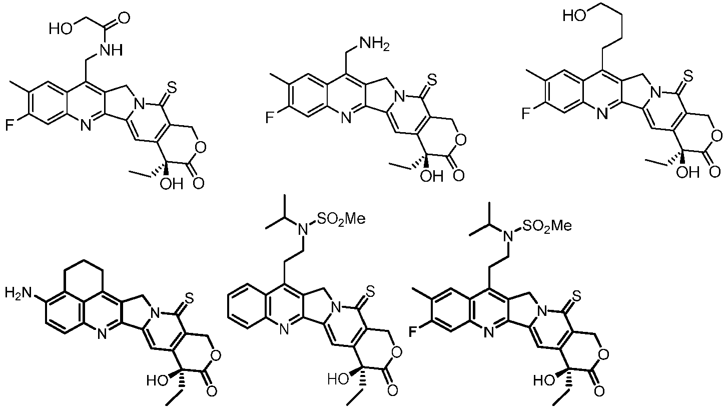

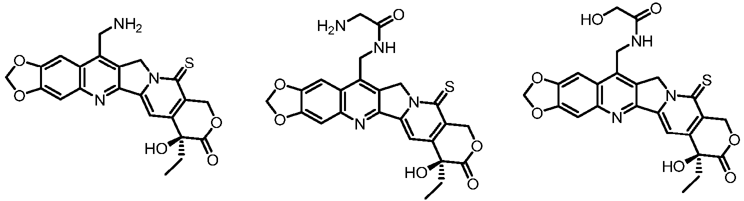

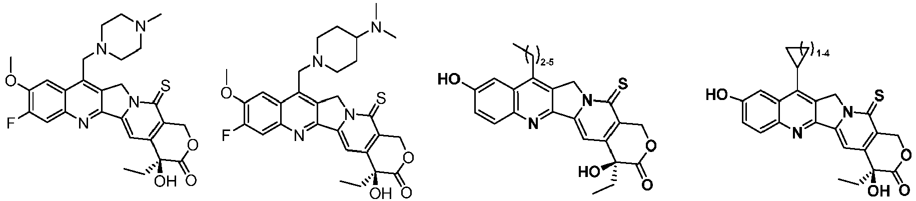

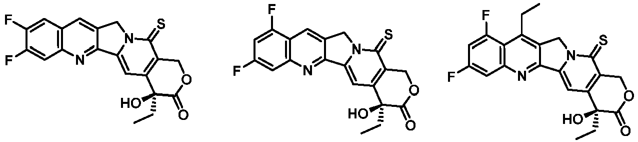

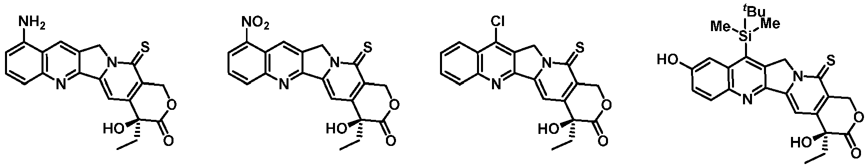

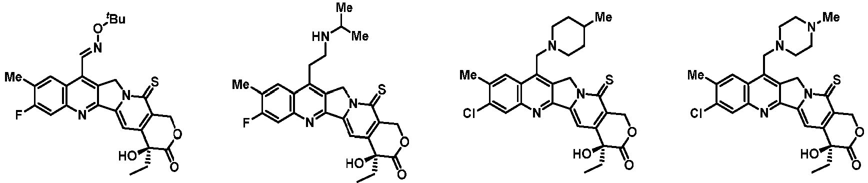

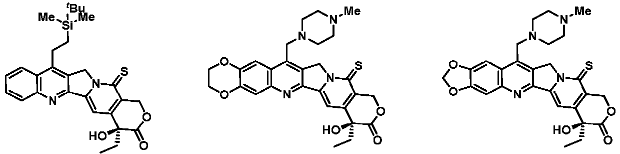

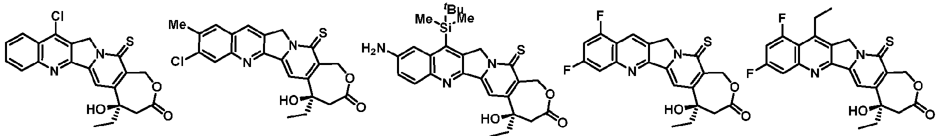

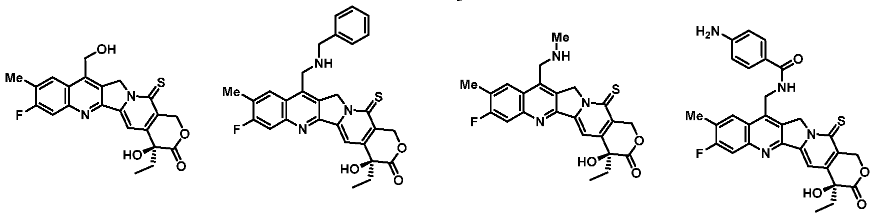

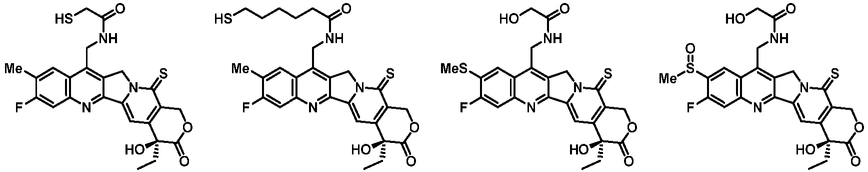

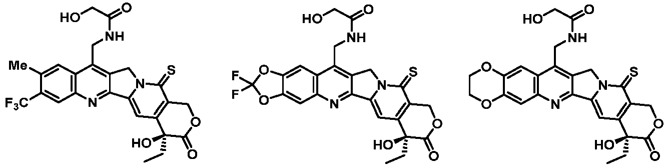

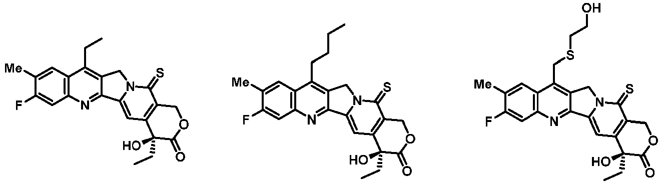

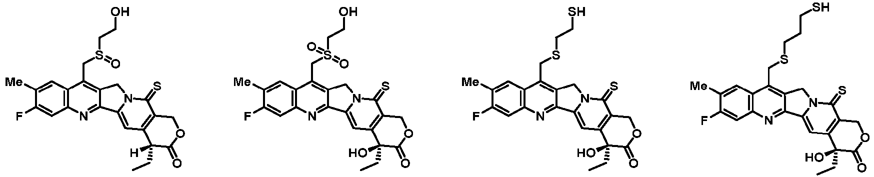

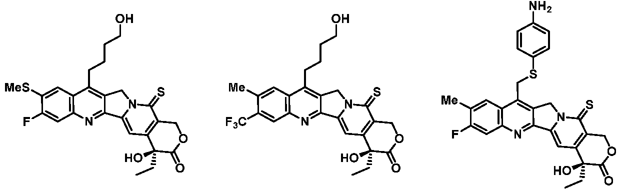

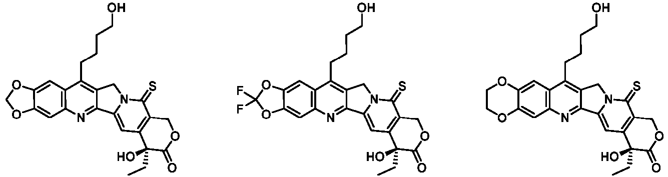

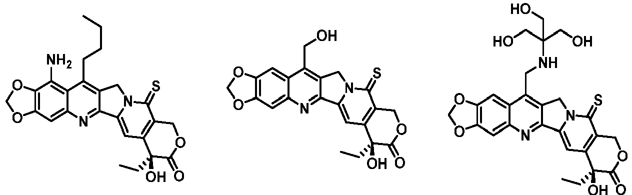

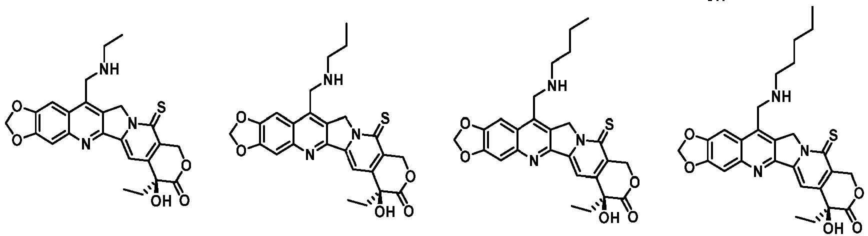

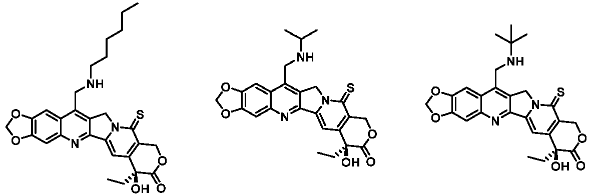

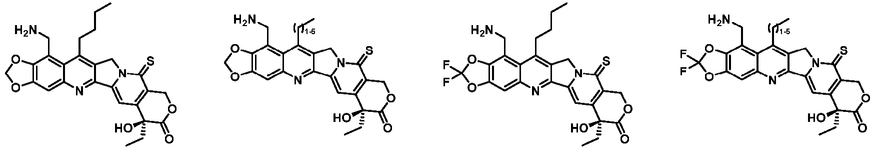

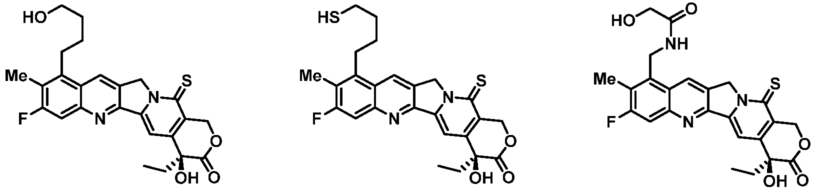

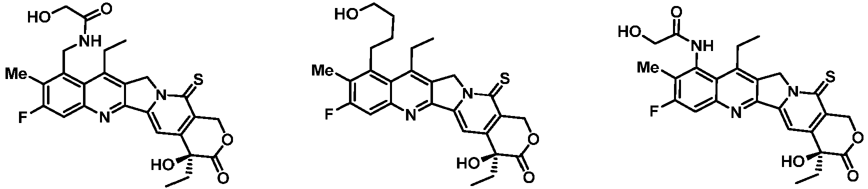

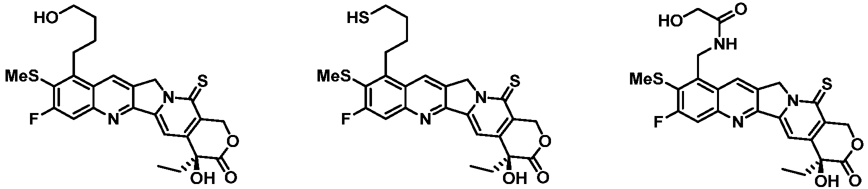

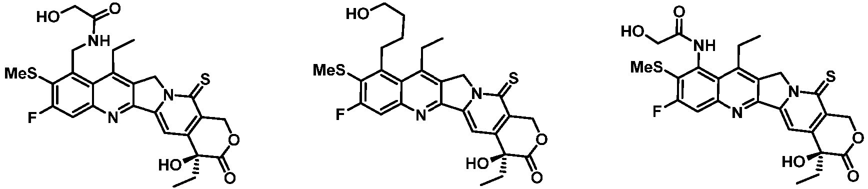

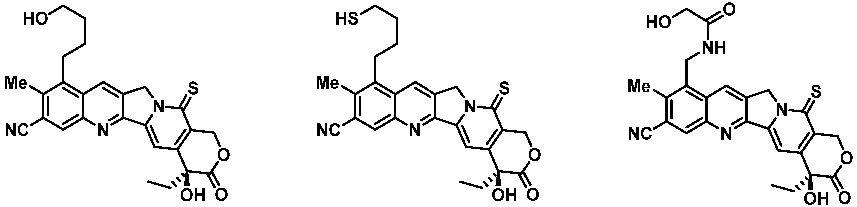

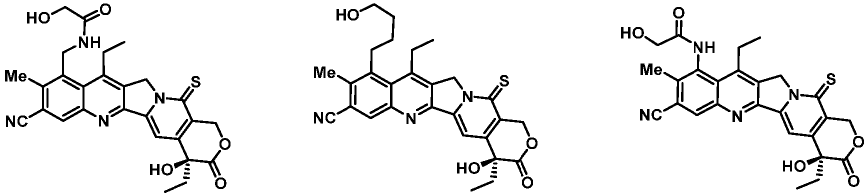

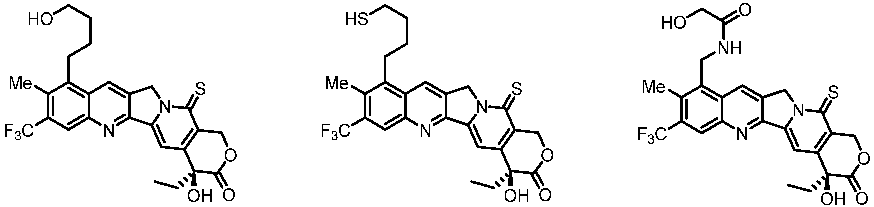

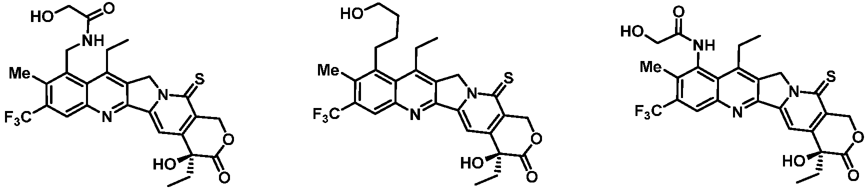

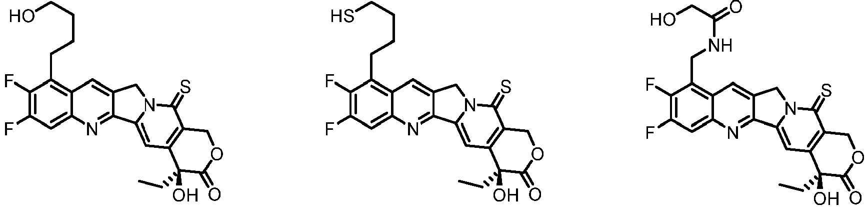

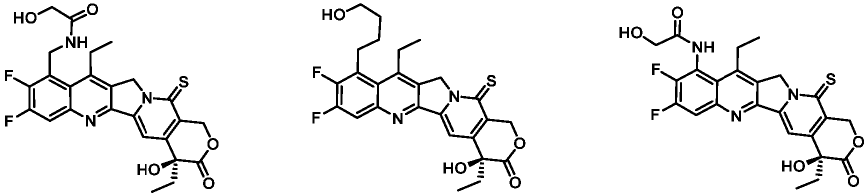

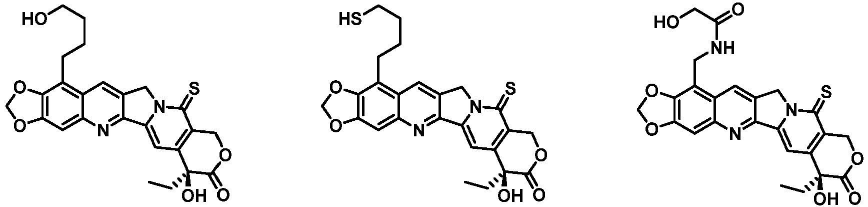

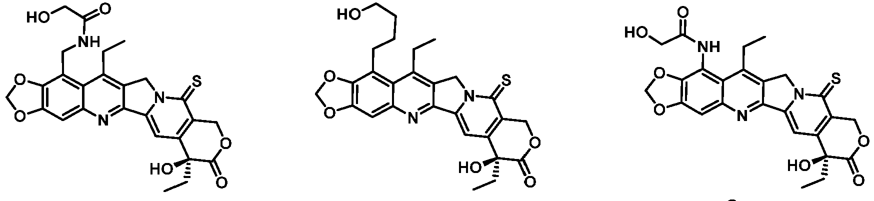

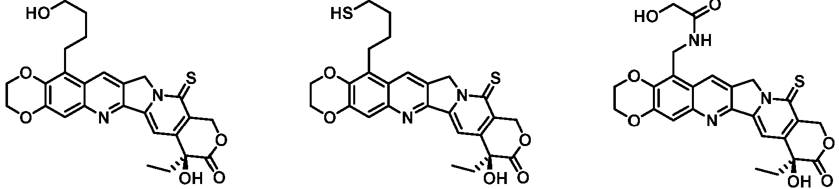

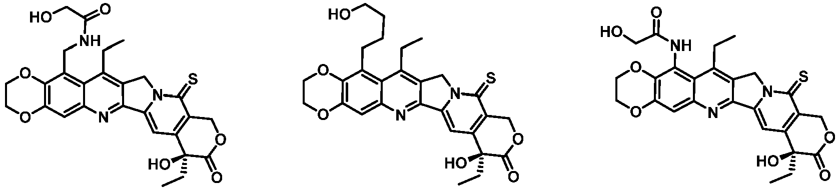

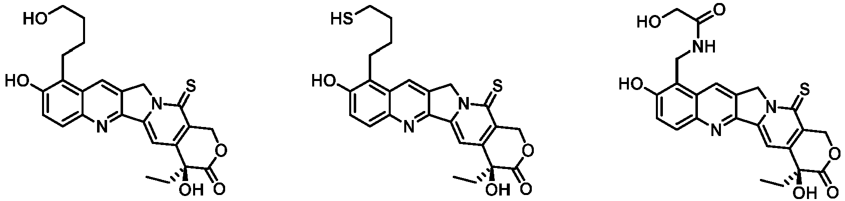

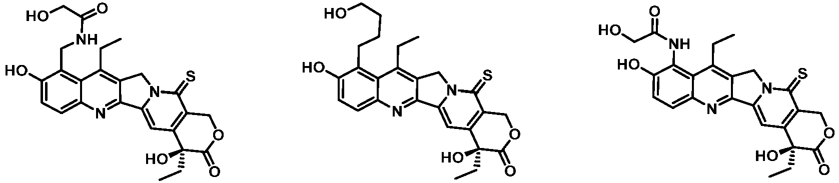

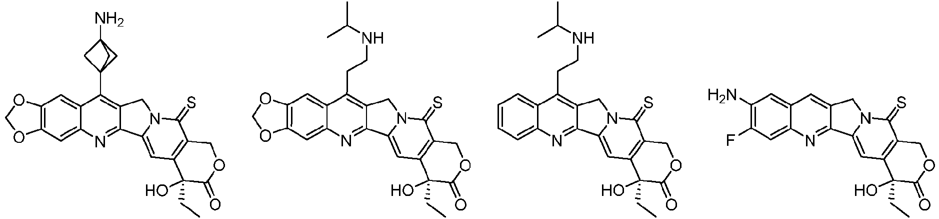

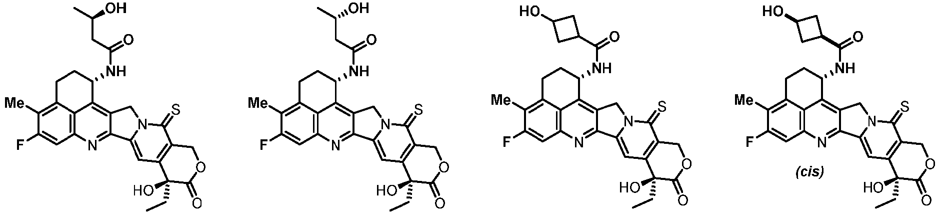

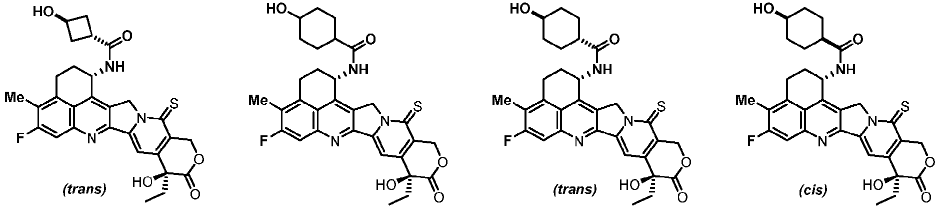

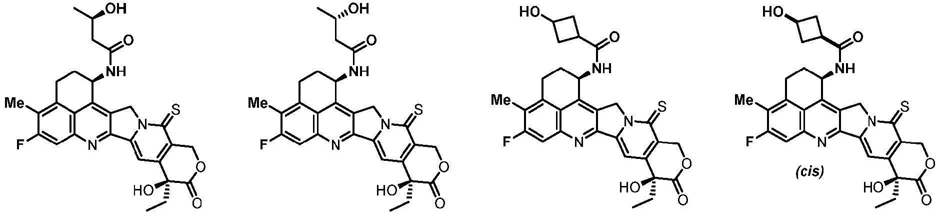

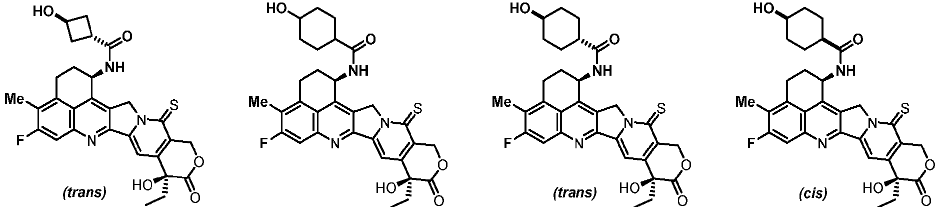

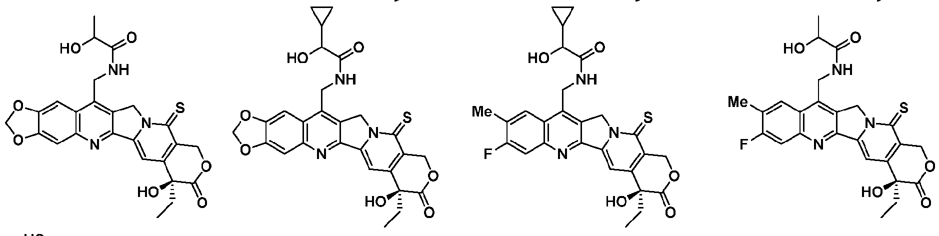

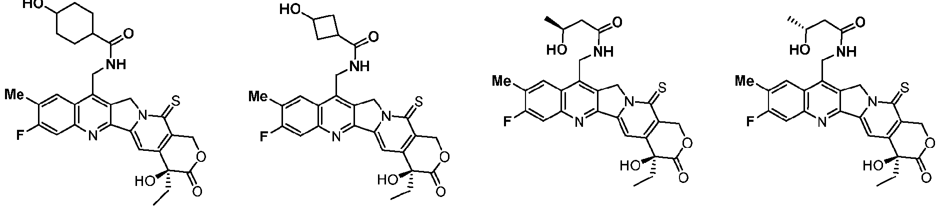

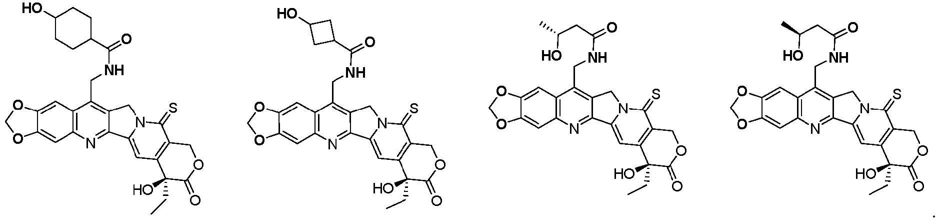

11. A pharmaceutical composition, comprising the compound of formula (I) according to any one of claims 1-10, or a pharmaceutically acceptable salt thereof, or a hydrate thereof, and one or more pharmaceutically acceptable excipients, diluents, or carriers.

12. A use of the compound of formula (I) according to any one of claims 1-10 in the preparation of a pharmaceutical composition for treating diseases associated with tumor cell proliferation.

13. The use of the compound of formula (I) according to any one of claims 1-10, wherein it is used as a toxin for antibody-drug conjugate to prepare an antibody drug conjugate.

14. A compound as shown in formula (II-b) or (III-b): 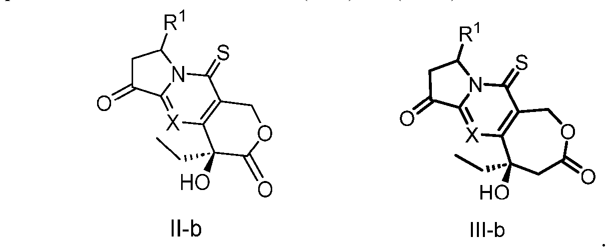 wherein, the definition of each group is as described in claim 1.

15. The compound according to claim 14, wherein the compound has a structure as shown in formula (IV-b) or (V-b):

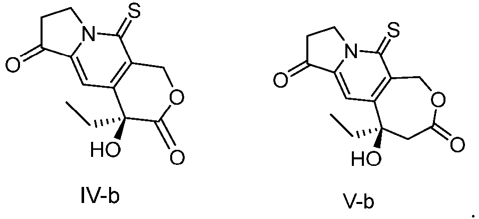

16. A preparation method for the compound of formula (I) according to any one of claims 1-10, wherein the method comprises the step: 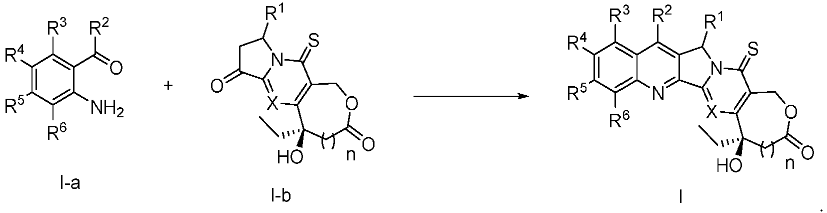 in an inert solvent, using a compound of formula (I-a) to react with a compound of formula (I-b) to obtain a compound of formula (I).
